(19) Europäisches Patentamt
European Patent Office
Office européen des brevets

(11) Publication number: **0 014 567**
**B1**

(12) **EUROPEAN PATENT SPECIFICATION**

(45) Date of publication of patent specification: **18.01.84**

(21) Application number: **80300284.9**

(22) Date of filing: **31.01.80**

(51) Int. Cl.³: **C 07 D 501/02,**
C 07 D 501/59,
C 07 D 501/16,
C 07 D 501/18,
C 07 D 499/04,
C 07 D 498/04
//(C07D498/04, 265/00,
205/00)

(54) **Processes for preparation of beta-lactam compounds.**

(30) Priority: **01.02.79 US 8470**
**01.02.79 US 8645**
**01.02.79 US 8647**

(43) Date of publication of application:
**20.08.80 Bulletin 80/17**

(45) Publication of the grant of the patent:
**18.01.84 Bulletin 84/3**

(84) Designated Contracting States:
**DE GB SE**

(56) References cited:
**FR - A - 2 019 764**
**GB - A - 1 041 985**
**US - A - 3 925 372**

(73) Proprietor: **ELI LILLY AND COMPANY**
**307, East McCarty Street**
**Indianapolis Indiana 46285 (US)**

(72) Inventor: **Hatfield, Lowell Deloss**
**4, Thornbriar Lane**
**Bargersville, Indiana (US)**
Inventor: **Blaszczak, Larry Chris**
**1327 N. Broadway**
**Indianapolis, Indiana (US)**
Inventor: **Fisher, Jack Wayne**
**2014 Woodcrest Road**
**Indianapolis, Indiana (US)**
Inventor: **Bunnell, Charles Arthur**
**2005, Crowfoot Drive**
**Lafayette, Indiana (US)**

(74) Representative: **Hudson, Christopher Mark et al,**
**European Patent Attorney Erl Wood Manor**
**Windlesham Surrey GU20 6PH (GB)**

**EP 0 014 567 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Processes for preparation of β-lactam compounds

An intensive research effort in the field of cephalosporin antibiotics has produced a number of clinically significant cephalosporin compounds. One of the more recent developments in this area has been the discovery of cephem compounds directly substituted with halogen at the C-3 portion. A number of 3-halo-3-cephems have been described by Chauvette in U.S. Patents Nos. 3,925,372, 4,064,343 and 3,962,227. These potent antibiotic compounds are prepared by halogenation of the corresponding 3-hydroxy-3-cephems. The halogenation of 3-hydroxy-3-cephems to provide 3-chloro and 3-bromo-3-cephems has typically been carried out by reacting the 3-hydroxy-3-cephem compounds with brominating or chlorinating agents including phosgene, oxalyl chloride, thionyl chloride, thionyl bromide and phosphorus halides such as phosphorus trichloride and phosphorus tribromide, usually in the presence of dimethylformamide.

Also, in the preparation of semi-synthetic penicillin and cephalosporin antibiotics most chemical modifications are preformed on β-lactam substrates bearing C-6 or C-7 acylamino groups which are stable to the process conditions but are not preferred for maximum antibiotic acitivity. Thus, a process step common to the production of most if not all of the known clinically significant penicillins and cephalosporins is the cleavage of the C-6 or C-7 acylamino group to provide the corresponding C-6 or C-7 amino compounds which are reacylated as desired. Undoubtedly the most widely used method for cleaving penicillin and cephalosporin acylamino side chains is that wherein the C-6 or C-7 acylamino compound is first converted to the corresponding imino halide and then to an imino ether which, upon acidic hydrolysis or alcoholysis, provides the nucleus (C-6 or C-7 amino) compounds. This general method and improvements thereof have been described in a number of U.S. Patents including Nos. 3,549,628, 3,575,970, 3,697,515, 3,845,043 and 3,868,368.

A number of acid halides, especially, acid chlorides, derived from phosphorus, carbon and sulfur or their oxygen acids have been disclosed as useful for preparing the imino halide intermediates of the three-step amido cleavage process. Phosphorus oxychloride, phosphorus pentachloride, phosphorus trichloride, thionyl chloride, phosgene, oxalyl chloride and catechyl-phosphorous trichloride have, in particular, been described as suitable imino halide forming reagents. Laboratory experience has shown phosphorus pentachloride to be a preferred acid halide reagent for intermediate imino halide preparation.

Cephalosporin sulfoxides are also widely used intermediates in the synthesis of cephalosporin antibiotics. Following the completion of the reactions or synthetic procedures employing the sulfoxide form of a cephalosporin, the sulfoxide function is reduced to provide the cephalosporin molecule in the reduced or sulfide state.

Prior to this invention one preferred method for reducing cephalosporin sulfoxides was that of Murphy et al., U.S. Pat. No. 3,641,014. According to this method, cephalosporin sulfoxides are reduced with 1) hdyrogen and a hydrogenation catalyst, 2) stannous, ferrous, cuprous, or manganous cations, 3) dithionite, iodide, or ferrocyanide, 4) trivalent phosphorous compounds, 5) halosilanes or 6) chloromethylene iminium chlorides wherein certain of these reducing agents require the use of an activator such as acetyl chloride or phosphorus trichloride. For example, sodium dithionate is activated with acetyl chloride in the reduction. Another method for the reduction of cephalosporin sulfoxides was disclosed by Hatfield in U.S. Patent No. 4,044,002 which describe the reduction of cephalosporin sulfoxides using acyl bromides in the presence of bromine scavengers. More recently Kukolja and Spry described the reduction/chlorination of 3-hydroxycephem sulfoxides using phosphorus trichloride, phosphorus pentachloride or phosgene in the presence of dimethylformamide.

Recently we have discovered a novel class of compounds derived not from phosphorus oxygen acids but from aryl esters thereof. More specifically we have discovered that selected triaryl phosphites react with equivalent amounts of chlorine or bromine to provide, initially, kinetically controlled products which, although thermodynamically unstable, can be used advantageously in the preparation of β-lactam compounds. These novel triaryl phosphite halogen compounds are disclosed and claimed in our co-pending application No. 80300282.3 (EP—A—0015079) filed on even date herewith.

The present invention relates to techniques for using the recently discovered triaryl phosphite halogen compound to effect:

a) halogenation of a C-6 or C-7 acylamine penicillin or cephalosporin,
b) halogenation of a 3-hydroxy-3-cephem,
c) one step halogenation of a C-7 acylamine-3-hydroxy-cephem
d) reduction of a cephalosporin sulfoxide
e) one step reduction/halogenation of a 3-hydroxy cephalosporin sulfoxide
f) one step reduction/halogenation of a C-7 acylamine cephalosporin sulfoxide
g) one step reduction/halogenation of a C-7 acylamino-3-hydroxycephalosporin sulfoxide.

The present invention provides a process for preparing a penicillin or cephalosporin derivative which comprises reacting from 1.0 to 5.0 equivalents of a triaryl phosphite-halogen complex of the formula

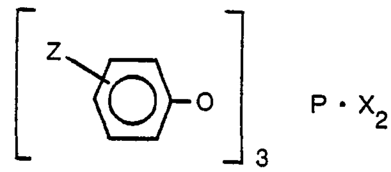

I

wherein X is Cl or Br, and Z is hydrogen, halo, $C_1$—$C_4$ alkyl or $C_1$—$C_4$ alkoxy, which is the kinetically controlled product of the reaction of equivalent amounts of a triaryl phosphite of the formula

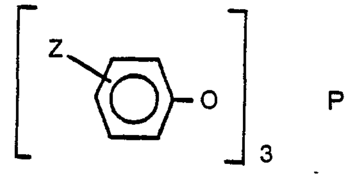

II

and chlorine or bromine in an inert organic solvent, in a substantially anhydrous inert organic solvent at a temperature of 30°C or below, selected from the following process embodiments:

1) a process for preparing penicillin or cephalosporin imino halides which comprises reacting a C-6 acylamino penicillin or a C-7 acylamino cephalosporin with about 1.0 to about 2.0 equivalents of a triaryl phosphite-halogen complex in the presence of 1.0 to 1.2 equivalents of a tertiary amine base per equivalent of halogenating compound employed, in a susbtantially anhydrous inert organic solvent at a temperature of 30°C or below, with the proviso that when the C-6 acylamino penicillin or C-7 acylamino cephalosporin is substituted by hydroxy, amino or carboxy groups those groups are first protected with conventional hydroxy, amino or carboxy protecting groups.

The C-6 acylamino penicillin or C-7 acylamino caphalosporin is, for example, a compound of the formula

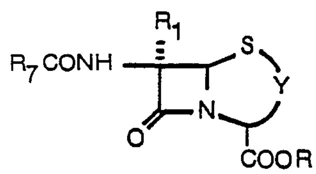

IV

wherein

R is a carboxylic acid protecting group;

$R^1$ is hydrogen or methoxy;

$R_7CO$— is an acyl group derived from a carboxylic acid; and

Y is divalent radical selected from the group consisting of

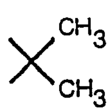 , 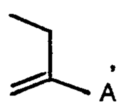 , 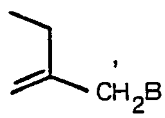 , 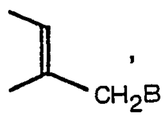 , 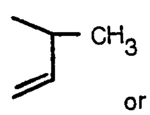 or 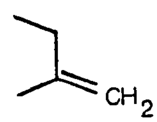

wherein

A is hydrogen, chloro, bromo, protected hydroxy, $C_1$—$C_4$ alkoxy, methyl, $C_1$—$C_4$ alkanesulfonyloxy or $C_1$—$C_4$ alkylphenylsulfonyloxy; and

B is

1) $C_2$—$C_4$ alkanoyloxy, carbamoyloxy, or $C_1$—$C_4$ alkylcarbamoyloxy;

2) $C_1$—$C_4$ alkoxy;

3) chloro or bromo;

4) a group of the formula —$SR_9$ wherein $R_9$ is

a) $C_1$—$C_4$ alkanoyl;

b) $C_1$—$C_4$ alkyl, phenyl or phenyl substituted with 1 or 2 substituents selected from the group consisting of $C_1$—$C_4$ alkyl, $C_1$—$C_4$ alkoxy, protected hydroxy, chloro, bromo, fluoro, nitro, cyano, methanesulfonamido and trifluoromethyl; or

c) a 5- or 6-membered heterocyclic ring containing 1 to 4 heteroatoms selected from the group consisting of oxygen, sulfur and nitrogen, said ring being unsubstituted or substituted by $C_1$—$C_4$ alkyl, $C_1$—$C_4$ alkoxy, chloro, bromo, oxo, halo($C_1$—$C_4$ alkyl), protected amino, protected amino($C_1$—$C_4$ alkyl), protected hydroxy, protected hydroxy($C_1$—$C_4$ alkyl), protected carboxy, or protected carboxy($C_1$—$C_4$ alkyl).

2) a process which comprises reacting the triaryl phosphite-halogen complex with a C-7 acylamino cephalosporin compound of the formula

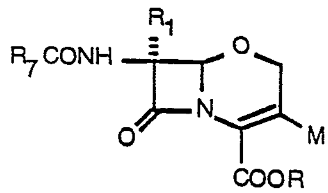

V

wherein R is a carboxylic acid protecting group;

$R_1$ is hydrogen or methoxy;

$R_7CO$— is an acyl group derived from a carboxylic acid; and

M is hydrogen, chloro, bromo, protected hydroxy, $C_1$—$C_4$ alkoxy, methyl, $C_1$—$C_4$ alkylphenylsulfonyloxy, or a group of the formula —$CH_2B$ wherein B is

1) $C_2$—$C_4$ alkanoyloxy, carbamoyloxy, or $C_1$—$C_4$ alkylcarbamoyloxy;

2) $C_1$—$C_4$ alkoxy;

3) chloro or bromo;

4) a group of the formula —$SR_9$ wherein $R_9$ is

a) $C_1$—$C_4$ alkanoyl;

b) $C_1$—$C_4$ alkyl, phenyl or phenyl substituted with 1 or 2 substituents selected from the group consisting of $C_1$—$C_4$ alkyl, $C_1$—$C_4$ alkoxy, protected hydroxy, chloro, bromo, fluoro, nitro, cyano, methanesulfonamido and trifluoromethyl; or

c) a 5- or 6-membered heterocycloic ring containing 1 to 4 heteroatoms selected from the group consisting of oxygen, sulfur and nitrogen, said ring being unsubstituted or substituted by $C_1$—$C_4$ alkyl, $C_1$—$C_4$ alkoxy, chloro, bromo, oxo, halo($C_1$—$C_4$ alkyl), protected amino, protected amino($C_1$—$C_4$ alkyl), protected hydroxy, protected hydroxy($C_1$—$C_4$ alkyl), protected carboxy, or protected carboxy($C_1$—$C_4$ alkyl).

3) A process for preparing a compound of the formula

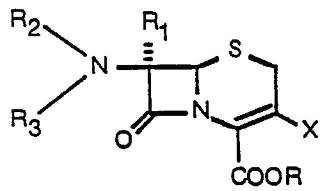

VI

which comprises reacting a compound of the formula

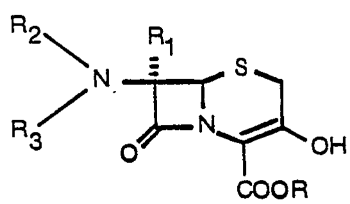

VII

with about 1.0 to about 1.3 equivalents of a triaryl phosphite-halogen complex in a substantially anhydrous inert organic solvent at a temperature below about 30°C. wherein in the above formulas

X is Cl or Br;

R is carboxylic acid protecting group;

$R_1$ is hydrogen or methoxy; and

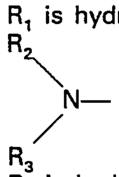 is amino protected by a conventional amino protecting group; or

$R_2$ is hydrogen or an acyl group derived from a carboxylic acid; and

$R_3$ is an acyl group derived from a carboxylic acid; or

$R_2$ and $R_3$ taken together with the nitrogen atom to which they are attached form a group of the formula

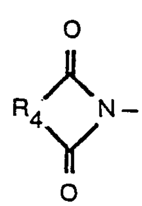

wherein $R_4$ is the residue of an acyl group derived from a dicarboxylic acid; provided that when $R_2$ and $R_3$ are substituted by amino, hydroxy or carboxy groups, those groups are first protected by one of the conventional amino, hydroxy or carboxy protecting groups.

4) a process for preparing an imino halide compound of the formula

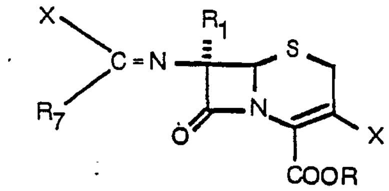

IX

which comprises reacting a compound of the formula

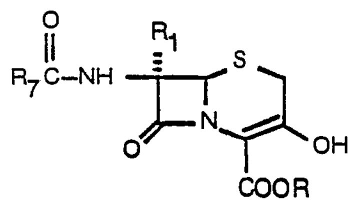

X

with about 2.0 to about 3.0 equivalents of a triaryl phosphite-halogen complex in the presence of about 1.0 to about 1.2 equivalent of a tertiary amine base per equivalent of halogenating compound employed, in a substantially anhydrous inert organic solvent at a temperature below about 30°C wherein in the above formulas

X is Cl or Br;

R is a carboxylic acid protecting group;

$R_1$ is hydrogen or methoxy;

and $R_7$ is the residue of an acyl group derived from a carboxylic acid; provided that when $R_7$ is substituted by amino, hydroxy or carboxy groups, those groups are first protected by one of the conventional amino, hydroxy, or carboxy protecting groups.

5) a process for reducing a cephalosporin sulfoxide to the corresponding cephalosporin which comprises reacting said cephalosporin sulfoxide with about 1.0 to about 1.3 equivalents of a triaryl phosphite-halogen complex in the presence of at least 1 equivalent of a halogen scavenger in a substantially anhydrous inert organic solvent at a temperature of about 30°C or below; provided that when the cephalosporin sulfoxide has a free amino, hydroxy or carboxy group on the C-7 substituent, those groups are first protected by conventional amino, hydroxy or carboxy protecting groups.

The cephalosporin sulfoxide is, for example, a compound of the formula

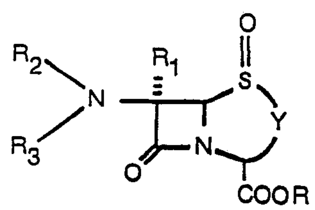

XII

wherein

R′ is hydrogen or a carboxylic acid protecting group;

$R_1$ is hydrogen or methoxy;

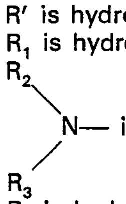 is amino protected by a conventional amino protecting group; or

$R_2$ is hydrogen or an acyl group derived from a carboxylic acid, and

$R_3$ is an acyl group derived from a carboxylic acid; or $R_2$ and $R_3$ taken together with the nitrogen atom to which they are attached form a group of the formula

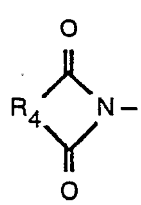

wherein $R_4$ is the residue of an acyl group derived from a dicarboxylic acid; and Y is a divalent radical selected from the group consisting of

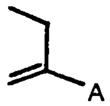 , 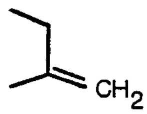 or 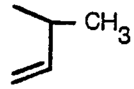

wherein A is hydrogen, chloro, bromo, hydroxy, protected hydroxy, $C_1$—$C_4$ alkoxy, methyl, $C_1$—$C_4$ alkanesulfonyloxy, $C_1$—$C_4$ alkylphenylsulfonyloxy, or a group of the formula —$CH_2B$ wherein B is

1) $C_2$—$C_4$ alkanoyloxy, carbamoyloxy, or $C_1$—$C_4$ alkylcarbamoyloxy;
2) $C_1$—$C_4$ alkoxy;
3) chloro or bromo;
4) $C_1$—$C_4$ alkoxycarbonyl or ($C_2$—$C_6$ haloalkoxy)-carbonyl; or
5) a group of the formula —$SR_9$ wherein $R_9$ is
a) $C_1$—$C_4$ alkanoyl;
b) $C_1$—$C_4$ alkyl, phenyl or phenyl substituted with 1 or 2 substituents selected from the group consisting of $C_1$—$C_4$ alkyl, $C_1$—$C_4$ alkoxy, protected hydroxy, chloro, bromo, fluoro, nitro, cyano, methanesulfonamido and trifluoromethyl; or
c) a 5- or 6-membered heterocyclic ring containing 1 to 4 heteroatoms selected from the group consisting of oxygen, sulfur and nitrogen, said ring being unsubstituted or substituted by $C_1$—$C_4$ alkyl, $C_1$—$C_4$ alkoxy, chloro, bromo, oxo, halo($C_1$—$C_4$ alkyl), protected amino, protected amino($C_1$—$C_4$ alkyl), protected hydroxy, protected hydroxy($C_1$—$C_4$ alkyl), protected carboxy, or protected carboxy($C_1$—$C_4$)-alkyl

or a cephalosporin sulfoxide compound in which $R_2$ is an acyl group of the formula $R_7CO$— wherein $R_7$ is

1) hydrogen, $C_1$—$C_6$ alkyl, halo($C_1$—$C_4$)alkyl, cyanomethyl, trifluoromethylthiomethyl, or 4-protected amino-4-protected carboxybutyl;
2) the group $R_a$ wherein $R_a$ is phenyl or phenyl substituted with 1 or 2 substituents selected from the group consisting of $C_1$—$C_4$ alkyl, $C_1$—$C_4$ alkoxy, protected hydroxy, chloro, bromo, fluoro, iodo, nitro, cyano, carbamyl, methanesulfonamido and trifluoromethyl;
3) an arylalkyl group of the formula

$$R^o—(Q)_m—CQ_1Q_2—$$

wherein $R^o$ is $R_a$ as defined above, 1,4-cyclohexadienyl, or a 5-membered heterocyclic ring containing 1 to 4 heteroatoms selected from the group consisting of oxygen, nitrogen, and sulfur, said ring being unsubstituted or substituted by $C_1$—$C_4$ alkyl, $C_1$—$C_4$ alkoxy, chloro, bromo, oxo, protected amino, protected amino($C_1$—$C_4$ alkyl), protected hydroxy or protected carboxy;
m is 1 or 0;
Q is oxygen or sulfur, and
$Q_1$ and $Q_2$ are indpendently hydrogen or methyl;
subject to the limitation that in the above formula when m is 1, $R^o$ is limited to $R_a$;
4) a substituted arylalkyl group of the formula

$$\begin{array}{l} R^oCH— \\ \quad\ | \\ \quad W \end{array}$$

wherein $R^o$ is as defined above and W is ureido, protected amino, protected hydroxy or protected carboxy; or
5) a substituted oximino group of the formula

$$\begin{array}{l} R^o—C— \\ \quad\ \ \| \\ \quad\ NR_b \end{array}$$

wherein $R^o$ is defined as in paragraph (3) immediately hereinabove and $R_b$ is $C_1$—$C_4$ alkoxy.
6) A process for preparing a compound of the formula

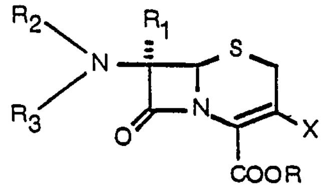

VI

by reacting a compound of the formula

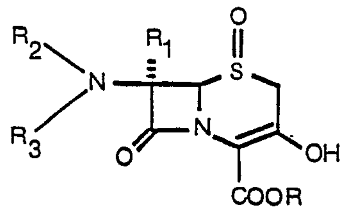

XIII

with about 2 to about 3 equivalents of a triaryl phosphite-halogen complex in the presence of at least 1 molar equivalent of a halogen scavenger in a substantially anhydrous inert organic solvent at a temperature of about 30°C or below; wherein in the above formulas

$R_1$ is hydrogen or methoxy;

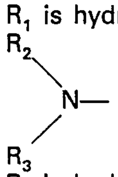 is amino protected by a conventional amino protecting group; or

$R_2$ is hydrogen or an acyl group derived from a carboxylic acid, and

$R_3$ is an acyl grouip derived from a carboxylic acid; or $R_2$ and $R_3$ taken together with the nitrogen atom to which they are attached form a group of the formula

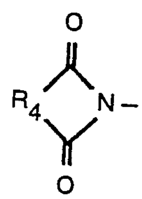

wherein $R_4$ is the residue of an acyl group derived from a dicarboxylic acid; provided that when the C-7 substituent

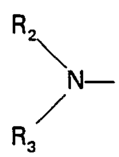

on the cephalosporin sulfoxide is substituted by hydroxy, amino or carboxy groups, those groups are first protected by conventional hydroxy, amino, or carboxy protecting groups.

7) A process for preparing a cephalosporin imino halide of the formula

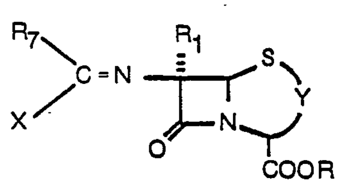

XIV

which comprises reacting a 7-acylamino cephalosporin sulfoxide of the formula

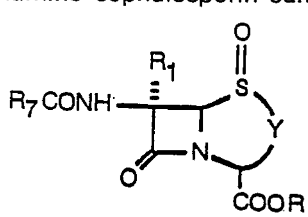

XV

with about 2 to about 3 equivalents of a triaryl phosphite-halogen complex in the presence of at least 1 equivalent of a halogen scavenger and about 1 to about 2 equivalents of a tertiary amine base in a substantially anhydrous inert organic solvent at a temperature of about 30°C or below, wherein in the above formulas

R is a carboxylic acid protecting group;

$R_1$ is hydrogen or methoxy;

$R_7$ is the residue of an acyl group derived from a $C_1$—$C_2$ carboxylic acid of the formula $R_7COOH$; and

Y is a divalent radical selected from the group consisting of

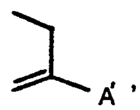, 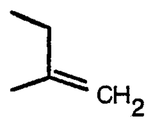 and 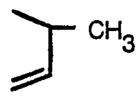

wherein A′ is hydrogen, chloro, bromo, protected hydroxy, $C_1$—$C_4$ alkoxy, methyl, $C_1$—$C_4$ alkanesulfonyloxy, $C_1$—$C_4$ alkylphenylsulfonyloxy, or a group of the formula —$CH_2B$ wherein

B is

1) $C_2$—$C_4$ alkanoyloxy, carbamoyloxy, or $C_1$—$C_4$ alkylcarbamoyloxy;

2) $C_1$—$C_4$ alkoxy;

3) chloro or bromo;

4) $C_1$—$C_4$ alkoxycarbonyl or ($C_2$—$C_6$ haloalkoxy)carbonyl; or

5) a group of the formula —$SR_9$ wherein $R_9$ is

a) $C_1$—$C_4$ alkanoyl;

b) $C_1$—$C_4$ alkyl, phenyl or phenyl substituted with 1 or 2 substituents selected from the group consisting of $C_1$—$C_4$ alkyl, $C_1$—$C_4$ alkoxy, protected hydroxy, chloro, bromo, fluoro, nitro, cyano, methanesulfonamido and trifluoromethyl; or

c) a 5- or 6-membered heterocyclic ring containing 1 to 4 heteroatoms selected from the group consisting of oxygen, sulfur and nitrogen, said ring being unsubstituted or substituted by $C_1$—$C_4$ alkyl, $C_1$—$C_4$ alkoxy, chloro, bromo, oxo, halo($C_1$—$C_4$ alkyl), protected amino, protected amino($C_1$—$C_4$ alkyl), protected hydroxy, protected hydroxy($C_1$—$C_4$ alkyl), protected carboxy, or protected carboxy($C_1$—$C_4$ alkyl); provided that when $R_7$ is substituted by hydroxy, amino or carboxy groups, those groups are first protected by conventional hydroxy, amino, or carboxy protecting groups.

8) a process for preparing a 3-halocephalosporin imino halide of the formula

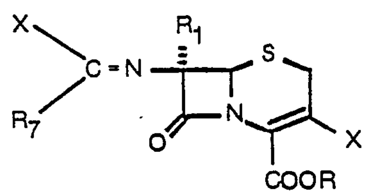

IX

which comprises reacting a 7-acylamino-3-hydroxycephalosporin sulfoxide of the formula

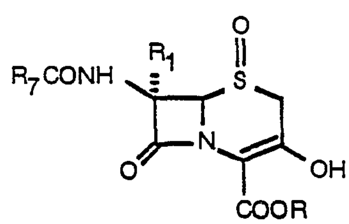

XVII

with about 3 to about 5 equivalents of a triaryl phosphite-halogen complex in the presence of at least 1 equivalent of a halogen scavenger and about 2.0 to about 5.0 equivalents of a tertiary amine base in a substantially anhydrous inert organic solvent at a temperature of about 30° or below; wherein in the above formulas

R is a carboxylic acid protecting group;

$R_1$ is hydrogen or methoxy;

X is Cl or Br; and

$R_7$ is the residue of an acyl group derived from a $C_1$—$C_{20}$ carboxylic acid of the formula $R_7COOH$; provided that when $R_7$ is substituted by hydroxy, amino or carboxy groups, those groups are first protected by conventional hydroxy, amino, or carboxy protecting groups.

Exemplary of the $C_6$ and $C_7$ acylamino groups are formamido, acetamido, propionamido, butyramido, chloroacetamido, 2-bromopropionamido, cyanoacetamido, trifluoromethylthioacetamido, 4-*tert*-butoxycarbonylamino-4-*tert*-butoxycarbonylbutyramido, benzamido, 4-methylbenzamido, 3-nitro-

benzamido, 2-iodobenzamido, 4-benzyloxybenzamido, 3-cyanobenzamido, 2,6-dichlorobenzamido, 4-trifluoromethylbenzamido, 3,4-diethoxybenzamido, and 3-methanesulfonamidobenzamido.

When $R_7$ is a group $R^o—(Q)_m—CQ_1Q_2—$ representative acylamino groups are phenylacetamido, 4-bromophenylacetamido, 3,5-dinitrophenylacetamido, 4-benzyloxyphenylacetamido, phenoxyacetamido, 4-chlorophenoxyacetamido, 2-propoxyphenoxyacetamido, 4-carbamylphenoxyacetamido, cyclohexadienylacetamido, phenylthioacetamido, 2,5-dichlorophenylthioacetamido, 3-nitrophenylthioacetamido, 2-trifluoromethylphenylthioacetamido, 2-phenylpropionamido, 2-phenoxypropionamido, 2-phenyl-2-methylpropionamido, 2-(4-chlorophenyl)propionamido, 2-furylacetamido, 2-thienylacetamido, 5-isoxazolylacetamido, 2-thiazolylacetamido, 2-thienylpropionamido, 5-thiazolylacetamido, 2-chloroacetamidothiazol-5-ylacetamido, 5-bromothien-2-ylacetamido, 1-tetrazolyacetamido, 5-tetrazolylacetamido and the like.

Illustrative of the acylamino groups when $R_7$ is a substituted arylalkyl group of the formula

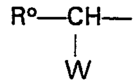

and when W is protected hydroxy are 2-formyloxy-2-phenylacetamido, 2-benzyloxy-2-(4-methoxyphenyl)acetamido, 2-(4-nitrobenzyloxy)-2-(3-chlorophenyl)acetamido, 2-chloroacetoxy-2-(4-methoxyphenyl)acetamido, 2-benzyloxy-2-phenylacetamido, 2-trimethylsilyloxy-2-(4-chlorophenyl)acetamido, 2-benzhydryloxy-2-phenylacetamido and the like groups. Representative of such groups when W is protected amino are 2-(4-nitrobenzyloxycarbonylamino)-2-phenylacetamido, 2-(2,2,2-trichloroethoxycarbonylamino)-2-phenylacetamido, 2-chloroacetamido-2-(1,4-cyclohexadien-1-yl)acetamido, 2-(4-methoxybenzyloxycarbonylamino)-2-(4-methoxyphenyl)acetamido, 2-benzhydryloxycarbonylamino-2-phenylacetamido, 2-(1-carbomethoxy-2-propenyl)amino-2-phenylacetamido, 2-(4-nitrobenzyloxycarbonylamino)-2-(2-thienyl)-acetamido and like groups.

When W is protected carboxy the group $R_7CONH—$ can be 2-(4-nitrobenzyloxycarbonyl)-2-(2-thienyl)acetamido, 2-benzhydryloxycarbonyl-2-phenylacetamido, 2-(2,2,2-trichloroethoxycarbonyl)-2-(4-chlorophenyl)acetamido, 2-tert-butoxycarbonyl-2-(4-benzyloxyphenyl)acetamido and like groups.

Imido group represented by the formula

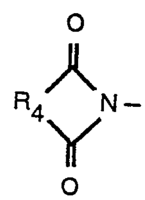

are maleimido, 3-ethylmaleimido, 3,4-dimethylmaleimido, succinimido, phthalimido, and 3,4,5,6-tetrahydrophthalimido.

Representative of $R_9$ in formula XII is an unsubstituted heterocyclicic ring are pyridyl, pyrazinyl, pyridazinyl, pyrimidyl, 1,2,4-triazinyl, pyrazolyl, imidazolyl, thiazolyl, 1,2,4-triazolyl, 1,2,3-triazolyl, 1,2,3-thiadiazolyl, 1,2,4-thiadiazolyl, 1,3,4-thiadiazolyl, 1,2,3-oxadiazolyl, 1,2,4-oxadiazolyl, 1,3,4-oxadiazolyl, 1H-tetrazolyl, 2H-tetrazolyl and the like.

A preferred group of heterocyclic rings represented by $R_9$ are

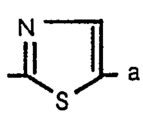 , 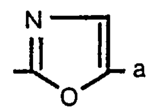 , 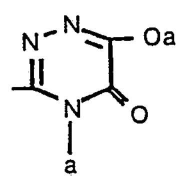 ,

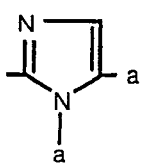 , 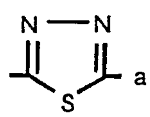 , 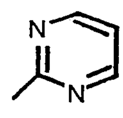 ,

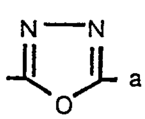 , 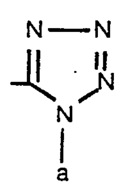 ,

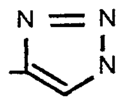 , 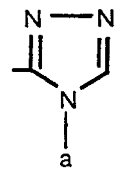 , 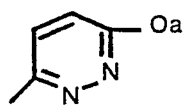 ,

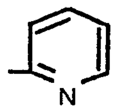 and 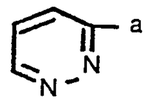

wherein a is hydrogen or $C_1$—$C_4$ alkyl.

The compounds used in these processes, triaryl phosphite-halogen complexes, are recently discovered compounds derived from the reaction of selected triaryl phosphites and chlorine or bromine.

Triaryl phosphites of the formula

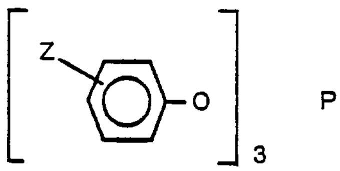 II

wherein Z is hydrogen, halo, $C_1$—$C_4$ alkyl or $C_1$—$C_4$ alkoxy, react with equivalent amounts of chlorine or bromine in a substantially anhydrous inert organic solvent to provide, initially, kinetically controlled products having the empirical formula

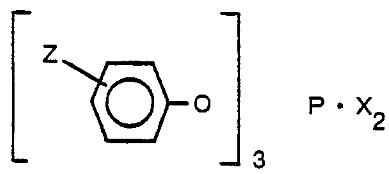 I

wherein Z is as defined above and X is Cl or Br.

The term "halo" in the definition of Z includes chloro, bromo or iodo. "$C_1$—$C_4$ Alkyl" includes methyl, ethyl, isopropyl, n-propyl, n-butyl, sec-butyl, tert-butyl and isobutyl. Representative "$C_1$—$C_4$ alkoxy" groups are methoxy, ethoxy, isopropoxy, tert-butoxy and n-butoxy.

The dot (·) in the general formula used to represent the kinetically controlled products employed in the present processes is used simply to designate that equivalent amounts of halogen and triaryl phosphite are combined chemically and in a way that can be distinguished from that in the thermodynamically stable derivatives which have been known in the art and which typically have been drawn without the dot [e.g. $(PhO)_3PCl_2$]. The exact molecular form of the triaryl phosphite-halogen kinetic complexes described herein has not been established definitively; however, physical-chemical data do indicate that the kinetic product is one wherein the phosphoric center aquires some cationic character. Herein the terms "kinetic compound", "kinetic complex", "triaryl phosphite-halogen complex (compound)", "kinetically controlled products" and "kinetically controlled halogenating (reducing) compounds" are used synonomously.

Suitable triaryl phosphites for the preparation of the kinetically controlled compounds used in the present process include triphenyl phosphite, tri(p-methoxyphenyl)phosphite, tri(o-chlorophenyl)-phosphite tri(p-chlorophenyl)phosphite, tri(p-tolyl)phosphite, tri(o-tolyl)phosphite, tri(m-bromophenyl)-phosphite, tri(p-bromophenyl)phosphite, tri(p-iodophenyl)phosphite, tri(p-*n*-propylphenyl)phosphite, tri(p-*tert*-butylphenyl)phosphite, tri(m-tolyl)phosphite, tri(p-isopropoxyphenyl)phosphite and the like. Triphenyl phosphite is preferred, primarily because of commercial availability.

Any of a wide variety of inert organic solvents may be employed as the medium for the preparation of the kinetically controlled compounds and for the reduction and reduction-halogenation processes described hereinbelow. By "inert organic solvent" is meant an organic solvent which under the reaction conditions of the preparation does not enter into any appreciable reaction with either the reactants or the products. Since the halogenating compounds are susceptible to reaction with protic compounds, such compounds, including water, alcohols, amines (other than tertiary), thiols, organic acids and other such protic compounds should be excluded from the reaction medium.

A substantially anhydrous aprotic organic solvent is preferred. The term "substantially anhydrous" as used in the present description means that although anhydrous organic solvents are generally preferred, trace amounts of water, such as that often found in commercially available solvents, can be tolerated. Although the kinetic products described herein will react with any water present in the

solvent medium, additional amounts of reagents can easily be added to compensate for the loss due to hydrolysis. It is preferred that conventional laboratory techniques be employed to dry the solvents employed and to exclude moisture from the reaction mixtures.

Suitable solvents include hydrocarbons, both aliphatic and aromatic, including pentane, hexane, heptane, octane, cyclohexane, cyclopentane, benzene, toluene, o-, m- or p-xylene, mesitylene and the like; ethers, cyclic and acyclic such as diethyl ether, butyl ethyl ether, tetrahydrofuran, dioxane, 1,2-dimethoxyethane and the like; carboxylic acid esters such as ethyl acetate, methylformate, methyl acetate, amyl acetate, *n*-butyl acetate, *sec*-butyl acetate, methyl propionate, methyl butyrate and the like; nitriles such as acetonitrile, propionitrile, butyronitrile and the like; halogenated hydrocarbons, both aromatic and aliphatic, such as chloroform, methylene chloride, carbon tetrachloride, 1,2-dichloroethane (ethylene dichloride), 1,1,2-trichloroethane, 1,1-dibromo-2-chloroethane, 2-chloropropane, 1-chlorobutane, chlorobenzene, fluorobenzene, o-, m-, or p-chlorotoluene, o-, m-, or p-bromotoluene, dichlorobenzene and the like; and nitro compounds such as nitromethane, nitroethane, 1- or 2-nitropropane, nitrobenzene and the like.

The particular inert organic solvent employed as a medium for the preparation of the kinetically controlled triaryl phosphite-halogen compounds or as a medium for their use in the present processes is not critical, however, such solvent properties as polarity, melting or boiling point, and ease of isolation of products may be considered in selecting a most suitable solvent.

Preferred solvents for the preparation of the kinetically controlled products and for the present processes described hereinbelow are hydrocarbons, especially aromatic hydrocarbons, and halogenated hydrocarbons. Halogenated hydrocarbons other than chloroform are more preferred. Methylene chloride is most preferred.

If a compound derived from the kinetically controlled reaction of a triaryl phosphite and chlorine or bromine is allowed to stand in solution it converts or isomerizes to the corresponding thermodynamically stable compound at varying rates depending on, among other things, the nature of the triaryl phosphite, the solvent, the halogen, and the solution temperature. Experimental data has also shown that the presence of an acid (HX) or an excess of triaryl phosphite will enhance the rate of conversion of the kinetic to the thermodynamic product.

Using $^{31}P$ nucleur magnetic resonance spectroscopy the half-life of the kinetically controlled product from the reaction of triphenyl phosphite and chlorine in methylene chloride at room temperature was determined to be about 8 hours. A half-life of about 39 hours was observed for the triphenyl phosphite-bromine kinetic complex under the same conditions. As mentioned above the observed half-life (rate of conversion) for any given kinetic complex described herein can be affected by the solvent and by the presence of a hydrogen halide acid (HX) or excess triaryl phosphite. Thus, for example, a shorter half-life will be observed where the solvent for the preparation of kinetic complex has not been rigorously dried; the hydrogen halide acid produced from reaction of the kinetic complex with the moisture present in the solvent will enhance the rate of conversion to the stable form. Table I presents a summary of several properties of the kinetically controlled product and the corresponding thermodynamically controlled product of the reaction of triphenyl phosphite and chlorine.

TABLE I

| | Kinetic product | | Thermodynamic product |
|---|---|---|---|
| 1. | $^{31}P$ nmr ($CH_2Cl_2$) − 3.7 ppm* | 1. | $^{31}P$ nmr ($CH_2CL_2$) + 22.7 ppm* |
| 2. | $^{t}1/2 = \simeq 8$ hours at room temperature in methylene chloride | 2. | Stable at room temperature |
| 3. | ir ($CH_2Cl_2$) 1120—1190 (vs), 1070 (vs), 1035 (s), 1010 (vs), 990 (vs), 640 (m). 625 (m), 580 (w). 510 (s), 465 (w).** | 3. | ir ($CH_2Cl_2$) 1130—1210 (vs), 1065 (vs), 1035 (s), 1010 (vs, 980 (vs), 625 (vw), 590 (m), 505 (s), 460 (s).** |
| 4. | Hydrolyzes to give HCl and $(PhO)_3PO$ | 4. | Hydrolyzes to give *inter alia* HCl, PhOH (phenol) and $(PhO)_2PCl$ |
| 5. | React with n-BuOH to give HCl, n-BuCl and $PhO_3PO$ | 5. | Reacts with n-BuOH to give HCl, PhOH (phenol), n-BuCl and $(PhO)_a$ — $(BuO)_b$ $POCl_c$ wherein a, b, c, = $0^a$, 1, 2 or 3 and a + b + c = 3 |

*Relative to $^{31}P$ of $H_3PO_4$; (+) indicates upfield shift; (—) indicates down field shift
**vs = very strong, s = strong, m = medium, w = weak

The term kinetically controlled product is a term of art which when used in reference to reactions yielding two (or more) products, refers to the product formed faster, regardless of its thermodynamic stability. If such a reaction is stopped well before the products achieve thermodynamic equilibrium, the reaction is said to be kinetically controlled since more of the faster formed product will be present. In some cases, including the reaction of triaryl phosphites with chlorine or bromine, the rate of formation of the kinetic product and the rate of thermodynamic equilibrium is such that the kinetically controlled product can be prepared and utilized before any significant amount of the kinetically controlled product equilibrates or isomerizes to the thermodynamically stable product.

To maximize the production and stability of the kinetically controlled product, reaction conditions are selected so as to minimize the potential for thermodynamic equilibrium of the initial product of the reaction. Most simply conditions for kinetic control are achieved both by lowering the reaction temperature and the temperature of the kinetic product after it is formed, and by minimizing the time allowed for thermodynamic equilibrium, such as, by utilizing the kinetic product in a subsequent reaction shortly after it has been prepared.

Typically the reactants, a triaryl phosphite and chlorine or bromine, are combined in a substantially anhydrous inert organic solvent at a temperature below about 30°C. Although the kinetically controlled products are formed at higher temperature, such conditions favor the formation of the thermodynamically controlled products. Preferably the triaryl phosphite-halogen compounds are prepared at temperatures at or below about 30°C. Minimum reaction temperature are, of course, determined by the freezing point of the solvent employed for the preparation. Most preferred reaction temperatures are in the range of about −70° to about 0°C.

It has been found that the triaryl phosphite itself reacts to some extent with its kinetic reaction product with chlorine or bromine, effectively increasing the rate of conversion to the corresponding thermodynamic product. It is preferred, therefore, but not required, that an excess of halogen be maintained in the reaction mixture during the formation of the kinetic compounds. This can be achieved practically by adding the triaryl phosphite to a solution of an equivalent amount of the halogen or by adding the halogen and the triaryl phosphite simultaneously to a quantity of inert organic solvent at the desired temperature. The co-addition of reagents is conducted at such a rate that the color of the halogen persists in the reaction mixture until the last drop of triaryl phosphite discharges the color. Alternatively excess halogen can be discharged using known halogen scavengers such as acetylenes, or olefins including alkenes, dienes, cycloalkenes, or bicycloalkenes. A preferred scavenger is a $C_2$ to $C_6$ alkene, for example, ethylene, propylene, butylene, or amylene.

The kinetically controlled triaryl phosphite-halogen complexes used in the process of the present invention are stabilized in solution by the addition of about 10 to about 100 mole percent of a tertiary amine base having a $pK_b$ value of about 6 to about 10. If, for example, about 50 mole percent of pyridine is added to a solution of the kinetically controlled product of the reaction of triphenyl phosphite and chlorine in methylene chloride, only trace amounts of the thermodynamic equilibrium product can be detected by $^{31}P$ nmr, even after prolonged periods at room temperature. The tertiary amine base can be added to a solution of the freshly prepared triaryl phosphite-halogen complex or, optionally, it can be employed in the reaction mixture of the triaryl phosphite and halogen to produce a stabilized solution of the kinetically controlled product used in the present invention.

The Halogen Scavenger

As the reduction process of the present invention proceeds, chlorine or bromine (depending on the triaryl phosphite-halogen complex employed) is produced as a by-product. In order to prevent undesirable side reactions between the halogen by-product and the cephalosporin product, a halogen scavenger is used in the reaction mixture to react with or inactivate the chlorine or bromine as it is formed. The term "halogen scavenger" as used herein in the description of the present invention refers to organic substances which react readily with chlorine or bromine and which do not react with the triaryl phosphite-halogen complex used as a reducing agent in the present process. Representative of halogen scavengers which can be employed in the present process are alkenes, cycloalkenes, bicycloalkenes, dienes, cyclodienes, bicyclodienes, alkynes or substituted aromatic hydrocarbons which readily undergo electrophilic substitution with bromine or chlorine, for example mono hydric phenols and the ethers and esters of monohydric and polyhydric phenols. Examples of such halogen scavengers include the $C_2$ to $C_{10}$ alkenes, such as ethylene, propylene, butene-1, butene-2, isobutylene, pentene-1, penten-2, 2-methylbutene-1, 3-methylbutene-1, hexene-1, heptene-1, octene-1, the isomeric nonenes, and the like; cycloalkenes having from 5 to 8 ring carbon atoms such as cyclopentene, cyclohexene, cycloheptene, and cyclooctene; $C_4$—$C_8$ dienes and cyclodienes having from 5—8 ring carbon atoms, for example, pentadiene, hexadiene, heptadiene, cyclopentadiene, cyclohexadiene, cyclooctadiene, 2,3-dimethylbutadiene-1,3-isoprene, and the like; alkynes having from 2—6 carbon atoms, such as acetylene, methylacetylene, ethylacetylene, dimethylacetylene, pentyne-1, pentyne-2, the isomeric hexynes, 3-methylbutyne-1, 3,3-dimethylbutyne-1, and like acetylenes wherein the acetylenic bond will rapidly add chlorine or bromine (phenylacetylene was found to be an unsatisfactory chlorine scavenger); bicyclic unsaturated hydrocarbons such a camphene and pinene; and phenol ethers, substituted phenol ethers, and lower alkanoyl phenol esters represented by the formula

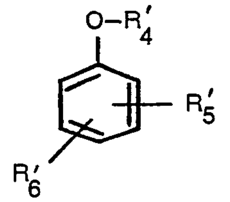

XVIII

wherein $R_4'$ is $C_1$—$C_4$ alkyl or $C_2$—$C_5$ alkanoyl, $R_5'$ and $R_6'$ are independently hydrogen, $C_1$—$C_4$ alkoxy, $C_2$—$C_5$ alkanoyl, or $C_1$—$C_4$ alkyl. Examples of such derivatives include the hydroquinone monomethyl ether, hydroquinone dimethyl ether, anisole, phenetole, m-dimethoxybenzene, veratrole, phenyl propionate, phenyl acetate, resorcinol diacetate, and like phenol ethers and esters which react readily with chlorine or bromine.

Preferred halogen scavengers are $C_2$—$C_6$ alkenes, for example, ethylene, propylene, butylene amylene, cyclopentene or cyclohexene.

Since theoretically at least 1 molar equivalent of halogen is produced for each equivalent of sulfoxide reduced in the present process, at least a molar equivalent amount of halogen scavenger is employed in the cephalosporin sulfoxide reduction process for each equivalent of cephalosporin sulfoxide starting material. Typically about 1 to about 3 molar equivalents of halogen scavenger is used for each equivalent of starting material; however, larger amounts of halogen scavenger can be employed without affecting the reduction process.

C-7 Acylamino cephalosporin and C-6 acylamino penicillin starting materials for the present halogenating process are all known compounds, or they can be derived from known compounds by conventional procedures. The patent and chemical literature is replete with teachings of how to prepare penicillin and cephalosporin compounds which can be used in the present process. For example, 3-exomethylene cepham compounds are described in U.S. Patent Nos. 3,932,393, 4,052,387 and 4,060,688. 2-Methyl-3-cephems are described in the *Journal of the American Chemical Society, 97* 5020 (1975) and *98,* 2342 (1976). Also the book *Penicillins and Cephalosporins,* E. H. Flynn, ed., Academic Press, New York, 1972, describes a wide variety of pencillins and cephalosporins and preparations thereof.

The starting materials for the present process can be represented by the general formula

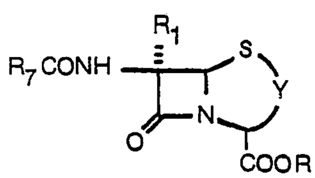

IV

wherein R, $R^1$, $R^7$ and Y are as defined above.

To the extent that there are no unprotected amino, hydroxy, carboxy groups or other protic substituents on these starting materials, the nature of the variables R, $R_1$, Y and $R_7$ is not critical to the present process. It is the C-6 or C-7 amido functionality which is modified under the conditions of the present process, from —CONH— to

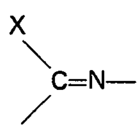

wherein X is chloro or bromo. R, $R_1$, $R_7$ and Y typically remain unaffected. Of course, as with most other chemical processes, yields of imino halide products or nucleus esters derived therefrom can vary from one substrate to another.

The C-7 acylamino cephalosporin starting material for the present process can be a 1-oxa-dethiacephem compound of the formula

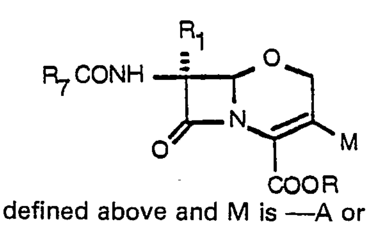

V

wherein R, $R_1$, and $R_7$ are as defined above and M is —A or —$CH_2$B as defined above. These, too, are known compounds, or they can be derived from known compounds by conventional procedures. They, as well as the corresponding 1-carba-dethiacephems and 1-aza-dethiacephems which can also be employed in the present process, are described in U.S. Patent No. 4,123,528.

Each of the process embodiments described above is conducted in the presence of a tertiary amine base. Typically from about 1.0 to about 1.2 equivalents and preferably about 1.0 equivalents of a tertiary amine base is employed for each equivalent of halogenating agent. Preferred tertiary amines bases for this process and the combination enol-halogenation/imino-halogenation described herein below are those having a $pK_b$ value of about 1 to about 10. More preferred are those tertiary amine bases having a $pK_b$ value of about 6 to about 10. Exemplary of suitable tertiary amine bases for use in the presence invention are trialkylamines such as trimethylamine, triethylamine, tri-n-propylamine, ethyldimethylamine, benzyldiethylamine and the like; dialkylarylamines such as dimethylaniline, diethylaniline, N,N-diethyl-4-methylaniline, N-methyl-N-ethylaniline, N,N-dimethyltoluidine and the like; cyclic and bicyclic tertiary amines such as pyridine, collidine, quinoline, isoquinoline, 2,6-lutidine, 2,4-lutidine, 1,5-diazabicyclo[4.3.0]nonene-5 (DBN), 1,5-diazabicyclo[5.4.0]undecene-5 (DBU), triethylenediamine and the like; and polymeric tertiary amine bases such as the copolymer formed from divinylbenzene and vinylpyridine described by Hallensleben and Wurm in *Angew. Chem. Intl. Ed. Engl., 15,* 163 (1976). Pyridine is a preferred tertiary amine base.

The Cephalosporin Sulfoxide

The present process can be applied generally to the reduction of any of wide variety of known cephalosporin sulfoxides. Representative of cephalosporin sulfoxides which can be reduced to the corresponding cephalosporin compounds are cephalosporin sulfoxides of the formula

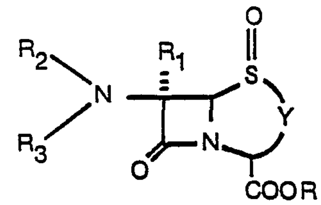

XII

wherein $R^1$, $R^2$, $R^3$, $R'$ and Y are as defined above.

To the extent that there are no unprotected amino or non-enolic hydroxy groups on these starting materials, the nature of the variables $R_1$, $R_2$, $R_3$ and Y are not critical. The groups $R_1$, $R_2$, $R_3$ and Y are typically not affected by the present process. Of course as with most other chemical processes the yields of cephalosporin products from the present process will vary from one cephalosporin to another.

The sulfoxides employed in this process embodiment have been described in U.S. patents. With reference to the above formula XII, the compounds wherein A is ($C_1$—$C_4$ alkoxy)carbonyl or ($C_2$—$C_6$ haloalkoxy)carbonyl are described by Spry in U.S. Patent No. 3,953,436. The 3-hydroxy-3-cephem sulfoxides and 3-exomethylenecepham sulfoxides are described by Chauvette in U.S. Patent No. 3,917,587, and by Kukolja in U.S. Patent No. 4,052,387. Those sulfoxides wherein A is $C_1$—$C_4$ alkanesulfonyloxy or a phenyl or substituted phenylsulfonyloxy group are prepared by the method disclosed in U.S. Patent No. 3,985,737. The 2-methyl-3-cephems are described in the *Journal of the American Chemical Society, 97,* 5020 (1975) and *98,* 2342 (1976). Further, Cooper described a generally applicable method for the synthesis of cephalosporin sulfoxides in U.S. Patent No. 3,647,786.

A preferred group of cephalosporin sulfoxides in the present process are those of the above formula wherein.

R′ is a carboxylic acid protecting group;

$R_1$ is hydrogen;

$R_2$ is hydrogen and $R_3$ is an acyl group of the formula $R_7CO$— wherein $R_7$ is an aryl-alkyl group of the formula

$$R^o—(Q)_m—CQ_1Q_2—$$

wherein

$R^o$ is 2-thienyl, phenyl or substituted phenyl, Q is O, m is 1 or 0, and $Q_1$ and $Q_2$ are hydrogen; and Y is a divalent radical of the formula

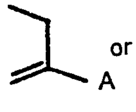

or

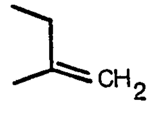

wherein A is as defined above.

When cephalosporin carboxylic acids are employed in the present process yields are typically lower because the kinetic complex reacts not only with the sulfoxide moiety but also with the carboxy group to form the corresponding acid halide which, under normal product isolation procedures, is hydrolyzed to the acid. Preferably, the C-4 carboxy function of the cephalosporin sulfoxide is protected prior to its reduction in the present process. To increase the reduction yields when cephalosporin sulfoxide acids are employed in the present process an additional equivalent of the kinetic complex can be

used. An aqueous work-up of the reaction mixture will allow the corresponding cephalosporin acid to be isolated.

The term "protected amino" as employed in the above definition has reference to an amino group substituted with one of the commonly employed amino blocking groups such as the *tert*-butoxycarbonyl group (*t*-BOC); the benzyloxycarbonyl group, the 4-methoxybenzyloxycarbonyl group, the 4-nitrobenzyloxycarbonyl group, the 2,2,2-trichloroethoxycarbonyl group, or the 1-carbomethoxy-2-propenyl group formed with methyl acetoacetate. Like amino protecting groups such as those described by J.W. Barton in *Protective Groups in Organic Chemistry,*, J.G.W. McOmie, Ed., Plenum Press, New York, N.Y., 1973, Chapter 2, shall be recognized as suitable.

The term "protected hydroxy" has reference to the readily cleavable groups formed with an hydroxyl group such as the formyloxy group, the chloroacetoxy group, the benzyloxy group, the benzhydryloxy group, the trityloxy group, the 4-nitrobenzyloxy group, the trimethylsilyloxy group, the phenacyloxy group, the tert-butoxy group, the methoxymethoxy group, the tetrahydropropyranyloxy group, and the like. Other hydroxy protecting groups, including those described by C. B. Reese in *Protective Groups in Organic Chemistry,* supra, Chapter 3 shall be considered as within the term "protected hydroxy" as used herein.

The term "carboxylic acid protecting group" has reference to the commonly used carboxylic acid protecting groups employed to block or protect the carboxylic acid functionality while reactions involving other functional sites of the compound are carried out. Such carboxy protecting groups are noted for their ease of cleavage by hydrolytic or by hydrogenolytic methods to the corresponding carboxylic acid. Examples of carboxylic acid ester protecting groups include methyl, *tert*-butyl, benzyl, 4-methoxybenzyl, $C_2$—$C_6$ alkanoyloxymethyl, 2-iodoethyl, 4-nitrobenzyl, diphenylmethyl (benzhydryl), phenacyl, 4-halophenacyl, dimethylallyl, 2,2,2-trichloroethyl, tri($C_1$—$C_3$ alkyl)silyl, succinimidomethyl and like ester forming moieties. In addition to ester protection of carboxy groups, such groups can also be protected as the mixed anhydride, such as that formed with acetyl chloride, propionyl chloride, isobutyryl chloride and like acid chlorides in the presence of a tertiary amine base. Other known carboxy protecting groups such as those described by E. Haslam in *Protective Groups in Organic Chemistry,* supra, Chapter 5, shall be recognized as suitable. The nature of such ester forming groups is not critical.

In the foregoing definitions hydroxy, amino and carboxy protecting groups are not exhaustively defined. The function of such groups is to protect the reactive function groups during the present process and then be removed at some later point in time without disrupting the remainder of the molecule. Many protecting groups are known in the art, and the use of other protecting groups not specifically referred to hereinabove are equally applicable to the substrates used in the processes of the present invention.

The triphenyl phosphite-halogen complexes (Z=H) are the preferred halogenating agents in the halogenation processes of this invention. The triphenyl phosphite-chlorine kinetic complex is most preferred for the present processes. For the enol-halogenation process, best results are seen when about 1.1 to about 1.2 equivalent of halogenating reagent are used for each equivalent of enol substrate. For the combination enol-halogenation/imino-halogenation process, preferably about 2.2 to about 2.4 equivalents, and most preferably about 2.3 equivalents, of halogenating compounds are employed for each equivalent of enol substrate.

The Reaction Conditions

The halogenation processes of this invention are preferably carried out at a temperature of about 0° or below. A reaction temperature of about −10° or below is more preferred. Usually the present processes are not conducted at a temperature below about −70°C. Most preferred is a reaction temperature of about −10 to about −70°C. It should be noted that the present chlorination processes can be conducted, although not advantageously, at temperatures above 30° and below −70°. The freezing point of the reaction medium and substrate solubility are limiting factors at low temperatures while the ability of the thermodynamically unstable halogenating agent is the main consideration in selection of higher reaction temperatures. Of course, if the halogenating agent has been stabilized in solution with a tertiary amine base as described hereinabove, the upper temperature range for the present process becomes even a less critical variable; higher temperatures could easily be employed without significant loss of the halogenating agent and without detriment to the halogenation process itself.

The imino halide forming embodiment of the present process is usually carried out at a temperature of about 30°C. or below. Preferably the present process is conducted at a temperature of about 0°C. or below and more preferably at about −10°C. or below. Usually the process is not conducted at a temperature less than about −70°C. Most preferred is a temperature range of about −10° to about −70°C.

It should be noted that the imino halide forming embodiment of the process of the present invention can be conducted at temperatures above 30° and below −70°. The freezing point of the reaction medium and substrate solubility are possible limiting factors at low temperatures while stability of the thermo-dynamically unstable halogenating agent and the product imino halides is the main considerations in avoiding selection of higher reaction temperatures. Of course, if the

halogenating agent has been stabilized in solution with a tertiary amine base as described hereinabove, the upper temperature range for the present process becomes a less critical variable; higher temperature could easily be employed without significant loss of the halogenating agent and without detriment to the halogenation process itself.

The reduction embodiment of the process of the present invention is conducted in a substantially anhydrous inert organic solvent. Such solvents have been described and exemplified above in the description of the triaryl phosphite-halogen complexes. Preferred solvents for the present process are hydrocarbons, especially aromatic hydrocarbons and halogenated hydrocarbons. Halogenated hydrocarbons other than chloroform are more preferred. Methylene chloride is most preferred.

This embodiment is usually carried out at a temperature of about 30°C. or below. Preferably it is conducted at a temperature of about 10°C or below. Usually it is not conducted at a temperature less than about −50°C. Most preferred is a temperature range of about −0° to about −30°C.

It should be noted that the reduction embodiment of the process of the present invention can be conducted at temperatures above 30°C and below −50°C. The freezing point of the reaction medium, substrate solubility and reaction rates are possible limiting factors at low temperatures while stability of the thermodynamically unstable triaryl phosphite-halogen complex and the product cephalosporins is the main considerations in avoiding selection of higher reaction temperatures. Of course, if the triaryl phosphite-halogen complex has been stabilized in solution with a tertiary amine base as described hereinabove, the upper temperature range for the present process becomes a less critical variable; higher temperatures could easily be employed without significant loss of the reducing agent and without detriment to the reduction itself.

Typically the reduction embodiment of the process is carried out simply by adding the cephalosporin sulfoxide either as a solid or in solution to a mixture of the triaryl phosphite-halogen complex (about 1 to about 1.3 molar equivalents per equivalent of sulfoxide) and a halogen scavenger (about 1 to about 3 molar equivalents per equivalent of sulfoxide) in an inert organic solvent at the desired temperature. The course of the reaction can be followed, for example, by comparative thin-layer chromatography. The reduction is usually complete after about 30 minutes to about 2 hours under preferred reaction conditions. Isolation and purification of the product cephalosporins can be accomplished by conventional laboratory techniques including, for example, extraction, crystallization and recrystallization, filtration, and trituration. The cephalosporin products are known compounds and useful as antibiotics (after removal of protecting groups) or as intermediates to other cephalosporin compounds.

The triaryl phosphite-halogen complexes utilized as reducing agents in the present process are also potent halogenating agents. Then can be used to convert both enolic hydroxy groups to the corresponding vinyl chlorides and, in the presence of base, amido groups to the corresponding imino halides. The multiple reactivity of the triaryl phosphite-halogen kinetic complexes is exploited in each of embodiments of the present invention. Thus, the present invention is also directed to the reduction/halogenation of cephalosporin sulfoxides. These additional aspects of the present invention are illustrated and summarized by reaction schemes I—II:

Scheme I: *Reduction/Enol-halogenation*

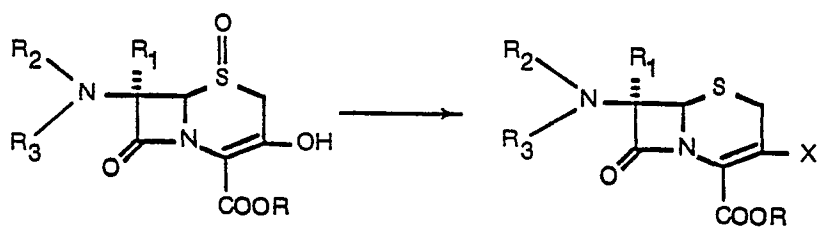

XIII VI

Scheme II: *Reduction/Imino-halogenation*

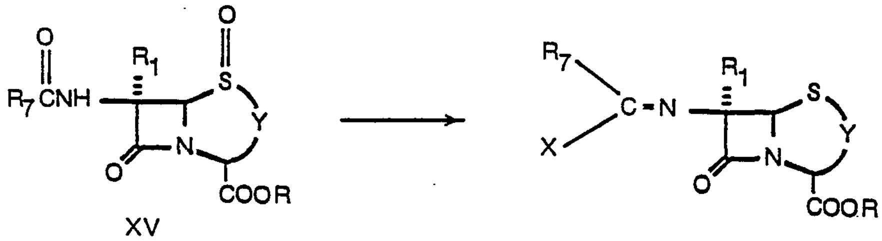

XV XIV

Scheme III: *Reduction/Enol-halogenation/Imino-halogenation*

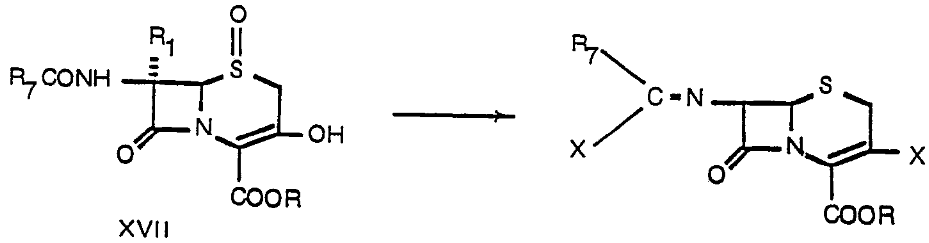

XVII

IX

In the above formulas R is a carboxy protecting group and $R_1$, $R_2$, $R_3$, $R_7$, X and Y are as defined hereinabove, provided that when Y is a radical of the formula

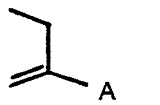

A is not hydroxy. The imino halide products of the reactions depicted in Schemes II and III can be isolated or converted by known procedures (alcoholysis via imino ether) to the corresponding nucleus esters

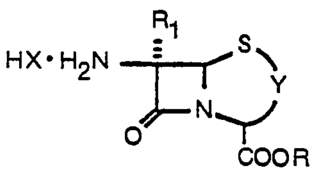

or

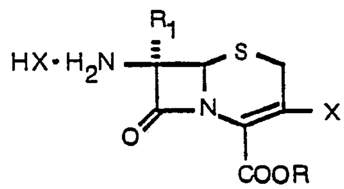

respectively.

XX

XXI

In the reduction/enol-halogenation illustrated by Scheme I above, a 3-halo cephalosporin is prepared by reacting a 3-hydroxy cephalosporin sulfoxide with about 2 to about 3 equivalents of the triaryl phosphite-halogen kinetic complex in the presence of at least 1 molar equivalent of a scavenger in a substantially anhydrous inert organic solvent at a temperature of about 30°C or below.

With reference to Scheme II above, representing another embodiment of the present invention, cephalosporin imino halides are prepared by reacting 7-acylamino cephalosporin sulfoxides with about 2 to about 3 equivalents of the triaryl phosphite-halogen kinetic complexes in the presence of at least 1 equivalent of a halogen scavenger and about 1.0 to about 2.0 equivalents of a tertiary amine base in a substantially anhydrous organic solvent at a temperature of about 30°C or below.

Scheme III above represents a preferred embodiment of the present invention wherein a 3-halocephalosporin imino halide is prepared by reacting a 7-acylamino-3-hydroxy cephalosporin sulfoxide with about 3 to about 5 equivalents of the triaryl phosphite-halogen complexes in the presence of at least 1 equivalent of a halogen scavenger and about 2 to about 5 equivalents of a tertiary amine base in a substantially anhydrous inert organic solvent at a temperature of about 30°C or below. Best results for the Scheme III process have been observed when about 4.4 equivalents of triphenyl phosphite-chlorine kinetic complex and about 3.8 equivalents of pyridine are employed to each equivalent of 7-acylamino-3-hydroxycephalosporin sulfoxide starting material using methylene chloride as a solvent.

The multi-effect/single step process embodiments of the present invention illustrated in Schemes I—III above are conducted under essentially the same conditions detailed hereinabove for the general reduction of cephalosporin sulfoxides using triaryl phosphite-halogen complexes. Except for the particular structural requirements for the cephalosporin sulfoxide reactants, the requirement for the presence of a tertiary amine base in the processes of Schemes II and III, and the stoichiometry unique to the individual multi-conversion processes, all reaction parameters for the multi-conversion processes of Schemes I—III are identical to those described for the basic process of this invention. This includes the aforedescribed temperature ranges, solvents, triaryl phosphite-halogen kinetic complexes, halogen scavengers and preferences therefor.

The cephalosporin products of the present processes can be isolated and purified by conventional laboratory techniques including, for example, extraction, crystallization and recrystallization, and trituration. Because the imino halide products are sensitive to acid catalyzed alcoholysis or hydrolysis and to nucleophilic attack, some precaution should be taken during product isolation to avoid exposing the products to conditions under which such reactions of the imino halide might take place. For example, under neutral conditions achieved by maintaining a concentration of a non-nucleophilic acid scavenger such as propylene oxide, solutions of the imino halide products can be washed with water

and brine and evaporated, usually under reduced pressure, to provide the product in substantially pure form.

Since the primary utility of the imino halide products is as intermediates to the corresponding C-7 aminocephalosporins, preferably the imino halide products of the present process are reacted without isolation from the reducing/halogenating reaction mixture with an excess of a $C_1$—$C_{15}$ aliphatic alcohol or more preferably a $\beta$-disubstituted primary aliphatic alcohol or a 1,2- or 1,3-diol to provide the corresponding nucleus esters.

The improved alcoholysis of cephem imino halides via an imino ether intermediate using $\beta$-disubstituted aliphatic alcohols and 1,2- or 1,3-diols to provide cephem nucleus esters is disclosed in U.S. Patents 3,845,043.

Preferred for imino etherification and subsequent alcoholysis of the imino halide products are a $C_4$—$C_{12}$ $\beta$-disubstituted primary aliphatic alcohol, a $C_3$—$C_{15}$ aliphatic 1,3-diol, or a $C_2$—$C_{12}$ aliphatic 1,2-diol.

Suitable $\beta$-disubstituted primary aliphatic alcohols are those compounds of the formula

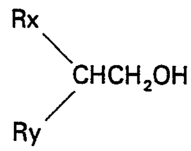

wherein each of Rx and Ry is an alkyl group such that the $\beta$-disubstituted primary aliphatic alcohol has from 4 to about 12 carbon atoms or Rx and Ry are taken together with the carbon atom to which they are bonded to form a cycloalkyl group having from 5 to 8 carbon atoms. Exemplary of such alcohols are isobutanol, 2-methylbutanol, 2-ethylbutanol, 2-ethylhexanol, hydroxymethylcyclopentane, hydroxymethylcyclohexane, 2-n-butyloctanol, 2-n-propylhexanol and like alcohols. Suitable 1,2 or 1,3-diols are those of the formula

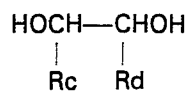

and

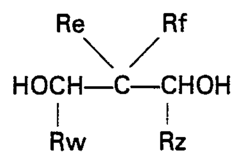

respectively wherein Rc and Rd are hydrogen or alkyl such that the 1,2-diol has from 2 to 12 carbon atoms and wherein Rw and Rz are each hydrogen, methyl or ethyl, and each of Re and Rf is hydrogen or a hydrocarbon moiety such that the 1,3-diol has from 3 to 15 carbon atoms. Representative of 1,2-diols are 1,2-propylene glycol, 2,3-butanediol, 1,2-butanediol, 3,4-pentanediol, and 3,4-hexanediol. Representative of 1,3-diols are 1,3-propanediol, 1,3-butanediol, 1,3-pentanediol, 2,2-dimethyl-1,3-propanediol, 2,2-diethyl-1,3-propanediol, 2,4-pentanediol, and 2,2-diphenyl-1,3-propanediol. Most preferred of alcohols or diols for cleavage of the imino-halide products of the present process are isobutanol, 1,2-propanediol and 1,3-propanediol.

An excess of the alcohol or diol is employed for cleavage of the imino halide products of the process of the present invention. The amount of excess alcohol or diol is not critical. When the aforedescribed 1,2- or 1,3-diols are employed about a 2—3 fold excess will suffice. When a $\beta$-disubstituted primary aliphatic alcohol is employed about a 3—6 fold excess is usually preferred. Of course larger amounts of the alcohol or diol may be employed without affecting the course of the reaction. Often a 10—15 fold excess of the preferred alcohol or diol is used. In general a 3 to 15 fold excess of alcohol or diol is preferred. When aliphatic alcohols other than those mentioned hereinabove as prefered are used to cleave the imino halide products of the present process, larger excesses, about 10—100 fold, are typically employed.

Usually the alcohol or diol is simply added to the halogenating reaction mixture in which the imino chloride has been prepared in accordance with the process of the present invention.

Alcoholysis of the imino halide (via imino ether formation) is acid catalyzed. The reaction mixture itself is usually acidic enough so that alcoholysis occurs upon alcohol or diol addition without the addition of acid to the reaction mixture. However, to enhance the rate of alcoholysis and therefore the rate of nucleus ester formation, the reaction mixture is preferably acidified with, for example, hydrogen chloride after the alcohol or diol has been added to the reaction mixture. This can be accomplished simply by bubbling HCl gas into the reaction mixture for a short period of time. Other acids, organic and inorganic can, however, be employed. Typically at least about 1 equivalent of hydrogen chloride is added to the reaction mixture to promote nucleus ester formation.

The product nucleus esters can be isolated often as their crystalline hydrochloride salts simply by filtering the crystallized product from the reaction mixture. Non-crystalline nucleus esters produced in accordance with the present procedure can be isolated from the reaction mixture using conventional laboratory techniques. Alternatively, the nucleus esters can be reacted (acylated) in solution, without being isolated. Acylation of the nucleus esters using well known laboratory procedures provides C-7

acylamino cephalosporins esters which either can be deesterified to provide known antibiotic compounds or they can be used as intermediates for further chemical modification.

Combining the aforedescribed reduction/enol-imino halogenation (Scheme III above), using a triaryl phosphite-chlorine complex, with subsequent alcoholysis of the resulting imino chloride constitutes an improved method of preparation of 7-amino-3-chloro-3-cephem-4-carboxylic acid esters from the corresponding 7-acylamino-3-hydroxy-3-cephem-4-carboxylic acid ester sulfoxides. Prior to this invention the total 3-function conversion was effected either in 3 separate steps, that is reduction, chlorination and side chain cleavage or in two steps, either combining reduction and chlorination (see U.S. Patent No. 3,115,643) with subsequent side chain cleavage or by combining chlorination and side chain cleavage after reduction of the sulfoxide entity, for example, using the method disclosed in U.S. Patent No. 4,044,002. With the discovery of the present process the reduction, chlorination and cleavage conversions can be effected in excellent yields in one reaction vessel without isolation of intermediates.

The 3-halocephem nucleus esters are known compounds. They can be acylated using conventional acylation techniques and subsequently deesterified to provide known antibiotic compounds. Of particular significance is the utility of these nucleus ester intermediates in the preparation of 7-(D-2-phenyl-2-aminoacetamido-3-chloro-3-cephem-4-carboxylic acid, a relatively new and clinically significant antibiotic.

In a preferred process embodiment of the present invention a 7-amino-3-chloro-3-cephem-4-carboxylic acid ester hydrochloride of the formula

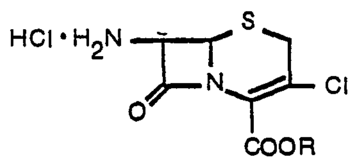

XIX

is prepared by

a) reacting a 7-acylamino-3-hydroxy-3-cephem-4-carboxylic acid ester sulfoxide with about 4.0 to about 5.0 equivalents of the kinetically controlled product of the reaction of equivalent amounts of triphenyl phosphite and chlorine in a substantially anhydrous inert organic solvent, in the presence of about 3.5 to about 4.0 equivalents of pyridine and about 1 to about 3 equivalents of a $C_2$—$C_6$ alkene in a substantially anhydrous inert organic solvent, at a temperature of about −10° to about −30°C;

b) adding about 3 to about 15 equivalents of isobutanol, 1,3-propanediol or 1,2-propanediol to the reaction mixture after formation of the 3-chloro-3-cephem imino chloride is complete; and

c) acidifying the reaction mixture with HCl.

A most preferred inert organic solvent is methylene chloride.

Preferred 3-hydroxy-3-cephem sulfoxide substrates are those bearing conventional penicillin and cephalosporin carboxamido groups at the C-7 position. A particularly preferred group of 3-hydroxy-3-cephem sulfoxides are those bearing an acylamino group of the formula $R^o$—$(Q)_m$—$CQ_1Q_2CONH$— wherein $R^o$ is 2-thienyl, phenyl or substituted phenyl, Q is O, m is 1 or 0 and $Q_1$ and $Q_2$ are hydrogen. More preferred for economic reasons and not necessarily for reactivity are the $C_7$-substituents phenylacetamido, phenoxyacetamido and 2-thienylacetamido. Similarly the 4-nitrobenzyl group is a preferred carboxy protecting group in the preferred process embodiment because of the crystalline nature of the product hydrochloride, and therefore the ease of isolation of a product nucleus ester of high purity.

The following examples are provided to further illustrate the present invention. It is not intended that this invention be limited in scope by reason of any of these examples. In the following examples and preparations nuclear magnetic resonance spectra are abbreviated nmr. The nuclear magnetic resonance spectra were obtained on a Varian Associates T-60 Spectrometer using tetramethylsilane as the reference standard. The chemical shifts are expressed in $\delta$ values in parts per million (ppm) and coupling constants (J) are expressed as Hz (cycles per second).

Example 1

4'-Nitrobenzyl 7-(1-chloro-2-phenylethylidene)imino-3-methyl-3-cephem-4-carboxylate

Into 50 ml of methylene chloride at −15°C was bubbled chlorine gas while simultaneously 3.2 ml (12.3 mmol) of triphenyl phosphite (TPP) was added dropwise to the solution. The chlorine and TPP were combined at such a rate that the faint yellow color of chlorine could be noted in the reaction mixture throughout the co-addition. Near the end of the TPP addition, chlorine addition was discontinued. TPP was then added until the yellow color of the reaction mixture was dissipated. Additional chlorine and the remaining TPP were then added to the reaction mixture until the last drop of TPP dissipated the chlorine color.

To the resulting solution of the prepared triphenyl phosphite-chlorine kinetic complex (TPP—C) at −15°C was added 4.68 g (10 mmol) of 4-nitrobenzyl 7-phenylacetamido-3-methyl-3-cephem-4-carboxylate and, dropwise over a period of 12 minutes, a solution of 1.01 ml (12.5 mmol) of pyridine in 4 ml of methylene chloride. The reaction mixture was stirred at −10 to −15° for another 15 minutes

after which time was added 2.1 ml of propylene oxide. The cooling bath was removed and the reaction mixture was stirred an additional 15 minutes as the temperature rose to about 0°C. The reaction mixture was washed with 25 ml of water, dried over calcium chloride dihydrate and evaporated *in vacuo* to a syrup which subsequently crystallized. The product thereby obtained was pulverized under 25 ml of diethyl ether containing six drops of propylene oxide, filtered, washed with ether, and dried in a vacuum at room temperature to provide 4.58 g (94.2%) of the title product as snow white crystals, m.p. 132—133°C.

nmr ($CDCl_3$), pyridine d-5) δ 2.18 (s, 3), 3.37 (ABq, 2, J=16 Hz), 3.96 (s, 2), 5.05 (d, 1, J=5 Hz), 5.37 (s, 2), 5.5 (d, 1, J=5 Hz), 7.3 (s, 5, ArH), and 7.4—8.4 (m, 4, ArH).

Anal calcd for $C_{23}H_{20}N_3O_5SCl$:

C, 56.35; H, 4.15; N, 8.65; S, 6.60; Cl, 7.30

Found: C, 56.60; H, 4.25; N, 8.83; S, 6.49; Cl, 7.07.

## Example 2

2′,2′,2′-Trichloroethyl 6-(1-chloro-2-phenylethylidene)imino penicillanate.

A solution of approximately 12.3 mmol of the triphenyl phosphite-chlorine complex in 45 ml of methylene chloride was prepared in accordance with the procedure described in Example 1. To this solution at −30°C was added 4.66 g (10 mmol) of 2′,2′,2′-trichloroethyl 6-phenylacetamido penicillanate. An additional 5 ml of methylene chloride was used to wash the penicillin ester into the reaction mixture. To the resulting solution was added dropwise over a 20 minute period a solution of 1.01 ml (12.5 mmol) of pyridine in 4 ml of methylene chloride. The reaction mixture was stirred at −20 to −30°C for about 15 minutes after which time 2.1 ml of propylene oxide was added to the mixture to destroy any HCl or excess chlorinating reagent remaining in the reaction mixture. After the reaction mixture was allowed to warm to approximately 0°C over a 15 minute period, the solution was washed with 25 ml of ice water and dried over calcium chloride dihydrate. Evaporation *in vacuo* of the dried solution provided 11 g of an oil which crystallized upon the addition of about 1 ml of diethyl ether. An additional 25 ml of diethyl ether containing 4 drops of propylene oxide was added to the crystallized product. After stirring at room temperature for 5 minutes, the white crystalline product was filtered, washed with 25 ml of diethyl ether and dried under reduced pressure at room temperature. 2.52 Grams of the title product was obtained: m.p. 84—85.5°C. An addition crop of 1.06 gm of the title product was obtained by evaporating the filtrate *in vacuo* to 12 gram slurry which was diluted with 20 ml of a 1:1 ether, hexane solution. Total yield — 74%.

nmr ($CCl_4$) δ 1.56 (s, 3), 1.68 (s, 3), 3.96 (s, 2), 4.57 (s, 1), 4.8 (s, 2), 5.3 (d, 1, J=4 Hz), 3.93 (d, 1, J=4 Hz) and 7.3 (s, 5).

Anal calcd for $C_{18}H_{18}N_2O_3SCl_4$:

C, 44.65; H, 3.75; N, 5.78; S, 6.62; Cl, 29.29

Found: C, 44.76; H, 3.84; N, 5.90; S, 6.17; Cl, 29.06.

## Example 3

4′-Nitrobenzyl 7-(1-chloro-2-phenoxyethylidene)imino-3-chloro-3-cephem-4-carboxylate

A solution of about 12.3 mmol of the triphenyl phosphite chlorine-complex in 45 ml of methylene chloride was prepared in accordance with the procedure described in Example 1. To this solution at −15°C. was added 5.04 g (10 mmol) of 4′-nitrobenzoyl 7-phenoxy-acetamido-3-chloro-3-cephem-4-carboxylate which was washed into the reaction mixture with an additional 5 ml of methylene chloride. Immediately a solution of 1.01 ml (12.5 mmol) of pyridine in 4 ml of methylene chloride was added dropwise over a 15 minute period to the reaction mixture. After the reaction mixture was stirred for an additional 15 minutes at −10 to −15°C, 2.1 ml of propylene oxide was added. The cooling bath was removed, and the temperature of the mixture was allowed to rise to about 0° over a 15 minute period after which time the reaction mixture was washed with 25 ml of ice water, dried over calcium chloride dihydrate and subsequently evaporated *in vacuo* to about 20 grams of a syrup. No crystals were observed after the addition of about 50 ml of diethyl ether to the product residue. After the ether was decanted from the product residue, the product residue was further dried *in vacuo* to 11 grams of a thick oil. This product residue was washed three times with 50 ml portions of 1:1 ether, hexane. Trituration of the resulting thick oil with 25 ml of diethyl ether resulted in crystallization of the product. The crystallized product was filtered, washed with ether, and vacuum dried at room temperature to provide 3.58 grams (68.6%) of the title product as light colored crystals: m.p. 94—97°C.

nmr ($CDCl_3$, pyridine d-5) δ 3.56 (ABq, 2, J=18 Hz), 4.8 (s, 2), 5.13 (d, 1, J=5 Hz), 5.3 (s, 2), 5.53 (bd, 1, J=5 Hz) and 6.8—8.3 (m, 9).

Anal calcd. for $C_{22}H_{17}N_3O_6SCl_2$:

C, 50.59; H, 3.28; N, 8.04; S, 6.14; Cl, 13.57

Found: C, 50.32; H, 3.36; N, 8.20; S, 5.92; Cl, 13.57.

Example 4

4'-Nitrobenzyl 6-(1-chloro-2-phenoxyethylidene)imino penicillanate.

A solution of 9.71 g (20 mmol) of 4'-nitrobenzyl 6-phenoxyacetamido penicillanate in 75 ml of methylene chloride was dried over calcium chloride dihydrate for about 15 minutes. The solution was filtered and evaporated to about 40 ml for addition to the TPP—C preparation. A solution of about 24.3 mmol of the triphenyl phosphite chlorine-complex in about 50 ml of methylene chloride was prepared at −15 to −20°C in accordance with the procedures described in Example 1. The solution of TPP—C was cooled to −40°C and the above-prepared solution of the penicillin ester was added. The temperature of the reaction mixture rose to about −22°C. A solution of 2.02 ml (25 mmol) of pyridine in 8 ml of methylene chloride was then added dropwise over a 15 minute period to the reaction mixture at −20 to −30°C. After stirring the mixture for about 15 minutes 4.2 ml of propylene oxide (60 mmol) was added. After the reaction mixture was allowed to warm to 0°C over about a 15 minute period, it was washed quickly with 50 ml of ice water and dried over calcium chloride dihydrate. The dried solution was filtered and evaporated under reduced pressure to about 27 grams of solution. Successively, a 50 ml volume of ether and two 20-ml volumes of carbon tetrachloride were added; the resulting solution was each time subsequently evaporated *in vacuo* to a product oil. The nuclear magnetic resonances spectrum of this crude product showed it to be the title product contaminated with triphenyl phosphate.

nmr ($CDCl_3$) δ 1.33 (s, 3), 1.46 (s, 3), 4.46 (s, 1), 4.8 (s, 2), 5.2 (s, 2), 5.3 (d, 1, J=4 Hz), 5.57 (d, 1, J=4 Hz), and 6.7—8.3 (m, 9).

Example 5

4'-Nitrobenzyl 7-(1-chloro-1-phenoxyethylidene)-imino-3-acetoxy-3-cephem-4-carboxylate

Chlorine gas was bubbled into 45 ml of methylene chloride cooled to −10°C simultaneously with the dropwise addition of 3.16 ml (12 mmol) of triphenyl phosphite. The co-addition of these reactants were monitored so as to maintain a slight yellow color (excess of chlorine) throughout the preparation until the last drop of phosphite added dissipated the yellow color. To the resulting solution was added 5.28 g (10 mmol) of 4'-nitrobenzyl 7-phenoxyacetamido-3-acetoxy-3-cephem-4-carboxylate which was washed into the reaction mixture with 5 ml of methylene chloride. Thereafter 1.01 ml (12.5 mmol) of pyridine in 5 ml of methylene chloride was added dropwise over a 15 minute period to the reaction mixture at −10°C. After stirring the mixture for an additional 15 minutes at −10°C, 2.1 ml (30 mmol) of propylene oxide was added. After stirring for 10 minutes at 0°C the mixture was washed with 50 ml of ice water, dried over calcium chloride, and evaporated *in vacuo* to an oil. Attempts to crystallize the product from ether failed. After evaporating at reduced pressure all solvents from the product residue, 25 ml of carbon tetrachloride was added and the resulting solution again evaporated to dryness. An nmr spectrum of the unpurified product showed it to be the title iminochloride.

nmr ($CDCl_3$) δ 2.06 (s, 3), 3.41 (ABq, 2, J=18 Hz), 4.83 (s, 2), 5.05 (d, 1, J=5 Hz), 5.28 (s, 2), 5.56 (bd, 1, J=5 Hz) and 6.8—8.3 (m, ArH).

Example 6

4'-Nitrobenzyl 7-[1-chloro-2-(2-thienyl)ethylidene]-imino-3-methyl-3-cephem-4-carboxylate

Following the procedure described in Example 1 a solution of about 12 mmol of the triphenyl phosphite chlorine-complex in 45 ml of methylene chloride was prepared. To that solution at −10°C was added 4.74 g (10 mmol) of 4'-nitrobenzyl 7-(2-thienylacetamido)-3-methyl-3-cephem-4-carboxylate which was washed into the reaction mixture with an additional 5 ml of methylene chloride. After 5 minutes 1.01 ml (12.5 mmol) of pyridine in 5 ml of methylene chloride were added dropwise over a 20 to 30 minute period. The reaction mixture was stirred at −10°C for about 30 minutes after which time the reaction mixture was allowed to warm to room temperature and to stir for approximately 2 hours. To the reaction mixture was then added 2.1 ml (30 mmol) of propylene oxide. After 10 minutes the mixture was washed with 50 ml of ice water, dried over calcium chloride dihydrate, and evaporated under reduced pressure to an oil. The product oil was crystallized by adding a 1:1 mixture of methylene chloride and ether. Filtration of the crystallizing mixture provided 2.03 grams (41.3%) of the title product: m.p. 129—132°C. An additional 1.95 grams (39.6%) of the title product was obtained by evaporation of the filtrate from the crystallizing mixture. Total yield — 80.9%.

nmr ($CDCl_3$) δ 2.16 (s, 3), 3.33 (ABq, 2, J=18 Hz), 4.16 (s, 2), 5.03 (d, 1, J=4 Hz), 5.33 (s, 2), 5.5 (bd, 1, J=4 Hz), and 6.8—8.4 (m, ArH).

Anal. calcd. for $C_{21}H_{18}N_3O_5S_2Cl$:

C, 51.27; H, 3.69; N, 8.54; S, 13.03

Found: C, 51.30; H, 3.72; N, 8.31; S, 12.91

Example 7

4′-Nitrobenzyl 7-(α-chlorobenzylidene)imino-3-methyl-3-cephem-4-carboxylate

A solution of the triphenyl phosphite chlorine complex was prepared in 45 ml of methylene chloride using 3.16 ml (12 mmol) of triphenyl phosphite in accordance with the procedures described in Example 1 above. To this solution at −10°C. were added 4.14 g (10 mmol) of 4′-nitrobenzyl 7-benzamido-3-methyl-3-cephem-4-carboxylate and 1.01 ml (12.5 mmol) of pyridine. The reaction mixture was removed from the ice bath and immediately exothermed to 0°C. After stirring the reaction mixture for about 3 minutes the iminochloride began to crystallize. After one hour at room temperature the reaction mixture was filtered to provide a crystalline product which was washed with ether and dried. 2.28 g (48.3%) of the title product was isolated: m.p. 175°C.

The filtrate from above was diluted with methylene chloride and washed successively with dilute HCl and sodium chloride solutions and subsequently dried over calcium chloride dihydrate. Evaporation *in vacuo* of the resulting dried solution gave an oil which, upon trituration with diethyl ether, provided a second crop of crystals of the title product which were filtered, washed with ether and dried. 1.72 g (36.4%) of the title product was isolated from the filtrate. Total yield — 84.7%.

nmr ($CDCl_3$) δ 2.20 (s, 3), 3.43 (ABq, 2, J=18 Hz), 5.15 (d, 1, J=5 Hz), 5.37 (s, 2), 5.75 (d, 1, J=5 Hz) and 7.2—8.4 (m, ArH).

Anal. calcd. for $C_{22}H_{18}N_3O_5SCl$:

| | | | | | |
|---|---|---|---|---|---|
| | C, 55.99; | H, 3.84; | N, 8.90; | S, 6.79; | Cl, 7.51 |
| Found: | C, 56.16; | H, 4.06; | N, 9.00; | S, 6.54; | Cl, 7.67. |

Example 8

4′-Nitrobenzyl 7-(1-chloro-2-phenoxyethylidene)-imino-3-methyl-3-cephem-4-carboxylate

A solution of the triphenyl phosphite-chlorine complex was prepared in 45 ml of methylene chloride from 3.95 ml (15 mmol) of triphenyl phosphite and chlorine in accordance with the procedures described in Example 1. To this solution was added 4.84 g (10 mmol) of 4′-nitrobenzyl 7-phenoxyacetamino-3-methyl-3-cephem-4-carboxylate which was washed into the reaction mixture with 5 ml of methylene chloride. Then 1.3 ml (15.6 mmol) of pyridine in 8 ml of methylene chloride was added dropwise over a 30 minute period to the reaction mixture at −10°C. The reaction mixture was then removed from the ice bath and allowed to stir for 30 minutes after which time 2.1 ml (30 mmol) of propylene oxide was added. After 10 minutes the reaction mixture was washed with 50 ml of ice water, dried over calcium chloride dihydrate, and evaporated *in vacuo* to an oil which crystallized with the addition of 50 ml of diethyl ether. Filtration provided 3.44 grams (68.6%) of the title product: m.p. 110°—111°C.

nmr ($CDCl_3$, pyridine d-5) δ 2.16 (s, 3), 3.26 (ABq, 2, J=18 Hz), 4.83 (s, 2), 5.01 (d, 1, J=5 Hz), 5.28 (s, 2), 5.52 (bd, 1, J=5 Hz) and 6.7—8.2 (m, ArH).

Example 9

4′-Nitrobenzyl 7-(1-chloro-2-phenoxyethylidene)-imino-3-methylenecephem-4-carboxylate/4′-nitrobenzyl 7-amino-3-methylenecephem-4-carboxylate hydrochloride

A solution of about 12.3 mmol of triphenyl phosphite-chlorine compound was prepared in accordance with the procedures described in Example 1. To that solution were added 4.84 g (10 mmol) of 4′-nitrobenzyl 7-phenoxyacetamido-3-methylenecephem-4-carboxylate and a solution of 1.01 ml (12.5 mmol) of pyridine in 4 ml of methylene chloride dropwise over a 15 minute period. The reaction was stirred for about 15 minutes at −10 to −15°C before adding 2.1 ml (30 mmol) of propylene oxide. After 15 minutes the reaction was washed quickly with 25 ml of ice water, dried over calcium chloride dihydrate for about 5 minutes, and evaporated *in vacuo* to provide about 11 grams of a thick oil which was dissolved in 25 ml of carbon tetrachloride. An nmr spectrum of the product obtained by evaporating the carbon tetrachloride solution showed the product to be the title iminochloride contaminated only with triphenyl phosphate.

nmr ($CCl_4$) δ 3.4 (ABq, 2), 4.87 (s, 2), 5.30 (m, 3), 5.45 (s, 2) and 6.7—8.4 (m, ArH).

The unpurified iminochloride was dissolved in 50 ml of methylene chloride and treated with 5.1 ml (55 mmol) of isobutanol and HCl gas. The temperature of the reaction mixture rose from about 20 to about 30°C before a cooling bath was applied to the crystallizing mixture. After two hours at room temperature the product was filtered, washed, and dried to provide 3.58 grams (92.7%) of near white crystals of 4′-nitrobenzyl 7-amino-3-methylenecepham-4-carboxylate hydrochloride: m.p. 180—181°C.

nmr (DMSO d-6) δ 3.67 (bs, 2), 5.0 (d, 1, J=5 Hz), 5.35—5.53 (m, 6), and 7.6—8.4 (m, ArH).

Example 10

4'-Nitrobenzyl 7-amino-3-methyl-3-cephem-4-carboxylate hydrochloride

To a solution of 4.1 ml of isobutanol (44 mmol) in 40 ml of methylene chloride at 25°C. was added 2.89 g (8 mmol) of 4'-nitrobenzyl 7-(1-chloro-2-phenylethylidene)imino-3-methyl-3-cepham-4-carboxylate prepared in Example 1. The resulting solution was treated with HCl gas at a moderate rate for about 1 minute and 15 seconds. The title nucleus ester hydrochloride began precipitating as a gelatinous solid which soon crystallized and filled the solution as a paste. Because stirring was inefficient the reaction mixture was diluted with an additional 40 ml of methylene chloride. The resulting diluted alcoholysis mixture was stirred at room temperature for 2 hours and thereafter filtered to provide 2.52 grams (81.6%) of the title product: m.p. 183.5°C. The filtrate when treated with HCl gas yielded an additional 0.47 grams of the title product (m.p. 183.5°C). Combined yield for the alcoholysis — 96.8%.

nmr (DMSO d-6) $\delta$ 2.21 (s, 3), 3.65 (ABq, 2, J=16 Hz); 5.18 (q, 2, J=4 Hz, $\beta$-lactam H), 5.41 (s, 2), and 7.6—8.4 (m, ArH).

Example 11

4'-Nitrobenzyl 7-amino-3-methyl-3-cephem-4-carboxylate hydrochloride

(A) From 4'-Nitrobenzyl 7-phenoxyacetamido-3-methyl-3-cephem-4-carboxylate.

A solution of the triphenyl phosphite-chlorine complex was prepared by bubbling chlorine through a solution of 2.89 ml (11 mmol) of triphenyl phosphite in 50 ml of methylene chloride at —15°C. To this solution were added 5.02 g (10 mmol) of 4'-nitrobenzyl 7-phenoxyacetamido-3-methyl-3-cephem-4-carboxylate and 0.85 ml (11.5 mmol) of pyridine. The reaction mixture was stirred for 1 hour at —15 to —10°C after which time was added 6.0 ml (64.8 mmol) of isobutanol. The cooling bath was removed, and the reaction mixture was allowed to warm to room temperature over a 2 hour period. The titled nucleus hydrochloride ester, which began to crystallize in about 15 minutes, was filtered, washed with methylene chloride, and dried. A total of 3.55 grams (92%) of the title product was obtained as white crystals: m.p. 189°C. (decomp.).

(B) From 4'-Nitrobenzyl 7-heptanoylamido-3-methyl-3-cephem-4-carboxylate.

The experimental procedure described in Paragraph A above was repeated in detail using 4.61 g (10 mmol) of 4'-nitrobenzoyl 7-heptanoylamido-3-methyl-3-cephem-4-carboxylate as the substrate. A total of 6.32 grams (93.8%) of the nucleus ester hydrochloride as snow white crystals was isolated: m.p. 188.5°C (decomp.).

(C) From 4'-Nitrobenzyl 7-phenoxyacetamido-3-methyl-3-cephem-4-carboxylate in tetrahydrofuran.

A solution of the triphenyl phosphite-chlorine complex was prepared by bubbling chlorine into a solution of 11 mmol of triphenyl phosphite in tetrahydrofuran (THF) at —10°C. To the solution was added 4.84 g (10 mmol) of 4'-nitrobenzyl 7-phenoxyacetamido-3-methyl-3-cephem-4-carboxylate. Subsequently 0.95 ml (11 mmol) of pyridine were added to the reaction mixture. The reaction was then allowed to stir at —10°C for 1 hour after which time it was allowed to warm to room temperature and stir for another 2 hours. Then 6.0 ml (65 mmol) of isobutanol was added. After 2 hours the reaction mixture was filtered. The crystalline nucleus hydrochloride ester thereby obtained was washed with THF and dried. Total yield — 3.03 grams (78.5%): m.p. 151—153°C (dec.).

(D) From 4'-Nitrobenzyl 7-phenoxyacetamido-3-methyl-3-cephem-4-carboxylate in acetonitrile.

A solution of triphenyl phosphite-chlorine complex was prepared by bubbling chlorine into a solution of about 11 mmol of triphenyl phosphite in 45 ml of acetonitrile at —10°C. To this solution were added 4.84 g (10 mmol) of 4'-nitrobenzyl 7-phenoxyacetamido-3-methyl-3-cephem-4-carboxylate and subsequently 0.95 ml (11 mmol) of pyridine at —10°C. After the reaction mixture was allowed to stir for 2 hours at —10°C the ice bath was removed. After an additional 2 hours, 6.0 ml (65 mmol) of isobutanol was added to the reaction mixture. With seeding the product crystallized, and after stirring for 1 hour, it was filtered, washed with acetonitrile, and dried. Total yield — 2.55 grams (66.1%): m.p. 184°C (dec.).

(E) From 4'-Nitrobenzyl 7-phenoxyacetamido-3-methyl-3-cephem-4-carboxylate in ethyl acetate.

The same procedure was followed as described in Paragraph D above except that ethyl acetate was used as a solvent for the triphenyl phosphite-chlorine reagent formation and for the cleavage process. Total yield 2.48 grams (64.2%): m.p. 177—179°C (dec.).

(F) From 4'-Nitrobenzyl 7-phenoxyacetamido-3-methyl-3-cephem-4-carboxylate using tri-o-tolyl phosphite-chlorine complex.

A solution of tri-o-tolyl phosphite-chlorine complex was prepared as follows: 3.91 g (11 mmol) of tri-o-tolyl phosphite was added to 45 ml of methylene chloride and cooled to —10°C under a nitrogen atmosphere. Chlorine gas was bubbled into the solution until the yellow color persisted. Then about 0.5 mmol of tri-o-tolyl phosphite was added to discharge the yellow color. To the solution were added 4.84 g (10 mmol) of 4'-nitrobenzyl 7-phenoxyacetamido-3-methyl-3-cephem-4-carboxylate and 1.01 ml (12.5 mmol) of pyridine. The reaction mixture was removed from the cooling bath and stirred for 90 minutes after which time 5.1 ml (55 mmol) of isobutanol was added. The product began to crystallize about 5 minutes after gaseous HCl was bubbled into the reaction mixture. After 90 minutes the

reaction mixture was filtered. The product was washed with 25 ml of methylene chloride and dried at reduced pressure. Total yield — 3.46 grams (89.6%): m.p. 184°C (dec.).

(G) From 4'-Nitrobenzyl 7-phenoxyacetamido-3-methyl-3-cephem-4-carboxylate using divinylbenzene-vinylpyridine copolymer as the base.

A solution of the triphenyl phosphite-chlorine kinetic complex was prepared in 50 ml of methylene chloride at −10°C by first bubbling chlorine through the solution and then adding dropwise triphenyl phosphite at such a rate that the yellow of the chlorine always persisted. When the dropwise addition of the triphenyl phosphite was about complete, the addition of chlorine was discontinued. Triphenyl phosphite was then added until the solution decolorized. A total of 3.0 ml (11.4 mmol) of triphenyl phosphite was used. To this solution was added 5.0 g (10.3 mmol) of 4'-nitrobenzyl 7-phenoxyacetamido-3-methyl-3-cephem-4-carboxylate followed immediately by 5.0 g of divinylbenzene-vinylpyridine copolymer. The reaction mixture was removed from the cooling bath and stirred for 2 hours at room temperature. The polymer was then filtered and washed with about 20 ml of methylene chloride. The filtrate was treated with 6.0 ml (64.8 mmol) of isobutanol. HCl gas was then bubbled through the mixture for about 2 minutes. The nucleus hydrochloride product began to crystallize in about 3 minutes, and after one hour, was filtered from the mixture, washed with methylene chloride and dried. A total of 2.98 grams (75%) of the nucleus hydrochloride ester was isolated: m.p. 183°C (dec.).

(H) From 4'-Nitrobenzyl 7-phenoxyacetamido-3-methyl-3-cephem-4-carboxylate using tri(p-methoxyphenyl)phosphite-chlorine complex.

A solution of tri(p-methoxyphenyl)phosphite chlorine complex was prepared as follows: 4.6 g (11.5 mmol) of tri(p-methoxyphenyl)phosphite in about 5 ml of methylene chloride was added dropwise to 45 ml of methylene chloride at −10 to −20°C with simultaneous addition of chlorine to a colorless endpoint. After the addition of all of the phosphite reagent, additional chlorine was added to give a faint yellow color; the color of excess chlorine rapidly dissipated without adding more phosphite. To the resulting solution was added 4.84 g (10 mmol) of 4'-nitrobenzyl 7-phenoxyacetamido-3-methyl-3-cephem-4-carboxylate which was washed into the reaction mixture with 5 ml of methylene chloride. Subsequently a solution of 1.01 ml of pyridine (12.5 mmol) in 4 ml of methylene chloride was added dropwise to the reaction mixture over a 15 minute period. After stirring the reaction mixture for 15 minutes at −10°, 5.1 ml of isobutanol (55 mmol) was added to the reaction mixture. HCl gas was bubbled into the reaction mixture, and shortly thereafter the cooling bath was removed. After 2 hours at room temperature the reaction mixture was filtered to provide 0.89 grams (23%) of the nucleus hydrochloride ester: m.p. 173—174°C.

(I) From 4'-Nitrobenzyl 7-phenoxyacetamido-3-methyl-3-cephem-4-carboxylate using triethylamine as the base.

A solution of the triphenyl phosphite-chlorine kinetic complex was prepared by adding chlorine gas simultaneously with 3.16 ml (12 mmol) of triphenyl phosphite to 45 ml of methylene chloride at −10°C. A slight yellow color was maintained throughout the preparation. An additional 0.5 mmol of triphenyl phosphite was added to dissipate the yellow chlorine color. To the resulting solution was added 4.84 g (10 mmol) of 4'-nitrobenzyl 7-phenoxyacetamido-3-methyl-3-cephem-4-carboxylate which was washed into the mixture with 5 ml of methylene chloride. After 5 minutes 1.8 ml (13 mmol) of triethylamine in 8 ml of methylene chloride were added over a 15 minute period. After stirring the reaction mixture for 15 minutes at −10°C, the cooling bath was removed from the reaction mixture and 5.1 ml (55 mmol) of isobutanol was added. Thereafter HCl gas was bubbled into the reaction mixture for about 3 minutes. The reaction mixture was seeded and allowed to warm to room temperature. After 2 hours at room temperature the reaction mixture was filtered to provide 1.28 grams (33.2%) of the nucleus hydrochloride ester: m.p. 180.5°C (dec.).

(J) From 4'-Nitrobenzyl 7-phenoxyacetamido-3-methyl-3-cephem-4-carboxylate using DBU as the base.

The same experimental procedure was followed as described in Paragraph I above except that 1.95 ml (13 mmol) of 1,5-diazabicyclo[5.4.0]undec-5-ene (DBU) was used in place of the triethylamine base. 0.59 g (15.3%) of nucleus ester hydrochloride product was isolated: m.p. 181°C (dec.).

(K) From 4'-Nitrobenzyl 6-phenoxyacetamido penicillanate, 1-oxide.

A solution of 5.02 g (10 mmol) of 4'-nitrobenzyl 6-phenoxyacetamido penicillinate, 1-oxide and 0.25 g (1 mmol) of pyridinium dichloromethane-phosphonate in 88 ml of 1,1,2-trichloroethane was heated to reflux for 4 hours. The reaction mixture was evaporated *in vacuo* to a volume of about 44 ml.

A solution of 12 mmol of the triphenyl phosphite chlorine reagent was prepared by bubbling chlorine gas into a solution of 3.15 ml of triphenyl phosphite in 44 ml of 1,1,2-trichloroethane at −10°C. The chlorine gas was bubbled into the solution until a yellow color persisted. The yellow color was then discharged by the addition of a drop of triphenyl phosphite.

The solution from the first paragraph above was then added to the solution (at −10°C) of the triphenyl phosphite-chlorine reagent. Thereafter 0.89 ml (11 mmol) of pyridine was added to the reaction mixture at −10°C. After 30 minutes at that temperature the reaction mixture was removed from the ice bath and allowed to warm to room temperature. After 30 minutes an additional 0.43 ml (5 mmol) of pyridine was added. After stirring the reaction mixture for an additional 30 minutes, 9.25 ml

(100 mmol) of isobutanol was added. The product crystallized as the reaction mixture was stirred overnight. Filtration provided 2.67 grams (69.2%) of the nucleus ester hydrochloride: m.p. 183°C (dec.).

(L) From 4′-Nitrobenzyl 6-phenoxyacetamido penicillanate, 1-oxide using 2,6-lutidiene as the base.

The same experimental procedure was followed as described in Paragraph K above except that 1.25 ml (11 mmol) of 2,6-lutidine was used in place of pyridine. Also HCl gas was bubbled into the reaction mixture for about 60 seconds after the addition of isobutanol. The product began to crystallize in about 2 to 3 minutes after the HCl addition. A total of 2.47 g (64%) of the nucleus ester hydrochloride was isolated: m.p. 173°C (dec.).

Example 12

4′-Nitrobenzyl 7-amino-3-methoxy-3-cephem-4-carboxylate, hydrochloride.

Chlorine was bubbled through a stirred solution of 0.4 ml (1.5 mmol) of triphenyl phosphite in 10 ml of methylene chloride at −10°C until the light yellow green color of excess chlorine persisted. One small drop of triphenyl phosphite discharged the color completely. To the resulting solution was added 0.5 g (1 mmol) of 4′-nitrobenzyl 7-phenoxyacetamido-3-methoxy-3-cephem-4-carboxylate followed by 0.12 ml (1.5 mmol) of pyridine. The reaction mixture was removed from the cooling bath and stirred 1.5 hours at room temperature after which time 0.6 ml (6.4 mmol) of isobutanol was added. The title nucleus hydrochloride begin to crystallize from the reaction mixture within 5 minutes after the addition of the alcohol. After 1.5 hours the reaction mixture was filtered to provide 0.3 g (75%) of the title product as off-white crystals: m.p. 185°C (dec.).

nmr (DMSO d-6) $\delta$ 3.92 (bs, 2), 4.0 (s, 3), 5.02 (d, 1, J=5 Hz), 5.32 (d, 1, J=5 Hz), 5.45 (s, 2) and 7.6—8.4 (m, ArH).

Anal. calcd. for $C_{15}H_{16}N_3O_6SCl$:

| | | | | | |
|---|---|---|---|---|---|
| | C, 44.84; | H, 4.01; | N, 10.46; | Cl, 8.82; | S, 7.98. |
| Found: | C, 44.69; | H, 4.17; | N, 10.34; | Cl, 9.05; | S, 7.77. |

Example 13

4′-Nitrobenzyl 7-amino-3-methylenecepham-4-carboxylate, hydrochloride

To a solution of 5.02 g (10 mmol) of 4′-nitrobenzyl 7-phenoxyacetamido-3-methylenecepham-4-carboxylate, 1-oxide and 2.4 ml (22.5 mmol) of amylene in 50 ml of methylene chloride at 15°C was added dropwise over 10 minutes 1.67 ml (22.5 mmol) of acetyl bromide. The reaction mixture was cooled to 0°C, 25 ml of ice water were added, and the reaction mixture was then allowed to stir for 30 minutes. The methylene chloride layer was separated, washed successively with 25 ml of water and 25 ml of dilute sodium chloride solution, dried over anhydrous sodium sulfate and evaporated *in vacuo* to a volume of 25 ml.

A solution of the triphenyl phosphite-chlorine kinetic complex was prepared by bubbling chlorine gas into a solution of 2.89 ml (11 mmol) of triphenyl phosphite in 25 ml of methylene chloride at −10°C until the yellow color persisted. Another 0.12 ml (0.46 mmol) of triphenyl phosphite was added to the solution to discharge the yellow color. To the resulting solution at −10°C was added a solution prepared in the foregoing paragraph. Then 0.93 ml (11.5 mmol) of pyridine was added. The reaction mixture was then removed from the ice bath and allowed to warm to room temperature. After 1 hour, 5.1 ml (55 mmol) of isobutanol was added to the reaction mixture. The product began to crystallize in the reaction mixture after about 10 minutes. After stirring the reaction mixture 90 minutes at room temperature, it was filtered to provide 3.17 g (82.1%) of the title nucleus ester hydrochloride: m.p. 182°C (dec.).

nmr (DMSO d-6) $\delta$ 3.6 (bs, 2), 4.95 (d, 2, J=5 Hz), 5.33—5.7 (m, 6), and 7.6—8.4 (m, ArH).

Example 14

Benzhydryl 7-amino-3-acetoxymethyl-3-cephem-4-carboxylate.

To a solution of 1.39 g (1.5 mmol) of 2,4-dichlorobenzoyl cephalosporin C dibenzhydryl ester in 10 ml of methylene chloride at −35°C was added 0.484 ml of pyridine (6 mmol). To the resulting solution was added a solution of the triphenyl phosphite-chlorine reagent prepared at −10°C from 1.57 ml (6 mmol) of triphenyl phospite and chlorine in 10 ml of methylene chloride. After 150 minutes at about 18°C the reaction mixture was cooled to −5°C and treated with 3.0 ml of isobutanol. The reaction mixture was allowed to warm to a temperature of about 20°C, after which time the solvent was evaporated from the reaction mixture leaving a dark brown syrup. The resulting product residue was dissolved in 20 ml of methylene chloride and 10 ml of water. The pH of the aqueous layer was adjusted to 0.9 with HCl. The methylene chloride layer was then separated and extracted with water at pH 7.5. The methylene chloride layer was then dried over magnesium sulfate and evaporated *in vacuo* to about 3.5 gm of a very dark brown syrup which was dissolved in 3.5 ml of a 3:7 ethyl acetate-toluene solution and

applied to the surface of 40 grams of silica gel in a 9 mm column. Chromatography using at first a 3:7 ethyl acetate/toluene eluant mixture and then a 1:1 toluene:ethyl acetate eluant mixture provided a total .24 g (36%) of the title product.

Example 15

7-Amino-3-acetoxymethyl-3-cephem-4-carboxylic acid (7-ACA).

To a slurry of 2.94 g (5 mmol) of 2,4-dichlorobenzoyl cephalosporin C, 0.16 ml (1.34 mmol) of quinoline, and 2.39 ml (15 mmol) of N,N-diethylaniline in 30 ml of methylene chloride at room temperature were added 2.45 ml (34.5 mmol) of acetyl chloride. After the reaction mixture was cooled to −25°C, 0.6 ml (3.75 mmol) of diethyl aniline, and a solution of the triphenyl phosphite-chlorine reagent derived from 3.68 ml (14 mmol) of triphenyl phosphite in 15 ml methylene chloride were added. The reaction mixture was then removed from the cooling bath and allowed to warm to room temperature over a 2 hour period. After the mixture was cooled to −15°C, 8.5 ml (116 mmol) of propylene glycol was added. The reaction mixture was stirred for approximately ½ hour at 20°C after which time it was cooled to −15°C and then combined with 25 ml of ice water. The aqueous layer was separated, and its pH was adjusted to 3.5 with 3.3 ml of ammonium hydroxide. After stirring for 1½ hours in an ice bath the aqueous solution was filtered to provide 0.4 g (29%) of 7-ACA.

Example 16

7-Amino-3-methyl-3-cephem-4-carboxylic acid (7-ADCA).

To a slurry of 3.40 g (10 mmol) of 7-phenoxyacetamido-3-methyl-3-cephem-4-carboxylic acid, 0.158 ml (1.34 mmol) of quinoline, and 2.38 ml (15 mmol) of N,N-diethylaniline in 30 ml. of methylene chloride at room temperature was added 2.46 ml (34.5 mmol) of acetyl chloride. The reaction mixture was allowed to stir for about 6 hours at a temperature of 18 to 22°C. The reaction mixture was then cooled to −15°C. Then 0.6 ml (3.75 mmol) of N,N-diethylaniline and a solution of the triphenyl phosphite-chlorine reagent derived from 3.68 ml of triphenyl phosphite and chlorine in 15 ml of methylene chloride, were added. The reaction mixture was then removed from the cooling bath and allowed to warm to near room temperature over the next seven minutes. The reaction mixture was then cooled to −20°C. Then 10.7 ml (116 mmol) of isobutanol added. Again the reaction mixture was removed from the cooling bath. About 45 minutes after the addition of the alcohol, a copious amount of solid precipitate was observed. After an additional ½ hour at room temperature the reaction mixture was cooled to 0°C and filtered to provide 1.95 g (73%) of 7-ADCA. Some impurities were visible in an nmr spectrum of the product.

Example 17

7-Amino-3-acetoxymethyl-3-cephem-4-carboxylic acid (7-ACA).

To a slurry of 4.18 g (9.76 mmol) of 7-phenoxyacetamido-3-acetoxymethyl-3-cephem-4-carboxylic acid sodium salt, 0.154 ml (1.31 mmol) of quinoline and 2.91 ml (18.2 mmol) N,N-diethylaniline in 29 ml of methylene chloride at room temerature was added 2.40 ml (33.6 mmol) of acetyl chloride. After 1 hour and 15 minutes at room temperature the reaction mixture was cooled to −35°C. To this mixture was added a solution of the triphenyl phosphite-chlorine kinetic complex prepared from 3.6 ml (13 mmol) of triphenyl phosphite and chlorine in 15 ml of methylene chloride. The reaction mixture was stirred at −25 to −20°C for about 60 minutes after which time was added 10.5 ml of isobutanol. The mixture was allowed to warm to 0° at which temperature it was stirred for 2 hours. The reaction was then added to a mixture of 50 g of ice and water. The aqueous layer was separated and its pH was adjusted to 3.5. The aqueous solution was then stirred for 1 hour in an ice bath under a stream of nitrogen. Filtration of the aqueous solution provided 2.7 grams (78%) of 7-ACA.

Example 18

7-Amino-3-acetoxymethyl-3-cephem-4-carboxylic acid (7-ACA)

4.55 g of cephalosporin C sodium salt was suspended in 142 ml of amylene inhibited chloroform. The solution was distilled to a volume of 67 ml. The chloroform suspension of cephalosporin C sodium salt was then cooled to 26°C. To that solution were added .464 ml (3.94 mmol) of quinoline, 6.95 ml (43.5 mmol) of diethylaniline, and 9.30 ml (131 mmol) of acetyl chloride. The mixture was then warmed with stirring to about 35°C over a 7 minute period, after which time the heat source was removed. After stirring for 2 hours the reaction mixture was filtered using hyflo on paper over glass paper in a Buchner funnel.

A solution of the triphenyl phosphite-chlorine reagent prepared at −20°C by adding chlorine and triphenyl phosphite (8.9 ml, 34 mmol) simultaneously to 35 ml of chloroform, was added to a mixture of the filtrate from above at −30°C and 3.2 ml (20 mmol) of diethylaniline. The reaction mixture was stirred at −20 to −15°C for 60 minutes after which time it was cooled to −35°C. Propylene glycol (15 ml) was then added. After stirring the reaction mixture for 2 hours at 0°C, it was poured onto 51 g of ice. The chloroform layer was separated and extracted again with another 5 gm of ice water. The aqueous extracts were combined, and the pH was adjusted to 3.5 with approximately 7.5 ml of ammonium hydroxide. The aqueous solution was then stirred for 60 minutes in an ice bath with an air stream

blown over the surface to remove residual chloroform. The slurry was then filtered and the product washed successively with 6 ml of water, 15 ml of methanol and 5 ml of acetone. Total yield of the product 7-ACA (air dried) was 1.87 g (73%).

Example 19

7-Amino-3-acetoxymethyl-3-cephem-4-carboxylic acid (7-ACA)

(A) 4.8 g (10 mmol) of cephalosporin C, sodium salt, dihydrate was suspended in 80 ml of methylene chloride (cyclohexane stabilized, dried over 4A molecular sieves). Diethyl aniline (dried over KOH), 7.4 g (8 ml, 50 mmol), and acetyl chloride, 4.7 g (4.3 ml, 60 mmol), were added. The mixture was stirred in an ice bath at 30 to 40°C for 1 hour and then at room temperature for 2 hours. Filtration removed 1.65 g of undissolved material. The reaction solution was cooled in an ice-alcohol bath before addition to a solution of the triphenyl phosphite-chlorine kinetic compound prepared as follows: triphenyl phosphite, 6.8 g (5.8 ml, 22 mmol), was added to 100 ml of dry methylene chloride and cooled to ice-alcohol temperature before the addition of chlorine gas until a yellow coloration persisted. The addition of a few drops of triphenyl phosphite gave a colorless solution. After mixing the two above described solutions at ice alcohol temperature, diethyl aniline, 3.3 g (3.5 ml, 22 mmol), in 20 ml of dry methylene chloride was added dropwise over a period of 10 minutes. The reaction mixture was stirred in the cold for 2 hours, then cooled further to about −35°C and treated with isobutanol (dried over 3A molecular sieves), 6.0 g (7.4 ml, 80 mmol). A stream and dry hydrogen chloride was then passed through the reaction mixture for about 30 seconds. The reaction mixture was refrigerated overnight. Twenty ml of water was then added to the methylene chloride solution. The resulting 2-phase mixture was stirred vigorously for 5 minutes. The methylene chloride layer was separated and washed with 20 ml of water. The aqueous layer and the aqueous wash were combined, washed with ethyl acetate and then adjusted to pH 3.8 with saturated ammonium bicarbonate solution. After 30 minutes at ice bath temperature the aqueous slurry was filtered to provide 1.5 g (vacuum dried, 83%) of 7-ACA.

(B) Cephalosporin C, sodium salt, dihydrate, 4.8 g (10 mmol), was suspended in 80 ml of tetrahydrofuran (dried over 5A molecular sieves). Diethyl aniline (dried over KOH), 7.4 g (8.0 ml, 50 mmol), and acetyl chloride, 4.7 g (4.3 ml, 60 mmol), were added. The mixture was stirred in a water bath at about 30 to 40°C for 1 hour and then at room temperature for about 2.5 hours. Filtration removed 5.7 g of undissolved material. The reaction solution was cooled in an ice-alcohol bath before addition to a solution of the triphenyl phosphite-chlorine complex prepared as in Paragraph A above, but using tetrahydrofuran as a solvent instead methylene chloride. After mixing the two solutions, a solution of diethylaniline, 3.3 g (22 mmol) in 20 ml of dry tetrahydrofuran, was added dropwise over a period of 10 minutes. The reaction mixture was stirred in the cold for 2 hours, cooled further to about −35°C, and then treated with 16 ml of propylene glycol. A stream of dry hydrogen chloride was passed through the reaction for about 15 seconds. The reaction solution was refrigerated overnight. Workup as described in Paragraph A immediately hereinabove yielded 1.2 g (45%) of 7-ACA.

(C) N-Chloroacetyl Cephalosporin C, quinoline salt, monohydrate, 3.3 g (5 mmol), was suspended in 40 ml of methylene chloride (cyclohexane stabilized, dried over 4A molecular sieves). Diethylaniline (dried over KOH), 3.0 g (20 mmol), and acetyl chloride, 1.9 g (1.8 ml, 25 mmol), were added. The mixture was stirred at room temperature for 1 hour. The reaction solution was cooled in an ice-alcohol bath before addition to the triphenyl phosphite-chlorine complex, a solution of which was prepared as in Paragraph A immediately hereinabove using 3.4 g (11 mmol) of triphenyl phosphite.

After mixing the two solutions described in the foregoing paragraph, a solution of diethylaniline, 1.6 g (11 mmol), in 10 ml of dry methylene chloride was added dropwise over a period of 10 minutes. The reaction was stirred in the cold for 2 hours and then cooled further to about −35°C and treated with 3.7 ml of isobutanol (dried over 3A molecular sieves). A stream of hydrogen chloride was passed through the reaction solution for about 15 seconds. The reaction mixture was then refrigerated overnight. Following the workup procedure described in Paragraph A immediately hereinabove 730 mg (54%) of 7-ACA was isolated.

Example 20

2',2',2'-Trichloroethyl 7-amino-3-methyl-3-cephem-4-carboxylate, hydrochloride, in benzene

(A) Chlorine gas and 3.16 ml (12 mmol) of triphenyl phosphite were added simultaneously to 45 ml of benzene at 10 to 15°C. A slight yellow color was maintained in the reaction mixture until the last drop of phosphite added cleared the solution. To this solution was added 4.64 g (10 mmol) of 2',2',2'-trichloroethyl 7-phenylacetamido-3-methyl-3-cephem-4-carboxylate. After stirring the reaction mixture for 5 minutes at 10 to 15°C a solution of 1.1 ml (12.5 mmol) of pyridine in 8 ml of benzene was added over 15 minutes. After stirring the reaction mixture for a total of 45 minutes, 5.1 ml (55 mmol) of isobutanol was added, and HCl was bubbled into the reaction mixture for about 90 seconds. The title product crystallized while the reaction mixture was stirred at room temperature for a period of 2 hours. Filtration provided 3.5 g (91.6%) of titled nucleus ester hydrochloride: m.p. 179°C (dec.).

nmr (DMSO d-6) $\delta$ 2.27 (s, 3), 3.6 (Abq, 2 J=16 Hz), 5.00 (s, 2), and 5.12 (q, 2, J=4 Hz, $\beta$-lactam H).

(B) The same procedure was followed as described in Example 20 Paragraph A immediately hereinabove except that all preparations were conducted as room temperature (20—25°C) instead of 10—15°C. A total of 3.26 g (85.4%) of the title nucleus ester hydrochloride was isolated: m.p. 179°C (dec.).

Example 21

4'-Nitrobenzyl 7-amino-3-chloro-3-cephem-4-carboxylate hydrochloride

Chlorine gas was bubbled into a solution of 2.63 ml (10 mmol) of triphenyl phosphite in 50 ml of methylene chloride at 0 to 5°C until a yellow color was obtained. The excess chlorine, evidenced by the yellow color of the solution, was dissipated by adding triphenyl phosphite dropwise until the yellow color was discharged. This required an additional 0.47 ml (1.8 mmol) giving a solution of 11.8 mmol of the triphenyl phosphite-chlorine kinetic compound. To this solution were added 5.04 g (10 mmol) of 4-nitrobenzyl 7-phenoxyacetamido-3-chloro-3-cephem-4-carboxylate and a solution of 1.01 ml (12.5 mmol) of pyridine in 2 ml of methylene chloride. With the addition of the pyridine solution the temperature of the reaction mixture rose from 5 to 12°C. The solution was then allowed to stir at room temperature for 2 hours after which time was added 5.1 ml (55 mmol) of isobutanol. Within 10 minutes the title nucleus ester hydrochloride began to crystallize from the reaction mixture. After 1 1/2 hours the mixture was filtered to provide, after drying, 3.71 g (91.4%) of the title product as nearly white crystals: m.p. 180—181°C (dec.).

nmr (DMSO d-6) $\delta$ 3.7 (bs, 2), 5.33 (q, 2, $\beta$-lactam H), 5.46 (s, 2), and 7.5—8.4 (ArH).

Example 22

4'-Nitrobenzyl 7-amino-3-methyl-3-cephem-4-carboxylate, hydrochloride

Chlorine gas was added to a solution of 2.89 ml (11 mmol) of triphenyl phosphite in 50 ml of methylene chloride at 0 to 5°C until a yellow color persisted in the reaction mixture. Then an additional 0.17 ml (0.65 mmol) of triphenyl phosphite was added to discharge the yellow color. To the resulting solution at 0 to 5°C were added 4.84 g (10 mmol) of 4'-nitrobenzyl 7-phenoxyacetamido-3-methyl-3-cephem-4-carboxylate which was washed into the reaction mixture with 5 ml of methylene chloride. Then 1.01 ml (12.5 mmol) of pyridine were added to the reaction mixture resulting in an increase in the temperature from 5° to 10°C. The mixture was then allowed to warm to room temperature and to stir for 2 hours after which time was added 5.1 ml (55 mmol) of isobutanol alcohol. After about 20 minutes a trace of HCl was bubbled into the reaction mixture. The product began to crystallize immediately. After 2.5 hours the reaction mixture was filtered to provide after drying 3.29 g (85.3%) of the title nucleus ester hydrochloride: m.p. 177°C (dec.).

An additional 0.32 grams of the title product were isolated after treatment of the filtrate from above with additional HCl gas. Total yield of the title product was 93%.

Example 23

4'-Nitrobenzyl 7-amino-3-methyl-3-cephem-4-carboxylate, hydrochloride

Chlorine gas was bubbled into a solution of 2.89 ml (11 mmol) of triphenyl phosphite in 50 ml of methylene chloride and 5 to 10°C until the solution became a pale yellow color indicating excess chlorine. Two drops of triphenyl phosphite were added to discharge the color. To the resulting solution at 5 to 10°C was added 4.67 g (10 mmol) of 4'-nitrobenzyl 7-phenylacetamido-3-methyl-3-cephem-4-carboxylate followed by 0.85 ml (10.5 mmol) of pyridine. The solution was then allowed to warm to room temperature. After 2 hours the mixture was cooled to 15°C before 5.1 ml (55 mmol) of isobutanol was added. The reaction mixture was then stirred for 2 hours at room temperature during which time the product crystallized. Filtration provided, in 3 crops, a total of 3.5 g (90.6%) of the title nucleus ester hydrochloride: m.p. 188°C (dec.).

Example 24

4'-Nitrobenzyl 7-amino-3-methyl-2-cephem-4-carboxylate, hydrochloride

The same procedure was followed as described in Example 23 above except that 4'-nitrobenzyl 7-phenoxyacetamido-3-methyl-2-cephem-4-carboxylate, 4.84 g (10 mmol), was substituted as the substrate. A total of 3.27 g (84.7%) of the title nucleus ester hydrochloride were isolated: m.p. 184°C (dec.).

nmr (DMSO d-6) $\delta$ 1.96 (s, ), 5.12 (bs, 2), 5.4 (m), 6.34 (bs, 1), and 7.6—8.4 (ArH).

Example 25

4'-Nitrobenzyl 7-amino-3-methylenecepham-4-carboxylate, hydrochloride

The same procedure was followed as described in Example 23 above except 4'-nitrobenzyl 7-phenoxyacetamido-3-methylenecepham-4-carboxylate 4.83 g (10 mmol), was used as the substrate. A total of 3.58 g (92.8%) of titled nucleus ester hydrochloride were isolated: m.p. 176.5°—177°C (dec.). The nmr spectrum of the product was identical to that described for the product in Example 9 above.

Example 26

4'-Nitrobenzyl 7-amino-3-acetoxy-3-cephem-4-carboxylate, hydrochloride

Chlorine gas was bubbled through a solution of 2.89 ml (11 mmol) of triphenyl phosphite in 50 ml of methylene chlorine at 5 to 10°C until the yellow color of chlorine persisted. The color was then discharged by the addition of 3 drops of triphenyl phosphite. The cooling bath was removed before 5.28 g (10 mmol) of 4'-nitrobenzyl 7-phenoxyacetamido-3-acetoxy-3-cephem-4-carboxylate and 0.85 ml (10.5 mmol) of pyridine were added. The reaction was then stirred at room temperature for 2 hours after which time 6.0 ml (64.8 mmol) of isobutanol was added. Within 8 minutes the product began crystallizing from the reaction mixture. After 2 hours the mixture was filtered to provide 2.57 g (59.9%) of the title nucleus ester hydrochloride as bright white crystals: m.p. — 160°C (dec.). Additional product was noted in the filtrate but no attempt was made to isolate that material.

nmr (DMSO d-6) $\delta$ 2.2 (s, 3), 3.93 (bs, 2), 5.45 (m) and 7.6—8.4 (ArH).

Example 27

4'-Nitrobenzyl 7-amino-3-methyl-3-cephem-4-carboxylate hydrochloride using tri(p-chlorophenyl) phosphite-chlorine kinetic complex

To 5.17 g (12.5 mmol) of tri(p-chlorophenyl)phosphite and 0.27 ml (3.28 mmol) of pyridine in 25 ml of methylene chloride at −70°C was added chlorine gas. Amylene (0.40 ml) was added to discharge excess chlorine. To the resulting solution were added 4'-nitrobenzyl 7-phenoxyacetamido-3-methyl-3-cephem-4-carboxylate (2.42 g, 5 mmol) and pyridine (0.79 ml, 9.22 mmol) in 4 ml of methylene chloride dropwise over 11 minutes. After 3 hours the cooling bath was removed and 6.94 ml of isobutanol was added. After the reaction mixture had warmed to about −10°C HCl gas was bubbled into the mixture for about 1 minute. After 15 minutes the reaction mixture was filtered to give 1.86 g (96%) of the titled product as a white solid. m.p. 184—185°C (dec.).

Example 28

Benzyl 7-(1-chloro-2-phenylethylidene)imino-7-methoxy-3-acetoxymethyl-3-cephem-4-carboxylate

To a solution of the triphenyl phosphite-chlorine complex prepared from chlorine and 12.3 mmol of triphenyl phosphite in the presence of 0.1 ml of pyridine in 45 ml of methylene chloride at −15°C, was added 5.11 g (10 mmol) of benzyl 7-phenylacetamido-7-methoxy-3-acetoxymethyl-3-cephem-4-carboxylate and dropwise over 10 minutes a solution of 1.01 ml (12.5 mmol) of pyridine in 4 ml of methylene chloride. After 50 minutes at −15 to −10°C, 2.1 ml (30 mmol) of propylene oxide was used. After an additional 10 minutes (reaction temperature to 0°C), the reaction mixture was washed with 25 ml of ice water, dried over $CaCl_2$ and evaporated *in vacuo* to 11 g of syrup. The product was triturated 3 times under carbon tetrachloride and then taken up in 50 ml of ether. The etheral solution was decanted from .5 g of precipitate and then evaporated *in vacuo* to about 25 ml. An oily product was obtained with the resulting etheral solution was diluted with 25 ml of hexane. The oil was washed twice with 1:1/hexane:ether and then evaporated *in vacuo* to a foam twice from carbon tetrachloride solutions to provide 2.5 g of the title product:

ir ($CHCl_3$) 1780 and 1730 $cm^{-1}$.

nmr ($CDCl_3$, pyridine d-5) $\delta$ 1.96 (s, 3), 3.3 (ABq), 3.43 (s, 2), 3.93 (s, 2), 4.86 (ABq), 4.93 (s, 1), 5.25 (s, 1) and 7.3 (ArH).

Example 29

4'-Nitrobenzyl 7-amino-3-methyl-3-cephem-4-carboxylate hydrobromide

To a solution of 25.4 ml of triphenylphosphite-bromine complex prepared by reacting 6.67 ml (25.4 mmol) of triphenyl phosphite and 1.30 ml (25.4 mmol) of bromine in the presence of 2.10 ml (26 mmol) of pyridine in 100 ml of methylene chloride at −10 to −15°C was added 4'-nitrobenzyl 7-phenoxyacetamido-3-methyl-3-cephem-4-carboxylate (9.67 g, 20 mmol). After 1 hour at −10 to −15°C, the reaction mixture was removed from the cooling bath. Isobutanol (13.88 ml, 150 mmol) was added. After stirring for 2 hours at room temperature the reaction mixture was filtered to provide 4.76 g (55.3%) of the titled product. m.p. 179—181°C (dec.).

Anal calcd for $C_{15}H_{16}N_3O_5SBr$:

| | | | | | |
|---|---|---|---|---|---|
| | C, 41.87; | H, 3.75; | N, 9.77; | S, 7.45; | Br, 18.57. |
| Found: | C, 42.04; | H, 3.57; | N, 9.54; | S, 7.54; | Br, 18.37. |

nmr (DMSO d-6) $\delta$ 2.2 (s, 3), 3.65 (bs, 2), 5.27 (m, 2, $\beta$-lactam-H), 5.42 (s, 2), and 7.6—8.4 (m, 4, ArH).

Example 30

Benzhydryl 7-($\alpha$-chloro-4-methylbenzylidenimino)-7-methoxy-3-(1-methyl-1,2,3,4-tetrazol-5-ylthio)methyl-1-dethia-1-oxa-3-cephem-4-carboxylate

To a solution of 200 mg of benzhydryl 7-(4-methylbenzamido)-7-methoxy-3-(1-methyl-1,2,3,4-tetrazol-5-ylthio)methyl-1-dethia-1-oxa-3-cephem-4-carboxylate in 10 ml of deuterochloroform at 0 to

$-15°C$ was added, over a period of several hours, 4 equivalents of triphenyl phosphite-chlorine complex (prepared in usual manner) and 4 equivalents of pyridine. The large excess of the complex and pyridine was required probably because of impurities in the oxa cephem starting material. Precipitation of salts and impurities with $CCl_4$ and then with ether give oils on evaporation of the solvent. A nmr spectrum of the oil from the ether extract showed signals for triphenyl phosphite in addition to those for the title product.

nmr ($CDCl_3$) $\delta$ 2.25 (s, 3), 3.53 (s, 3), 3.65 (s, 3), 4.16 (s, 2), 4.53 (bs, 2) and 5.16 (s, 1, C-6 H).

### Example 31

4'-Nitrobenzyl 7-phenylacetamido-3-chloro-3-cephem-4-carboxylate

Chlorine was bubbled through a solution of 2.89 ml (11 mmol) of triphenyl phosphite in 50 ml of methylene chloride at $-15°C$ until the yellow color indicative of excess chlorine persisted. The color was then discharged by the addition of 2 drops of triphenyl phosphite. To the resulting solution of the triphenyl phosphite-chlorine reagent were added 4.54 g (10 mmol) of 4'-nitrobenzyl 7-phenylacetamido-3-hydroxy-3-cephem-4-carboxylate and, dropwise over a 40 minute period, a solution of 0.89 ml (11 mmol) of pyridine in 8 ml of methylene chloride. During the pyridine solution addition the temperature of the reaction mixture was maintained at $-15$ to $-10°C$. The reaction mixture was then stirred at $-15$ to $-10°C$ for an additional 60 minutes after which time the reaction mixture was removed from the cooling bath. Then 1 ml of conc. HCl was added to the mixture to effect hydrolysis of the small amount of imino chloride which had been formed. After stirring the reaction mixture for 30 minutes at room temperature the mixture was diluted with 100 ml of 3A ethanol, stirred 15 minutes, and then filtered to provide 2.67 grams (54.7%) of the title product as white crystals: m.p. 214°C (decomp.). A second crop of the title product was obtained by concentrating the filtrate under a reduced pressure to a volume of about 50 ml. An additional 1.52 grams (31.1%) of the title product was isolated. Total yield — 85.8%.

nmr (DMSO d-6) $\delta$ 3.62 (s, 2), 3.94 (ABq, 2, J=18 Hz), 5.3 (d, 1, J=5 Hz), 5.52 (s, 2), 5.82 (q, 1, J=5 and 8 Hz) and 7.2—8.4 (ArH).

Anal calcd for $C_{22}H_{18}N_3O_6SCl$:

| | | | | | |
|---|---|---|---|---|---|
| | C, 54.16; | H, 3.72; | N, 8.61; | Cl, 7.27; | S, 6.57. |
| Found: | C, 53.91; | H, 3.92; | N, 8.44; | Cl, 7.27; | S, 6.55. |

### Example 32

4'-Nitrobenzyl 7-phenoxyacetamido-3-chloro-3-cephem-4-carboxylate

Following the procedure of Example 31 the triphenyl phosphite-chlorine kinetic product was prepared from 6.31 ml of triphenyl phosphite and chlorine in 45 ml of methylene chloride at $-15°C$. To this solution at $-15$ to $-10°C$ 5.24 g (10 mmol) of 4'-nitrobenzyl 7-phenoxyacetamido-3-hydroxy-3-cephem-4-carboxylate was added and washed into the reaction mixture with 5 ml of methylene chloride. Then 1.01 ml (12.5 mmol) of pyridine in 8 ml of methylene chloride was added dropwise to the solution over a 30 minutes period. After stirring the reaction mixture for 2 hours at $-10°C$ 1 ml of conc. HCl was added. After stirring an additional 30 minutes the reaction mixture was washed with three 100 ml-portions of water, dried over magnesium sulfate, and evaporated *in vacuo* to an oil which was subsequently crystallized from 100 ml of 2B ethanol to provide 4.19 grams (83.2%) of the title product: m.p. 142.5°—146°C.

nmr ($CDCl_3$) $\delta$ 3.7 (ABq, 2, J=18 Hz), 4.60 (s, 2), 5.12 (d, 1, J=5 Hz), 5.4 (s, 2), 5.93 (q, 1, J=5 and 9 Hz), and 6.8—8.4 (ArH).

Anal calcd for $C_{22}H_{18}N_3O_7SCl$:

| | | | | | |
|---|---|---|---|---|---|
| | C, 52.44; | H, 3.60; | N, 8.34; | S, 6.36; | Cl, 7.04. |
| Found: | C, 52.67; | H, 3.73; | N, 8.12; | S, 6.15; | Cl, 6.95. |

### Example 33

4'-Nitrobenzyl 7-phenoxyacetamido-3-chloro-3-cephem-4-carboxylate using tri(o-tolyl)phosphite-chlorine complex

Chlorine gas was bubbled into a solution of 3.9 g (10 mmol) of tri(o-tolyl)phosphite in 45 ml of methylene chloride at $-10°C$ until a yellow color persisted. The color was then discharged by the addition of approximately 0.5 mmol of the phosphite. To the resulting solution at $-10°C$ was added 5.4 g (10 mmol) of 4'-nitrobenzyl 7-phenoxyacetamido-3-hydroxy-3-cephem-4-carboxylate which was washed into the solution with 5 ml of methylene chloride. Then 1.01 ml (1.5 mmol) of pyridine was added. After allowing the reaction mixture to stir for 90 minutes at $-10°C$, 1 ml of conc. HCl was added to the reaction mixture. After stirring for an additional 30 minutes the reaction mixture was washed successively with two 25 ml-portions of water and 25 ml of dilute sodium chloride solution, dried over sodium sulfate, and evaporated *in vacuo* to an oil which crystallized from 50 ml of 2B ethanol to provide 3.35 grams (66.5%) of the title product. An nmr spectrum of the product was identical to that of the product obtained in Example 32.

Example 34

4'-Nitrobenzyl 7-phenoxyacetamido-3-chloro-3-cephem-4-carboxylate

(A) Chlorination without base. A solution of the triphenyl phosphite-chlorine reagent was prepared as described in Example 31 above from 2.89 ml of triphenyl phosphite in methylene chloride at —10°C. To this solution was added 4.86 g (10 mmol) of 4'-nitrobenzyl 7-phenoxyacetamido-3-hydroxy-3-cephem-4-carboxylate. The reaction mixture was stirred at —10°C for 2 hours. Comparative thin layer chromatography showed that the chlorination after about 2 hours was approximately 50% complete; some imino chloride was also noted.

(B) *2,6-Lutidine*. To the reaction mixture described in Paragraph A immediately above was added 1.2 ml (10.5 mmol) of 2,6-lutidine. After stirring the reaction mixture at —10°C for 60 minutes 1 ml of conc. HCl was added. The reaction mixture was then removed from the cooling bath and stirred an additional 30 minutes after which time it was washed successively with two 100 ml-portions of water and 100 ml of dilute sodium chloride solution. The reaction mixture was then dried over magnesium sulfate and evaporated *in vacuo* to an oil which crystallized from 75 ml of 2B ethanol to provide 3.83 grams (76%) of the title product: m.p. 124°—126°C.

Example 35

4'-Nitrobenzyl 7-amino-3-chloro-3-cephem-4-carboxylate, hydrochloride

(A) Methylene chloride, pyridine.

Chlorine gas was bubbled into a solution of 6.31 ml (25 mmol) of triphenyl phosphite in 45 ml of methylene chloride at —10°C until the yellow color of excess chlorine persisted. The color was then discharged with the addition of several drops of triphenyl phosphite. To this solution of the triphenyl phosphite-chlorine reagent at —15°C was added 4.86 g (10 mmol) of 4'-nitrobenzyl 7-acetamido-3-hydroxy-3-cephem-4-carboxylate. Subsequently 2.02 ml (12.5 mmol) or pyridine in 8 ml of methylene chloride was added dropwise to the reaction mixture over a 40 minutes period. After stirring the reaction mixture for 30 minutes at —10°C, 9.25 ml (100 mmol) of isobutanol was added. The reaction mixture was then removed from the ice bath and treated with gaseous HCl for about 30 seconds. Although the product began to crystallize within 5 minutes, the reaction mixture was stirred at about 20° for 2 hours and then filtered to provide 3.33 grams (82%) of the titled nucleus ester hydrochloride: m.p. 181°C (dec.).

nmr (DMSO d-6) $\delta$ 4.06 (bs, 2), 5.33 (q, 2, J=4.5 Hz, $\beta$-lactam H), 5.5 (s, 2), 7.8—8 (ArH) and ~ 8.6 (very broad s, —$NH_3^+$).

(B) 1,2-Dichloroethane, pyridine.

The same procedure was followed as described in Example 35A immediately above except that the solvent methylene chloride was replaced with 1,2-dichloroethane. A total of 3.10 grams (76.4%) of titled nucleus ester hydrochloride product was isolated.

(C) Methylene chloride, quinoline.

The same procedure was followed as described in Paragraph A above except that the pyridine base was replaced with quinoline. A total of 3.2 grams (79.8%) of the titled product was isolated: m.p. 181°C (dec.).

(D) Methylene chloride, isoquinoline.

The same procedure was followed as described in Paragraph A above except that isoquinoline was employed in place of the pyridine base. The reaction mixture was notably darker than in previous experiments. A total of 2.29 grams (56.4%) of the title product was isolated: m.p. 181°C (dec.).

(E) Methylene chloride, N,N-dimethylaniline.

The same procedure was followed as described in Paragraph A above however, N,N-dimethylaniline was employed at the base in place of pyridine. A total of 0.91 grams (22.4%) of the title product was isolated: m.p. 182°C (dec.).

(F) Acetonitrile, pyridine.

Chlorine gas was bubbled into a mixture of 7.9 ml (30 mmol) of triphenyl phosphite in 45 ml of acetonitrile at —10°C. Because the mixture solidified it was allowed to warm to 10°C whence the reaction mixture again liquified. The addition of chlorine gas was continued until a yellow color persisted in the mixture. Then 0.1 ml of triphenyl phosphite was added to decolorize the solution (about 30.4 mmol of the triphenyl phosphite-chlorine kinetic compound was formed). To this solution was added 5.4 g (10 mmol) of 4'-nitrobenzyl 7-phenoxyacetamido-3-hydroxy-3-cephem-4-carboxylate. Thereafter 2.42 ml (30 mmol) of pyridine in 8 ml of acetonitrile was added dropwise over a 30 minute period with the temperature of the reaction mixture at 0 to 10°C. After stirring the reaction mixture for 1 hour, the cooling bath was removed, and the reaction mixture was allowed to stir at room

temperature for 90 minutes. Then 9.25 ml (100 mmol) of isobutanol was added. After 90 minutes at room temperature the reaction mixture was filtered to provide 0.95 g (23.4%) of the titled nucleus ester hydrochloride: m.p. 186°C (dec.).

(G) From 4'-Nitrobenzyl 7-phenylacetamido-3-hydroxy-3-cephem-4-carboxylate.

A solution of the triphenyl phosphite-chlorine kinetic compound was prepared in accordance with the procedure described in Paragraph 35A above using chlorine and 2.89 ml (11 mmol) of triphenyl phosphite in 45 ml of methylene chloride. To this solution was added 2.3 g (5 mmol) of 4'-nitrobenzyl 7-phenylacetamido-3-hydroxy-3-cephem-4-carboxylate. Then a solution of 0.89 ml (11 mmol) of pyridine and 5 ml of methylene chloride was added dropwise with stirring at −15 to −10°C over a 15 minutes interval. After the reaction mixture was stirred for 1.5 hours at −15 to −10°C, the cooling bath was removed, and 6 ml (64.8 mmol) of isobutanol was added. As the mixture was stirred for the next hour, the mixture warming to 23°C, the product crystallized from the reaction mixture. Filtration of the mixture provided 1.59 grams (78.3%) of the nucleus ester hydrochloride as white crystals: m.p. 188°C (dec.).

(H) Using tri(o-tolyl)phosphite-chlorine kinetic complex.

Chlorine gas was bubbled into a solution of 9.24 g (26 mmol) of tri(o-tolyl)phosphite in 45 ml of methylene chloride at −10°C until a yellow color persisted. About 0.5 mmol of the phosphite was then added to the mixture to consume the excess chlorine. To the solution was added 5.44 g (10 mmol) of 4'-nitrobenzyl 7-phenoxyacetamido-3-hydroxy-3-cephem-4-carboxylate which was washed into the reaction mixture with 5 ml of methylene chloride. A solution of 2.58 ml (32 mmol) of pyridine in 8 ml of methylene chloride was then added dropwise to the reaction mixture at −10°C over a period of 30 minutes. After the reaction mixture was stirred for 30 minutes at −10°C, 9.25 ml (100 mmol) of isobutanol was added. The reaction mixture was then removed from the ice bath, and HCl gas was bubbled in for about 60 seconds. The reaction mixture was then allowed to stir at room temperature for 90 minutes after which time it was filtered to provide 3.31 g (81.5%) of the title nucleus ester hydrochloride: m.p. 183°C (dec.).

## Example 36

4'-Nitrobenzyl 7-(1-chloro-2-phenoxyethylidene)imino-3-chloro-3-cephem-4-carboxylate

The same procedure was followed as described in Example 35A above except that instead of adding isobutanol to the reaction mixture 4.2 ml of propylene oxide was added. Thereafter the reaction mixture was allowed to stir for 15 minutes at 0°C. The reaction mixture was then washed with 50 ml of ice water and then dried over calcium chloride dihydrate. Evaporation *in vacuo* of the dried solution yielded 21 g of the dark color syrup. The addition of diethyl ether (containing a few drops of propylene oxide) to the residue deposited a small amount of tar. Then 5 ml of methylene chloride was then added to the mixture, and the resulting solution was decanted from about 1 g of a black tar. Evaporation *in vacuo* of the solution gave a syrup which was triturated under 50 ml of 1:1 ether/hexane and decanted, three times, which provided a semi solid which after being stored in a refrigerator for several days was triturated under ether to provide 1.08 g of a solid identified by nmr as 4'-nitrobenzyl 7-phenoxyacetamido-3-chloro-3-cephem-4-carboxylate. Evaporation of the filtrate *in vacuo* provided a foam which was dissolved in a few ml of methylene chloride. The resulting solution was diluted with some ether and then with about 50 ml of 2B alcohol (containing a few drops of propylene oxide). The titled imino chloride (0.24 g) crystallized (m.p. 97—98°C) from the solution. The structure of the product was confirmed by its nmr spectrum.

nmr ($CDCl_3$, pyridine d-5) $\delta$ 3.56 (ABq, 2, J=18 Hz), 4.8 (s, 2), 5.03 (d, 1, J=5 Hz), 5.3 (s, 2), 5.53 (d, 1, J=5 Hz) and 6.9—8.3 (ArH).

## Example 37

4'-Nitrobenzyl 7-amino-3-chloro-3-cephem-4-carboxylate, hydrochloride

(A) A solution of about 25.5 mole of the triphenyl phosphite-chlorine compound was prepared by bubbling chlorine gas into a solution of 6.31 ml (24 mmol) of triphenyl phosphite in 45 ml of methylene chloride at −10°C until an excess of chlorine was noted. Additional triphenyl phosphite (about 1.5 mmol) was added to the solution to discharge the yellow color. To this solution was added 5.24 g (10 mmol) of 4'-nitrobenzyl 7-phenoxyacetamido-3-hydroxy-3-cephem-4-carboxylate which was washed into the reaction mixture with an additional 5 ml of methylene chloride. Subsequently a solution of 2.02 ml of pyridine in 8 ml of methylene chloride was added dropwise over a period of 40 minutes. The temperature of the reaction mixture was maintained at −10 to −15°C. After the reaction mixture was stirred for 25 minutes at −10 to −15°C, 9.25 ml of isobutanol (100 mmol) was added to the reaction mixture. Immediately thereafter the reaction mixture was removed from the cooling bath, and gaseous HCl was bubbled into the mixture for about 30 seconds. The reaction mixture was then seeded and allowed to stir at 20°C for about 2 hours. Filtration provided 3.49 g (86%) of the titled nucleus hydrochloride as white crystals: m.p. 179—180°C (decomp.).

(B) Essentially the same procedure was followed as described in Paragraph A immediately hereinabove except that 3.61 ml of 1,3-propanediol was substituted for the isobutanol. A total 3.25 g (80%) of the titled product was isolated: m.p. 182°C (decomp.).

Examples 38—50

Following the general experimental procedure described in Example 31 the following conversions are carried out employing halogenating compounds derived from the indicated triaryl phosphite and halogen.

*Example 38.* 2',2',2'-Trichloroethyl 7-phenylacetamido-3-chloro-3-cephem-4-carboxylate from 2',2',2'-trichloroethyl 7-phenylacetamido-3-hydroxy-3-cephem-4-carboxylate; triphenyl phosphite-chlorine.

*Example 39.* Benzhydryl 7-formamido-3-bromo-3-cephem-4-carboxylate from benzhydryl 7-formamido-3-hydroxy-3-cephem-4-carboxylate; triphenyl phosphite-bromine.

*Example 40.* *tert*-Butyl 7-acetamido-3-chloro-3-cephem-4-carboxylate from *tert*-butyl 7-acetamido-3-hydroxy-3-cephem-4-carboxylate; tri(4-methoxyphenyl)phosphite-chlorine.

*Example 41.* 4'-Methoxybenzyl 7-benzamido-3-chloro-3-cephem-4-carboxylate from 4'-methoxybenzyl 7-benzamido-3-hydroxy-3-cephem-4-carboxylate; tri(o-tolyl)phosphite-chlorine.

*Example 42.* 2-Iodoethyl 7-phenoxyacetamido-3-chloro-3-cephem-4-carboxylate from 2-iodoethyl 7-phenoxyacetamido-3-hydroxy-3-cephem-4-carboxylate; triphenyl phosphite-chlorine.

*Example 43.* 4'-Nitrobenzyl 7-methoxy-7-phenylacetamido-3-bromo-3-cephem-4-carboxylate from 4'-nitrobenzyl 7-methoxy-7-phenylacetamido-3-hydroxy-3-cephem-4-carboxylate; triphenyl phosphite-bromine.

*Example 44.* 4'-Chlorophenacyl-7-(2-phenylpropionamido)-3-chloro-3-cephem-4-carboxylate from 4'-chlorophenacyl-7-(2-phenylpropionamido)-3-hydroxy-3-cepham-4-carboxylate; tri(4-ethylphenyl)phosphite-chlorine.

*Example 45.* Benzyl 7-methoxy-7-(2-thienyl)acetamido-3-chloro-3-cephem-4-carboxylate from benzyl 7-methoxy-7-(2-thienyl)acetamido-3-hydroxy-3-cephem-4-carboxylate; triphenyl phosphite-chlorine.

*Example 46.* 4'-Nitrobenzyl 7-(5-tetrazolyl)acetamido-3-chloro-3-cephem-4-carboxylate from 4'-nitrobenzyl 7-(5-tetrazolyl)acetamido-3-hydroxy-3-cephem-4-carboxylate; tri(2-ethoxyphenyl) phosphite-chlorine.

*Example 47.* Pivaloyloxymethyl 7-[2-tert-butoxycarbonylamino-2-phenylacetamido]-3-bromo-3-cephem-4-carboxylate from pivaloyloxymethyl 7-[2-tert-butoxycarbonylamino-2-phenylacetamido]-3-hydroxy-3-cephem-4-carboxylate; tri(p-propylphenyl)phosphite-bromine.

*Example 48.* 4'-Nitrobenzyl 7-[2-(4-nitrobenzyloxycarbonylamino)-2-phenylacetamido]-3-chloro-3-cephem-4-carboxylate from 4'-nitrobenzyl 7-[2-(4-nitrobenzyloxycarbonylamino)-2-phenylacetamido]-3-hydroxy-3-cephem-4-carboxylate; triphenyl phosphite-chlorine.

*Example 49.* 4'-Nitrobenzyl 7-[2-chloroacetamidothiazol-5-ylacetamido]-3-chloro-3-cephem-4-carboxylate from 4'-nitrobenzyl 7-[2-chloroacetamidothiazol-5-ylacetamido]-3-hydroxy-3-cephem-4-carboxylate; tri(o-tolyl)phosphite-chlorine.

*Example 50.* 2',2',2'-Trichloroethyl 7-chloroacetamido-3-bromo-3-cephem-4-carboxylate from 2',2',2-trichloroethyl 7-chloroacetamido-3-hydroxy-3-cephem-4-carboxylate; triphenyl phosphite-bromine.

Examples 51—59

Following the experimental procedure described in Example 35A, 4'-nitrobenzyl 7-amino-3-chloro-3-cephem-4-carboxylate hydrochloride is prepared from the following named 3-hydroxycephems using the chlorinating agent derived from chlorine and the indicated triaryl phosphite.

*Example 51.* 4'-Nitrobenzyl 7-formamido-3-hydroxy-3-cephem-4-carboxylate; triphenyl phosphite.

*Example 52.* 4'-Nitrobenzyl 7-phenylacetamido-3-hydroxy-3-cephem-4-carboxylate; tri(o-tolyl)phosphite.

*Example 53.* 4'-Nitrobenzyl 7-(2-thienylacetamido)-3-hydroxy-3-cephem-4-carboxylate; triphenyl phosphite.

*Example 54.* 4'-Nitrobenzyl 7-phenoxyacetamido-3-hydroxy-3-cephem-4-carboxylate; triphenyl phosphite.

*Example 55.* 4'-Nitrobenzyl 7-benzamido-3-hydroxy-3-cephem-4-carboxylate; triphenyl phosphite.

*Example 56.* 4'-Nitrobenzyl 7-phenylthioacetamido-3-hydroxy-3-cephem-4-carboxylate; tri(o-tolyl)phosphite.

*Example 57.* 4'-Nitrobenzyl 7-[2-(tert-butoxycarbonylamino)-2-phenylacetamido]-3-hydroxy-3-cephem-4-carboxylate; triphenyl phosphite.

*Example 58.* 4'-Nitrobenzyl 7-phenoxyacetamido-3-hydroxy-3-cephem-4-carboxylate; tri(p-methoxyphenyl)phosphite.

*Example 59.* 4'-Nitrobenzyl 7-phenylacetamido-3-hydroxy-3-cephem-4-carboxylate; tri(p-tolyl)phosphite.

Examples 60—67

Following the general experimental procedure described in Example 35A, the following conversions are carried out using the chlorinating compound derived from chlorine or bromine and the indicated triaryl phosphite.

*Example 60.* *tert*-Butyl 7-amino-3-chloro-3-cephem-4-carboxylate from *tert*-butyl 7-phenylacetamido-3-hydroxy-3-cephem-4-carboxylate; triphenyl phosphite.

*Example 61.* 4'-Nitrobenzyl 7-methoxy-7-amino-3-chloro-3-cephem-4-carboxylate from 4'-Nitrobenzyl 7-methoxy-7-phenoxyacetamido-3-hydroxy-3-cephem-4-carboxylate; triphenyl phosphite.

*Example 62.* 2',2',2'-trichloroethyl 7-amino-3-bromo-3-cephem-4-carboxylate from 2',2',2'-trichloroethyl 7-acetamido-3-hydroxy-3-cephem-4-carboxylate; tri-o-tolyl phosphite.

*Example 63.* Benzyl 7-amino-3-chloro-3-cephem-4-carboxylate from benzyl 7-(4-chlorophenoxyacetamido)-3-hydroxy-3-cephem-4-carboxylate; tri(p-ethoxyphenyl)phosphite.

*Example 64.* Benzhydryl 7-methoxy-7-amino-3-chloro-3-cephem-4-carboxylate from benzhydryl 7-methoxy-7-phenylacetamido-3-hydroxy-3-cephem-4-carboxylate; triphenyl phosphite.

*Example 65.* 4'-Nitrobenzyl 7-amino-3-bromo-3-cephem-4-carboxylate from 4'-nitrobenzyl 7-(3-nitrobenzamido)-3-hydroxy-3-cephem-4-carboxylate; triphenyl phosphite.

*Example 66.* 4'-Methoxybenzyl 7-amino-3-chloro-3-cephem-4-carboxylate from 4'-methoxybenzyl 7-[2-formyloxy-2-phenylacetamido]-3-hydroxy-3-cephem-4-carboxylate; tri(m-tolyl)phosphite.

*Example 67.* 4-Nitrobenzyl 7-amino-3-bromo-3-cephem-4-carboxylate from 4'-nitrobenzyl 7-(2-thienylacetamido)-3-hydroxy-3-cephem-4-carboxylate; triphenyl phosphite.

Examples 68—75

Following the general experimental procedures described in Example 36, the following compound conversions are carried out using a chlorinating compound derived from chlorine or bromine and the indicated triaryl phosphite.

*Example 68.* 4'-Nitrobenzyl 7-methoxy-7-(α-chlorobenzylidene)imino-3-chloro-3-cephem-4-carboxylate from 4'-nitrobenzyl 7-methoxy-7-benzamido-3-hydroxy-3-cephem-4-carboxylate; triphenyl phosphite.

*Example 69.* Benzyl 7-(1-chloro-2-phenylethylidene)imino-3-chloro-3-cephem-4-carboxylate from benzyl 7-phenylacetamido-3-hydroxy-3-cephem-4-carboxylate; tri(o-tolyl)phosphite.

*Example 70.* 2',2',2'-trichloroethyl 7-[1-chloro-2-(2-thienyl)ethylidene)imino]-3-chloro-3-cephem-4-carboxylate from 2',2',2'-trichloroethyl-7-(2-thienylacetamido)-3-hydroxy-3-cephem-4-carboxylate; triphenyl phosphite.

*Example 71.* 4'-Methoxybenzyl 7-(1-chloroethylidene)-3-chloro-3-cephem-4-carboxylate from 4'-methoxybenzyl 7-acetamido-3-hydroxy-3-cephem-4-carboxylate; triphenyl phosphite.

*Example 72.* 4'-Nitrobenzyl 7-(1-bromo-2-phenoxyethylidene)imino-3-bromo-3-cephem-4-carboxylate from 4'-nitrobenzyl 7-phenoxyacetamido-3-hydroxy-3-cephem-4-carboxylate; triphenyl phosphite.

*Example 73.* *tert*-Butyl 7-(1-chloro-2-chloroacetoxy-2-phenylethylidene)imino-3-chloro-3-cephem-4-carboxylate from *tert*-butyl 7-(2-chloroacetoxy-2-phenylacetamido)-3-hydroxy-3-cephem-4-carboxylate; tri(o-methoxyphenyl)phosphite.

*Example 74.* 4'-Nitrobenzyl 7-(4-chloro-α-chlorobenzylidene)imino-3-chloro-3-cephem-4-carboxylate from 4'-nitrobenzyl 7-(4-chlorobenzamido)-3-hydroxy-3-cephem-4-carboxylate; triphenyl phosphite.

*Example 75.* 4'-Nitrobenzyl 7-(1-bromo-2-phenylethylidene)imino-3-bromo-3-cephem-4-carboxylate from 4'-nitrobenzyl 7-phenylacetamido-3-hydroxy-3-cephem-4-carboxylate; triphenyl phosphite.

Example 76

4'-Nitrobenzyl 7-phenoxyacetamido-3-bromo-3-cephem-4-carboxylate. Triphenyl phosphite-bromine.

To a solution of 2.30 ml (45 mmol) of bromine in 90 ml of methylene chloride at −70°C was added 12.22 ml (46.6 mmol) triphenyl phosphite to discharge the bromine color. To this solution was added 10.6 g (20 mmol) of 4'-nitrobenzyl 7-phenoxyacetamido-3-hydroxy-3-cephem-4-carboxylate which was washed into the reaction mixture with 10 ml of methylene chloride. The mixture was warmed to −35 to −30°C, and a solution of 3.64 ml (45 mmol) of pyridine in 16 ml of methylene chloride was added dropwise over 35 minutes. After 4 hours 50 ml of ice water was added to the reaction mixture. The resulting solution was stirred for 1/2 hour. Three layers were noted. The methylene chloride layer, the middle layer, was washed with 50 ml of water and brine and then dried with anhydrous $Na_2SO_4$. The solvent was evaporated *in vacuo* to a weight of 29.7 grams. The addition of 150 ml of methanol induced crystallization of the titled product: 3.78 g (dried); m.p. 138—139°C.

nmr (DMSO d-6) δ 4.0 (ABq, $C_2$—H), 4.65 (s, 2, side chain $CH_2$), 5.28 (d, 1, J=5 Hz), 5.47 (s, 2, ester $CH_2$), 5.8 (q, 1, J=5 Hz and 8 Hz) and 6.9—8.4 (ArH).

Example 77

Benzyl 7-(1-chloro-2-phenylethylidene)imino-7-methoxy-3-acetoxymethyl-3-cephem-4-carboxylate

To a solution of the triphenyl phosphite-chlorine complex prepared from chlorine and 12.3 mmol of triphenyl phosphite in the presence of .1 ml of pyridine in 45 ml of methylene chloride at −15°C were added 5.11 g (10 mmol) of benzyl 7-phenylacetamido-7-methoxy-3-acetoxymethyl-3-cephem-4-carboxylate and dropwise over 10 minutes a solution of 1.01 ml (12.5 mmol) of pyridine in 4 ml of methylene chloride. After 50 minutes at −15 to −10°C, 2.1 ml (30 mmol) of propylene oxide was added. After an additional 10 minutes (reaction temperature to 0°C), the reaction mixture was washed with 25 ml of ice water, dried over $CaCl_2$ and evaporated *in vacuo* to 11 g of syrup. The product was triturated 3 times under carbon tetrachloride and then taken up in 50 ml of ester. The etheral solution was decanted from 0.5 g of precipitate and then evaporated *in vacuo* to about 25 ml. An oily product was obtained with the resulting etheral solution was diluted with 25 ml of hexane. The oil was washed twice with 1:1/hexane:ether and then evaporated *in vacuo* twice from carbon tetrachloride solutions to a foam providing 2.5 g of the title product.

ir ($CHCl_3$) 1780 and 1730 $cm^{-1}$.

nmr ($CDCl_3$, pyridine d-5) $\delta$ 1.96 (s, 3), 3.3 (ABq), 3.43 (s, 2), 3.93 (s, 2), 4.86 (ABq), 4.93 (s, 1), 5.25 (s, 1) and 7.3 (ArH).

Example 78

4′-Nitrobenzyl 7-amino-3-chloro-3-cephem-4-carboxylate hydrochloride using tri(p-chlorophenyl)phosphite-chlorine kinetic complex

To 10.34 g of tri(p-chlorophenyl)phosphite and 0.53 ml (6.5 mmol) of pyridine in 50 ml of methylene chloride at −70°C was added chlorine in 15 ml of methylene chloride. Amylene (0.52 ml) was added to discharge excess chlorine. To the resulting solution of the tri(p-chlorophenyl)phosphite-chlorine complex was added of 4′-nitrobenzyl 7-phenoxyacetamido-3-hydroxy-3-cephem-4-carboxylate (5.28 g) using 10 ml of methylene chloride to wash the substrate into the reaction mixture. Then 1.57 ml (19.5 mmol) of pyridine in 9 ml of methylene chloride was added dropwise over 33 minutes. After 2 hours the reaction mixture was allowed to warm to 2°C. Isobutanol (6.94 ml) was added, and HCl gas was bubbled through the mixture for 2 minutes. The mixture was evaporated *in vacuo* to a syrup to which was added 50 ml of ethyl acetate. The gum was triturated with about 100 ml of methanol. A white solid, tri(p-chlorophenyl)phosphate, was filtered. The filtrate was evaporated *in vacuo* to dryness. To the residue were added 15 ml of 1:1-toluene/ethyl acetate and just enough methanol to dissolve the gummy residue. Upon standing for about 5 minutes, 0.97 g of the titled product crystallized as a white solid. m.p. 184—186°C (dec.).

Example 79

4′-Nitrobenzyl 7-phenylacetamido-3-methylenecepham-4-carboxylate

To 75 ml of methylene chloride at −20°C, chlorine gas and 10 ml of triphenyl phosphite were added at such a rate that a pale green color persisted in the reaction medium throughout the co-addition. The temperature of the reaction medium was maintained at −20 to −25°C. After the addition was complete, 3 ml of amylene was added. The resulting solution of triphenyl phosphite-chlorine kinetic complex (TPP—C) was stored at −30°C.

To a stirred mixture of 5.0 ml of the above described TPP—C solution and 0.5 ml of amylene was added 500 mg of 4′-nitrobenzyl 7-phenylacetamido-3-methylenecepham-4-carboxylate 1-oxide. After the reaction mixture was stirred at 10°C for 45 minutes, 2 ml of methanol was added. The mixture was evaporated *in vacuo* to dryness. The product residue was slurried with ether. Filtration afforded 410 mg of the title product.

Nuclear magnetic resonance data for this product and those products from Examples 80—86 are presented in tabular form in Table II hereinbelow.

Example 80

4′-Nitrobenzyl 7-phenoxyacetamido-3-methylenecepham-4-carboxylate

In accordance with the procedure described in Example 79, 500 mg of 4′-nitrobenzyl 7-phenoxyacetamido-3-methylenecepham-4-carboxylate, 1-oxide was reduced to provide 370 mg of the title product.

Example 81

4′-Nitrobenzyl 7-phenoxyacetamido-3-chloro-3-cephem-4-carboxylate

In accordance with the procedure described in Example 79, 500 mg of 4′-nitrobenzyl 7-phenoxyacetamido-3-chloro-3-cephem-4-carboxylate, 1-oxide was reduced to provide 310 mg of the title product.

Example 82

4′-Nitrobenzyl 7-(2-thienylacetamido)-3-methyl-3-methyl-3-cephem-4-carboxylate

Following the experimental procedure described in Example 79, 500 mg of 4′-nitrobenzyl 7-(2-thienylacetamido)-3-cephem-4-carboxylate, 1-oxide was reduced to provide 260 mg of the title product.

Example 83

4'-Nitrobenzyl 7-heptanolyamino-3-methyl-3-cephem-4-carboxylate

In accordance with the procedure described in Example 79, 500 mg of 4'-nitrobenzyl 7-heptanoylamino-3-methyl-3-cephem-4-carboxylate, 1-oxide was reduced to provide 270 mg of the title product.

Example 84

4'-Methoxybenzyl 7-(2-thienylacetamido)-3-methyl-3-cephem-4-carboxylate

Following the procedure described in Example 79, 500 mg of 4'-methoxybenzyl 7-(2-thienylacetamido)-3-methyl-3-cephem-4-carboxylate, 1-oxide was reduced to provide 470 mg of the title product.

Example 85

Benzyl 7-(2-thienylacetamido)-3-methyl-3-cephem-4-carboxylate

Following the same general procedure in Example 79, 300 mg of benzyl 7-(2-thienylacetamido)-3-methyl-3-cephem-4-carboxylate, 1-oxide was reduced using 3 ml of the described solution of triphenyl phosphite-chlorine complex and .3 ml of amylene to provide 240 mg of the title product.

Example 86

2',2',2'-Trichloroethyl 7-phenoxyacetamido-3-methylenecepham-4-carboxylate

Following the same general procedure described in Example 79, 300 mg of 2',2',2'-trichloroethyl-7-phenoxyacetamido-3-methylenecepham-4-carboxylate, 1-oxide was reduced using 3 ml of the TPP—C solution and .3 ml of amylene to provide 80 mg of the title product.

TABLE II

Nuclear magnetic resonance data ($CDCl_3$) $\delta$ for products of Examples 1—8

| Example No. | C-2H | C-6H | C-7H | NH | ester-$CH_2$ | side chain $CH_{21}$ |
|---|---|---|---|---|---|---|
| 79 | 3.60 | 5.27 | 5.50 | 9.13 | 5.45 | 3.60 |
| 80 | 3.53 | 5.3 | 5.5 | 9.07 | 5.4 | 4.63 |
| 81 | 3.93 | 5.30 | 5.83 | 9.18 | 5.26 | 3.78 |
| 82 | 3.60 | 5.15 | 5.73 | 9.05 | 5.45 | 3.83 |
| 83 | 3.55 | 5.12 | 5.68 | 8.67 | 5.45 | |
| 84 | 3.50 | 5.07 | 5.63 | 9.05 | 5.18 | 3.78 |
| 85 | 3.50 | 5.07 | 5.63 | 9.13 | 5.26 | 3.78 |
| 86 | 3.63 | 5.3 | 5.5 | 9.13 | 5.02 | 4.63 |

Example 87

4'-Nitrobenzyl 7-phenoxyacetamido-3-methylenecepham-4-carboxylate using stabilized TTP—C

To a solution of 0.8 ml (10 mmol) of pyridine in 150 ml of methylene chloride at −20°C, chlorine gas and 20 ml of triphenyl phosphite were added at such a rate that a pale green color persisted throughout the co-addition. The temperature of the reaction medium was held at −20°C. To the resulting solution of stabilized triphenyl phosphite-chlorine kinetic complex were added 8 ml of amylene and 19.13 g of 4'-nitrobenzyl 7-phenoxyacetamido-3-methylenecepham-4-carboxylate, 1-oxide. The reaction mixture was stirred for about 1 hour at −15° to −20°C. The mixture was then warmed to room temperature and concentrated *in vacuo* to a syrup. Methanol (40 ml) was added. After stirring for 30 minutes the solution was filtered affording 11.58 g of the title product — confirmed by nmr comparison with authentic material.

Example 88

4'-Nitrobenzyl 7-phenoxyacetamido-3-hydroxy-3-cephem-4-carboxylate

A solution of TTP—C complex was prepared by the co-addition of 6.1 ml of triphenyl phosphite and chlorine to 45 ml of methylene chloride at −15°C. Triphenyl phosphite was added until starch-iodide test was negative for chlorine. To the resulting solution at −15°C were added 3 ml of amylene and 10.6 g of 4'-nitrobenzyl 7-phenoxyacetamido-3-hydroxy-3-cephem-4-carboxylate, 1-oxide. After 40 minutes the reaction mixture was allowed to warm to room temperature and then filtered to remove

unreacted starting material (5.08 g). The filtrate was concentrated *in vacuo* to about 35 ml. After cooling the solution to 0°C acetic acid (10 ml) was added. Filtration provided, in two crops, 1.81 grams of the acetic acid solvate of the title product.

nmr ($CDCl_3$) $\delta$ 2.05 (s, 3, $CH_3COOH$), 3.67 (bs, 2), 4.53 (s, 2), 5.01 (d, 1, J=4 Hz), 5.31 (ABq, 2), 5.65 (q, 1, J=4 and 9 Hz) and 6.8—8.4 (ArH).

Example 89

4'-Nitrobenzyl 7-phenoxyacetamido-3-chloro-3-cephem-4-carboxylate

Following the same procedure described in Example 88, 17.1 ml of triphenyl phosphite was used to prepare the TPP—C complex in 70 ml of methylene chloride at −20°C. Amylene (2.2 ml) was added followed by 10.6 g of 4-nitrobenzyl 7-phenoxyacetamido-3-hydroxy-3-cephem-4-carboxylate, 1-oxide. The temperature of the reaction mixture rose to −8°C. After 45 minutes, the addition of a solution of 3 ml of pyridine in 15 ml of methylene chloride over a 70 minute period was begun. The reaction temperature was maintained at −10 to −15°C for 45 minutes after the addition of pyridine was complete. The reaction mixture was concentrated *in vacuo* to about 35 ml, and 10 ml of ethanol (2B) was added. Further concentration of the solution and the addition of several ml of acetic acid resulted in crystallization of 3.2 g (in two crops) of the title product which was isolated by filtration. Structure of the product was confirmed by nmr comparison with authentic sample of title product.

Example 90

4'-Nitrobenzyl 7-amino-3-chloro-3-cephem-4-carboxylate, hydrochloride

A solution of triphenyl phosphite-chloride kinetic complex was prepared by adding chlorine and triphenyl phosphite (36.8 ml, 3.5 equivalents per equivalent of cephem sulfoxide used below −22.3 g) simultaneously to 150 ml of methylene chloride at about −20 to about −10°C, maintaining a pale yellow color in the reaction mixture throughout the co-addition. With the addition of the last drops of triphenyl phosphite to the mixture, it gave a negative starch-iodide test for chlorine. After cooling the mixture to −25°C, 5.1 ml of amylene and subsequently 22.3 g of 4'-nitrobenzyl 7-phenoxyacetamido-3-hydroxy-3-cephem-4-carboxylate, 1-oxide were added. After stirring 25 minutes at −15 to −10°C, the dropwise addition of 11 ml (3.4 equivalents per equivalent of cephem sulfoxide) of pyridine in 30 ml of methylene chloride was begun. Pyridine addition was extended over 53 minutes. Fifteen minutes after pyridine addition was complete, 37 ml (10 equivalents) of isobutanol was added and HCl was bubbled into the reaction mixture for 6 minutes. The title product crystallized from solution and was isolated by filtration, washed with 100 ml of methylene chloride and dried *in vacuo*. Yield — 6.4 g (37%).

nmr (DMSO-$d_6$) $\delta$ 4.06 (bs, 2), 5.33 (q, 2, J=4.5 Hz, $\beta$-lactam H), 5.5 (s, 2), 7.8—83 (ArH) and ~8.6 (very broad s, —$NH_3$+).

Examples 91—134

The reaction described in Example 90 was studied in detail in an attempt to optimize reaction conditions. Table III summarizes the results of these studies. The same general procedure was followed as described in Example 90 using the amounts of reagents and reaction times indicated in the Table. The substrate cephem sulfoxide and its amount (22.3 g), the amount of methylene chloride solvent for the pyridine (30 ml), and the amount of isobutanol (37 ml) was held constant in each of the tabulated examples.

TABLE III
Summary of Results for Examples 12—56

| Ex. No. | TPP—C (equiv*) | TPP (mls) | ta** (min) | Amylene (ml/equiv*) | $CH_2Cl_2$ (ml) | tb*** (min) | Pyridine (ml/equiv) | Product (g, % corrected yield) |
|---|---|---|---|---|---|---|---|---|
| 90 | 3.5 | 36.8 | 25 | 5.1/1.2 | 150 | 53 | 11.0/3.4 | 6.4/37.0 |
| 91 | 4.5 | 47.3 | 55 | 5.1/1.2 | 150 | 99 | 13.6/4.2 | 12.42/71.6 |
| 92 | 4.0 | 42.1 | 40 | 5.1/1.2 | 150 | 76 | 12.3/3.8 | 13.06/76.4 |
| 93 | 4.5 | 47.3 | 25 | 5.1/1.2 | 150 | 53 | 13.6/4.2 | 12.94/75.7 |
| 94 | 3.5 | 36.8 | 55 | 5.1/1.2 | 150 | 99 | 13.6/4.2 | 9.48/55.9 |
| 95 | 4.5 | 47.3 | 55 | 5.1/1.2 | 150 | 99 | 11.0/3.4 | 12.13/70.6 |
| 96 | 3.5 | 36.8 | 55 | 5.1/1.2 | 150 | 53 | 13.6/4.2 | 7.73/44.8 |
| 97 | 4.0 | 42.1 | 40 | 5.1/1.2 | 150 | 122 | 12.3/3.8 | 13.32/78.3 |
| 98 | 3.5 | 36.8 | 25 | 5.1/1.2 | 150 | 53 | 13.6/4.2 | 9.52/55.1 |
| 99 | 4.0 | 42.1 | 40 | 5.1/1.2 | 150 | 76 | 9.7/3.0 | 5.43/31.7 |
| 100 | 4.0 | 42.1 | 10 | 5.1/1.2 | 150 | 76 | 12.3/3.8 | 13.58/79.2 |
| 101 | 4.5 | 47.3 | 55 | 5.1/1.2 | 150 | 53 | 11.0/3.4 | 11.65/68.6 |
| 102 | 3.5 | 36.8 | 55 | 5.1/1.2 | 150 | 53 | 11.0/3.4 | 10.37/61.2 |
| 103 | 4.0 | 42.1 | 40 | 5.1/1.2 | 150 | 76 | 12.3/3.8 | 12.24/70.8 |
| 104 | 4.5 | 47.3 | 25 | 5.1/1.2 | 150 | 99 | 13.6/4.2 | 12.35/73.3 |

TABLE III (Continued)

| Ex. No. | TPP—C (equiv*) | TPP (mls) | ta** (min) | Amylene (ml/equiv*) | $CH_2Cl_2$ (ml) | tb*** (min) | Pyridine (ml/equiv) | Product (g, % corrected yield) |
|---|---|---|---|---|---|---|---|---|
| 105 | 4.0 | 42.1 | 40 | 5.1/1.2 | 150 | 76 | 12.3/3.8 | 12.85/75.5 |
| 106 | 4.0 | 42.1 | 40 | 5.1/1.2 | 150 | 76 | 14.9/4.6 | 12.36/71.4 |
| 107 | 3.0 | 31.6 | 40 | 5.1/1.2 | 150 | 76 | 12.3/3.8 | ——/~5 |
| 108 | 3.5 | 36.8 | 55 | 5.1/1.2 | 150 | 99 | 11.0/3.4 | 9.15/54.2 |
| 109 | 3.5 | 36.8 | 25 | 5.1/1.2 | 150 | 99 | 13.6/4.2 | 7.69/44.7 |
| 110 | 4.0 | 42.1 | 40 | 5.1/1.2 | 150 | 76 | 12.3/3.8 | 12.18/72.1 |
| 111 | 5.0 | 52.7 | 40 | 5.1/1.2 | 150 | 76 | 12.3/3.8 | 13.48/78.8 |
| 112 | 4.0 | 42.1 | 70 | 5.1/1.2 | 150 | 76 | 12.3/3.8 | 12.93/75.6 |
| 113 | 4.5 | 47.3 | 55 | 5.1/1.2 | 150 | 53 | 13.6/4.2 | 13.25/77.2 |
| 114 | 4.5 | 47.3 | 25 | 5.1/1.2 | 150 | 53 | 11.0/3.4 | 12.66/73.6 |
| 115 | 4.5 | 47.3 | 25 | 5.1/1.2 | 150 | 99 | 11.0/3.4 | 11.45/66.3 |
| 116 | 3.5 | 36.8 | 25 | 5.1/1.2 | 150 | 99 | 11.0/3.4 | 10.70/61.8 |
| 117 | 4.0 | 42.1 | 40 | 5.1/1.2 | 150 | 30 | 12.3/3.8 | 12.42/72.2 |
| 118 | 4.0 | 42.1 | 0 | 5.1/1.2 | 150 | 76 | 12.3/3.8 | 13.16/76.5 |
| 119 | 4.0 | 42.1 | 40 | 0 | 150 | 76 | 12.3/3.8 | 0/0 |
| 120 | 4.0 | 42.1 | 10 | 5.1/1.2 | 150 | 76 | 9.0/2.8 | 3.32/18.3 |
| 121 | 4.0 | 42.1 | 40 | 5.1/1.2 | 100 | 76 | 12.3/3.8 | 12.68/72.3 |

TABLE III (Continued)

| Ex. No. | TPP—C (equiv*) | TPP (mls) | ta** (min) | Amylene (ml/equiv*) | $CH_2Cl_2$ (ml) | tb*** (min) | Pyridine (ml/equiv) | Product (g, % corrected yield) |
|---|---|---|---|---|---|---|---|---|
| 122 | 4.0 | 42.1 | 10 | 5.1/1.2 | 150 | 76 | 12.3/3.8 | 8.2/48.4 |
| 123 | 4.0 | 42.1 | 10 | 6.4/1.5 | 150 | 76 | 12.3/3.8 | 13.33/78.6 |
| 124 | 4.0 | 42.1 | 10 | 6.4/1.5 | 150 | 76 | 12.3/3.8 | 13.90/81.0 |
| 125 | 4.0 | 42.1 | 10 | 8.5/2.0 | 200 | 76 | 12.3/3.8 | 13.19/75.4 |
| 126 | 4.0 | 42.1 | 10 | 5.1/1.2 | 150 | 76 | 12.3/3.8 | 14.4/83.1 |
| 127 | 4.0 | 42.1 | 10 | 6.4/1.5 | 150 | 40 | 12.3/3.8 | 13.16/75.7 |
| 128 | 4.2 | 44.5 | 10 | 6.4/1.5 | 150 | 76 | 12.3/3.8 | 13.54/81.6 |
| 129 | 4.2 | 44.5 | 10 | 6.4/1.5 | 200 | 40 | 12.3/3.8 | 11.05/65.0 |
| 130 | 4.2 | 44.5 | 10 | 6.4/1.5 | 200 | 60 | 12.3/3.8 | 14.09/82.8 |
| 131 | 4.2 | 44.5 | 10 | 6.4/1.5 | 200 | 60 | 12.3/3.8 | 14.00/81.7 |
| 132 | 4.4 | 46.3 | 10 | 6.4/1.5 | 200 | 60 | 12.3/3.8 | 14.16 |
| 133 | 4.4 | 46.3 | 10 | 6.4/1.5 | 200 | 60 | 12.3/3.8 | 14.35 |
| 134 | 4.2 | 44.5 | 10 | 6.4/1.5 | 200 | 60 | 12.3/3.8 | 13.77 |

* equivalents per each equivalent of cephem sulfoxide starting material
** ta is the time after cephem sulfoxide addition that pyridine addition started
*** tb is the time period over which the pyridine solution is added to the reaction mixture

Example 135

4′-Nitrobenzyl 7-amino-3-chloro-3-cephem-4-carboxylate

A solution of the triphenyl phosphite-chlorine (TPP—C) complex was prepared from 23 ml of triphenyl phosphite and chlorine in 100 ml of methylene chloride by the procedure described in Example 90. To this solution at −10 to −15°C was added 5.28 ml of cyclopentene (3.0 equivalents per equivalent of cephem sulfoxide starting material) and subsequently 11.15 g of 4′-nitrobenzyl 7-phenoxyacetamido-3-hydroxy-3-cephem-4-carboxylate, 1-oxide. A solution of 6.2 ml of pyridine in 15 ml of methylene chloride was added dropwise over a 60 minute period while the reaction temperature was maintained at −10 to −15°C. Thereafter 18.5 ml of isobutanol was added and gaseous HCl was bubbled through the mixture for about 3 minutes. The reaction mixture was then allowed to warm to room temperature, and after 2 hours was filtered to provide the title product in 80.4% yield.

Examples 136—139

The same procedure and reagent amounts (equiv.) were used as described in Example 135, except that the halogen scavenger was varied. Table IV summarizes the results of Examples 135—139.

TABLE IV

Summary of Examples 135—139

| Ex. No. | Scavenger | amount (3.0 equiv.) | Yield(%) |
|---|---|---|---|
| 135 | cyclopentene | 5.28 ml | 80.4 |
| 136 | cyclohexene | 6.08 ml | 72.8 |
| 137 | cycloheptene | 7.1 ml | 78.2 |
| 138 | 1,5-cyclooctadiene | 7.4 ml | 73.4 |
| 139 | m-dimethoxybenzene | 7.9 ml | 60.5 |

Example 140

4′-Nitrobenzyl 7-amino-3-chloro-3-cephem-4-carboxylate, hydrochloride (acetonitrile)

(A) Following the general procedure described in Example 90 the TPP—C complex was prepared from chlorine and 23.0 ml of triphenyl phosphite in 100 ml of acetonitrile. To that solution were added 3.2 ml of amylene and 11.15 gm of 4′-nitrobenzyl 7-phenoxy-acetamido-3-hydroxy-3-cephem-4-carboxylate, 1-oxide. Pyridine (6.2 ml) in acetonitrile was then added dropwise. After the pyridine addition was complete 18.5 ml of isobutanol was added. Gaseous HCl was bubbled into the reaction mixture during which time the temperature of the reaction mixture rose to 40°C. An ice bath was used to cool the mixture to about 25°C. The title product crystallized from the mixture at 28°C and was isolated in 46.5% yield.

(B) The same general procedure was followed as described in Paragraph A above except that 100 ml of tetrahydrofuran was used as the reaction medium. About 25 ml of methylene chloride was added to the mixture after the addition of the isobutanol and HCl. Yield of title product — 35.1%.

Example 141

4′-Nitrobenzyl 7-amino-3-chloro-3-cephem-4-carboxylate (room temperature)

A solution of triphenyl phosphite-chlorine complex was prepared by adding chlorine and 22.9 ml of triphenyl phosphite simultaneously to a mixture of 0.93 ml of pyridine in 100 ml of methylene chloride at 21° to 25°C. The reagents were added to such a rate that a pale green color persisted in the reaction mixture throughout the co-addition. To this solution were added 4.2 ml of amylene and subsequently 11.2 grams of 4-nitrobenzyl 7-phenoxyacetamido-3-chloro-3-cephem-4-carboxylate, 1-oxide. The reaction temperature rose to about 30°C. It was cooled to 22° before 5.3 ml of pyridine in 15 ml of methylene chloride was added dropwise over a period of 1 hour. Fifteen minutes after pyridine addition was completed 18.5 ml of isobutanol was added. HCl was bubbled into the solution for 5 minutes. Filtration after 2 hours afforded 5.69 grams of the title product.

Example 142

4′-Nitrobenzyl 7-amino-3-methylenecepham-4-carboxylate, hydrochloride

Triphenyl phosphite-chlorine complex was prepared from chlorine and 31.6 ml of triphenyl phosphite by the procedure described in Example 90. Amylene (5.1 ml) and 4′-nitrobenzyl 7-phenoxy-acetamido-3-methylenecepham-4-carboxylate 1-oxide (19.13 g) were added. After 30 minutes the dropwise addition of 6.3 ml of pyridine in 16 ml of methylene chloride was initiated. The addition was extended over 1 hour. After 15 minutes and additional 3.1 ml of pyridine in 8 ml of methylene chloride

was added over 1/2 hour. Fifteen minutes after the final addition of pyridine was complete 37 ml of isobutanol was added. HCl was bubbled through the reaction mixture for 6 minutes. Filtration after 2 hours provided 10.5 g (69.5%) of the title product.

nmr (DMSO-$d_6$) $\delta$ 3.67 (bs, 2), 5.0 (d, 1, J=5 Hz), 5.35—5.53 (m, 6) and 7.6—8.4 (m, ArH).

Example 143

4'-Nitrobenzyl 7-phenoxyacetamido-3-methylenecepham-4-carboxylate. Triphenyl phosphite-bromine kinetic complex

(A) A solution of triphenyl phosphite-bromine complex was prepared by adding 19.9 ml of triphenyl phosphite to 3.9 ml of bromine in 150 ml of methylene chloride at —30°C. A faint color was noted in the reaction mixture even after a starch-iodide test for bromine was negative. To this solution at —45°C was added amylene (8 ml) and subsequently 19.14 gm of 4'-nitrobenzyl 7-phenoxyacetamido-3-methylenecepham-4-carboxylate, 1-oxide. Comparative thin layer chromatography indicated that the reduction was complete after 20 minutes. The reaction mixture was allowed to warm to room temperature before it was concentrated *in vacuo* to about 40 ml. To the resulting solution was added 40 ml of methanol. Crystals of the title product began to form within 30 seconds. Filtration provided 14.06 g (76.8%) of the title product; nmr data confirmed its structure.

(B) The same procedure was followed as described in paragraph A above except that the solution of the triphenyl phosphite-bromine complex was cooled to —60°C before the addition of amylene and the 3-methylenecepham sulfoxide. The reaction was conducted at —40° to —45°C. Thin layer chromatography showed the reaction to be complete after 1 hour. A total of 14.06 g of the title product was isolated.

Examples 144—153

The following cephalosporin sulfoxides are reduced in accordance with the general procedure described in Example 79 using the indicated triaryl phosphite-halogen complex:

*Example 144.* Benzhydryl 7-formamido-3-acetoxymethylcephem-4-carboxylate 1-oxide; triphenyl phosphite-chlorine complex.

*Example 145.* 4'-Methoxybenzyl 7-[2-(2-thienyl)acetamido]-3-chloro-3-cephem-4-carboxylate 1-oxide; triphenyl phosphite-bromine complex.

*Example 146.* 2',2',2'-Trichloroethyl 7-chloroacetamido-3-bromomethyl-3-cephem-4-carboxylate 1-oxide; tri(p-methoxyphenyl)phosphite-chlorine complex.

*Example 147.* Benzyl 7-benzamido-3-methyl-3-cephem-4-carboxylate 1-oxide; triphenyl phosphite-chlorine complex.

*Example 148.* 4'-Nitrobenzyl 7-phenoxyacetamido-3-cephem-4-carboxylate 1-oxide; triphenyl phosphite-chlorine complex.

*Example 149.* t-Butyl 7-[2-(2-furyl)-2-methoximinoacetamido]-3-(1-methyl-1,2,3,4-tetrazol-5-yl)thiomethyl-3-cephem-4-carboxylate 1-oxide; triphenyl phosphite-chlorine complex.

*Example 150.* Benzhydryl 7-(2-formyloxy-2-phenylacetamido)-3-(1-methyl-1,2,3,4-tetrazol-5-yl)thiomethyl-3-cephem-4-carboxylate 1-oxide; tri(p-chlorophenyl)phosphite-chlorine complex.

*Example 151.* 4'-Nitrobenzyl 7-(4-nitrobenzyloxycarbonylamino)-3-methoxymethyl-3-cephem-4-carboxylate 1-oxide; tri(tolyl)phosphite-chlorine complex or triphenyl phosphite-bromine complex.

*Example 152.* 4'-Methoxybenzyl 7-phenylacetamido-3-acetylthiomethyl-3-cephem-4-carboxylate 1-oxide; triphenyl phosphite-chlorine complex.

*Example 153.* Benzhydryl 7-[2-(2-thienyl)acetamido]-3-methoxycarbonyl-3-cephem-4-carboxylate 1-oxide; tri(p-methoxyphenyl)phosphite-bromine complex.

Examples 154—163

In accordance with Scheme II in the foregoing specification the 7-acylamino cephalosporin sulfoxides used as starting materials in Examples 144—153 are converted first to the corresponding cephalosporin imino halides and subsequently to the corresponding 7-amino cephalosporin esters using the triaryl phosphite-halogen complex indicated, pyridine as the base, and isobutanol, 1,2-propanediol or 1,3-propanediol for alcoholysis of the imino chloride.

Examples 163—172

In accordance with the general procedure described in Example 90 above the following designated 7-acylamino-3-hydroxy cephalosporin sulfoxide esters are converted to the corresponding 7-amino-3-chlorocephalosporin esters using the indicated reagents.

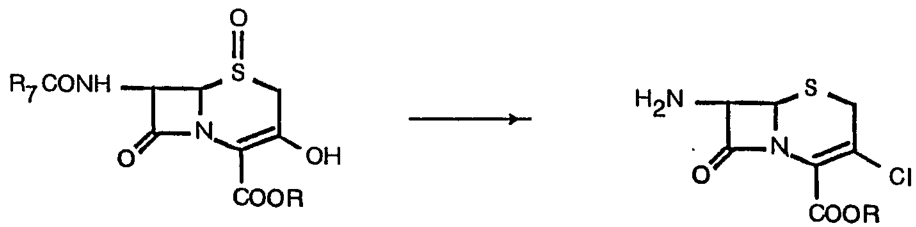

| Ex. | R | R | [Triaryl]phosphite | Solvent | Base | Alcohol |
|---|---|---|---|---|---|---|
| 163 | 4-nitrobenzyl | benzyl | triphenyl | $CH_2Cl_2$ | pyridine | isobutanol |
| 164 | benzhydryl | methyl | tritolyl | $CH_2ClCH_2Cl$ | quinoline | 1,2-propanediol |
| 165 | 2,2,2-tri chloroethyl | hydrogen | triphenyl | $CHCl_2CHCl$ | triethyl-amine | 1,3-propanediol |
| 166 | 4-nitro-benzyl | 2-thienyl-methyl | tri(4-chloro phenyl) | $CH_2Cl_2$ | diethyl-aniline | 2-methyl-butanol |
| 167 | benzyl | benzyl | tri(4-methoxy-phenyl | $CH_2ClCH_2Cl$ | pyridine | 1,2-butane-diol |
| 168 | pivaloyl-oxymethyl | phenoxy-methyl | triphenyl | chloro-benzene | isoquino-line | isobutanol |
| 169 | tert-butyl | 4-chlorophenyl thiomethyl | tritolyl | $CH_2Cl_2$ | DBU | 1,2-pro-panediol |
| 170 | 4-nitro-benzyl | α-formyl-oxybenzyl | triphenyl | Solvent | Base | Alcohol |
| 171 | phenacyl | phenoxy-methyl | tri(4-methoxy-phenyl) | $CH_2ClCH_2Cl$ | pyridine | 1,3-butane-diol |
| 172 | benzhydryl | α-benzhydryloxy-carbonylbenzyl | triphenyl | $CHBr_2CH_2Cl$ | 2,6-lutidine | isobutanol |
| | | | | $CH_2Cl_2$ | pyridine | isobutanol |

Example 173

7-(2-Thienylacetamido)-3-methyl-3-cephem-4-carboxylic acid

A solution of triphenyl phosphite-chlorine complex in methylene chloride was prepared at −20° to −35°C by addition of triphenyl phosphite (10 ml) to excess chlorine in methylene chloride (75 ml). Amylene (3 ml) was used to quench excess chlorine.

To the triphenyl phosphite-chlorine complex solution (30 ml, 12.9 mmol) at 0°C was added amylene (0.5 ml) and 7-(2-thienylacetamido)-3-methyl-3-cephem-4-carboxylic acid sulfoxide (0.90 g, 2.2 mmol). The sulfoxide dissolved after 5 minutes at 0°—5°C. The reaction was stirred at 0°—5°C for 25 minutes, during which time a precipitate formed. Water (0.1 ml) was added, and the mixture was stirred 5 minutes. After ether (50 ml) was added, the product was collected by filtration. After drying (45°C, 120 mm) for 2 days, 0.5 g of the sulfide was obtained.

nmr (DMSO d-6) $\delta$ 8.21 (d, J=8 Hz, NH), 7.38 (m), 6.96 (d, J=4 Hz), 5.67 (d, d, J=5, 8 Hz, $H_7$), 4.81 (d, J=5 Hz, $H_6$), 3.82 (s), 3.60 (AB, $H_2$), 2.03 (s, methyl).

## Claims

1. A process for preparing a penicillin or cephalosporin derivative which comprises reacting from 1.0 to 5.0 equivalents of a triaryl phosphite-halogen complex of the formula

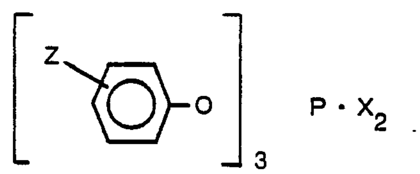

I

wherein X is Cl or Br, and Z is hydrogen, halo, $C_1$—$C_4$ alkyl or $C_1$—$C_4$ alkoxy, which is the kinetically controlled product of the reaction of equivalent amounts of a triaryl phosphite of the formula

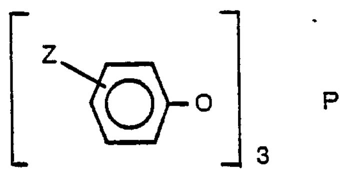

II

and chlorine or bromine in an inert organic solvent, in a substantially anhydrous inert organic solvent at a temperature of 30°C or below, with a penicillin or cephalosporin selected from

(a) a C-6 acylamino penicillin or a C-7 acylamino cephalosporin provided that when the C-6 acylamino penicillin or C-7 acylamino cephalosporin is substituted by hydroxy, amino or carboxy groups, those groups are first protected with conventional hydroxy, amino or carboxy protecting groups, to produce a penicillin or cephalosporin imino halide; the reaction being carried out in the presence of 1.0 to 1.2 equivalents of a tertiary amine base per equivalent of halogenating compound employed, and from 1.0 to 2.0 equivalents of halogenating compound being employed;

(b) a compound of the formula

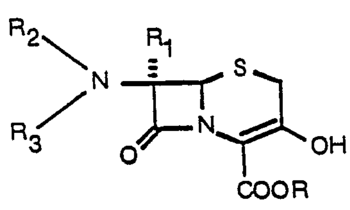

VII

to produce a compound of the formula

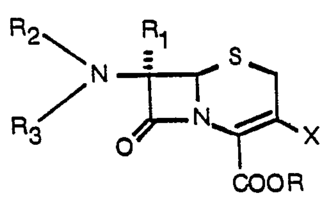

wherein

X is Cl or Br;

R is carboxylic acid protecting group;

$R_1$ is hydrogen or methoxy; and

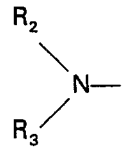

is amino protected by a conventional amino protecting group; or

$R_2$ is hydrogen or an acyl group derived from a carboxylic acid; and

$R_3$ is an acyl group derived from a carboxylic acid; or

$R_2$ and $R_3$ taken together with the nitrogen atom to which they are attached form a group of the formula

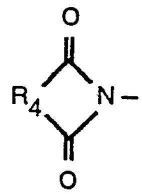

wherein $R_4$ is the residue of an acyl group derived from a dicarboxylic acid; provided that when $R_2$ and $R_3$ are substituted by amino, hydroxy or carboxy groups, those groups are first protected by one of the conventional amino, hydroxy or carboxy protecting groups; the reaction employing from 1.0 to 1.3 equivalents of halogenating compound;

(c) a compound of the formula

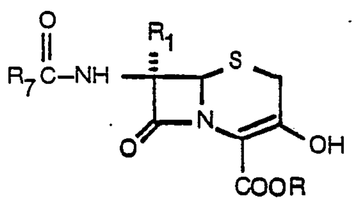

X

to produce a compound of the formula

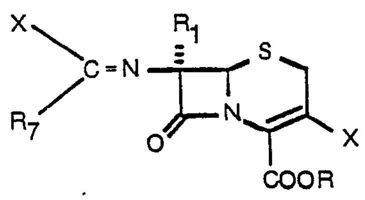

IX

wherein X, R and $R_1$ are as defined above and $R_7$ is the residue of an acyl group derived from a carboxylic acid; provided that when $R_7$ is substituted by amino, hydroxy or carboxy groups, those groups are first protected by one of the conventional amino, hydroxy, or carboxy protecting groups; the reaction being carried out in the presence of 1.0 to 1.2 equivalent of a tertiary amine base per equivalent of halogenating compound employed and from 1.0 to 1.2 equivalents of halogenating compound being employed;

(d) a cephalosporin sulfoxide provided that when the cephalosporin sulfoxide has a free amino, hydroxy or carboxy group on the C-7 substituent, those groups are first protected by conventional amino, hydroxy or carboxy protecting groups, to produce the corresponding cephalosporin; the reaction being carried out in the presence of at least 1 equivalent of a halogen scavenger and from 1.0 to 1.3 equivalents of halogenating compound being employed;

(e) a compound of the formula

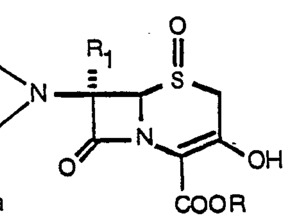

XIII

to produce a compound of the formula

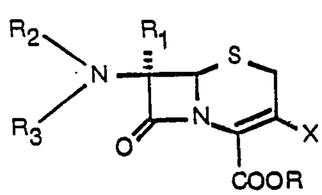

VI

wherein X, R, $R_1$, $R_2$ and $R_3$ are as defined above; the reaction being carried out in the presence of at least 1 molar equivalent of a halogen scavenger, and from 2.0 to 3.0 equivalents of halogenating compound being employed;

(f) a 7-acylamino cephalosporin sulfoxide of the formula

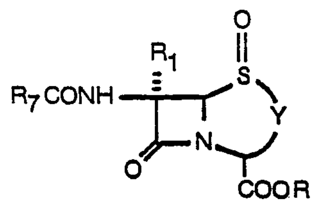

XV

to produce a cephalosporin imino halide of the formula

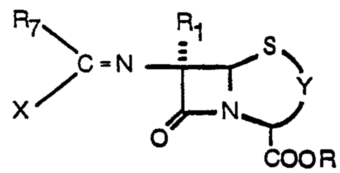

XIV

wherein

X, R and $R_1$ are as defined above and $R_7$ is the residue of an acyl group derived from a $C_1$—$C_{20}$ carboxylic acid of the formula $R_7COOH$; and Y is a divalent radical selected from the group consisting of

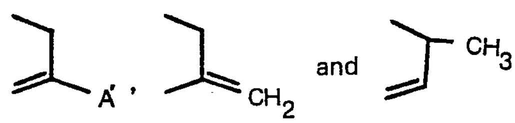

wherein A′ is hydrogen, chloro, bromo, protected hydroxy, $C_1$—$C_4$ alkoxy, methyl, $C_1$—$C_4$ alkanesulfonyloxy, $C_1$—$C_4$ alkylphenylsulfonyloxy, or a group of the formula $—CH_2B$ wherein

B is

1) $C_2$—$C_4$ alkanoyloxy, carbamoyloxy, or $C_1$—$C_4$ alkylcarbamoyloxy;
2) $C_1$—$C_4$ alkoxy;
3 chloro or bromo;
4) $C_1$—$C_4$ alkoxycarbonyl or ($C_2$—$C_6$ haloalkoxy)carbonyl; or
5) a group of the formula $—SR_9$ wherein $R_9$ is
(a) $C_1$—$C_4$ alkanoyl;
(b) $C_1$—$C_4$ alkyl, phenyl or phenyl substituted with 1 or 2 substituents selected from the group consisting of $C_1$—$C_4$ alkyl, $C_1$—$C_4$ alkoxy, protected hydroxy, chloro, bromo, fluoro, nitro, cyano, methanesulfonamido and trifluoromethyl; or
(c) a 5- or 6-membered heterocyclic ring containing 1 to 4 heteroatoms selected from the group consisting of oxygen, sulfur and nitrogen, said ring being unsubstituted or substituted by $C_1$—$C_4$ alkyl, $C_1$—$C_4$ alkoxy, chloro, bromo, oxo, halo ($C_1$—$C_4$ alkyl), protected amino, protected amino ($C_1$—$C_4$ alkyl), protected hydroxy, protected hydroxy ($C_1$—$C_4$ alkyl), protected carboxy, or protected carboxy ($C_1$—$C_4$ alkyl); provided that when $R_7$ is substituted by hydroxy, amino or carboxy groups, those groups are first protected by conventional hydroxy, amino, or carboxy protecting groups; the reaction being carried out in the presence of at least 1 equivalent of a halogen scavenger and 1 to 2 equivalents of a tertiary amine base; the reaction employing from 2.0 to 3.0 equivalents of halogenating compound;

(g) a 7-acylamino-3-hydroxy-cephalosporin sulfoxide of the formula

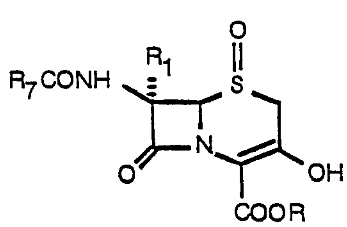

XVII

to produce a 3-halo-cephalosporin imino halide of the formula

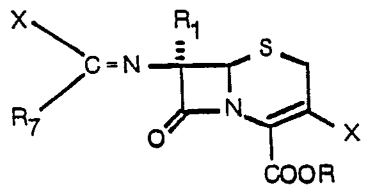

IX

wherein X, R, $R_1$ and $R_7$ are as defined above; the reaction being carried out in the presence of at least 1 equivalent of a halogen scavenger and 2.0 to 5.0 equivalents of a teriary amine base, and from 3.0 to 5.0 equivalents of halogenating compound being employed.

2. The process of claim 1(a) which comprises reacting the C-6 acylamino penicillin or C-7 acylamino cephalosporin with a triphenyl phosphite-chlorine complex.

3. The process of claim 1(a) or 2 which comprises reacting the triaryl phosphite-halogen complex with a C-6 acylamino penicillin or C-7 acylamino cephalosporin compound of the formula

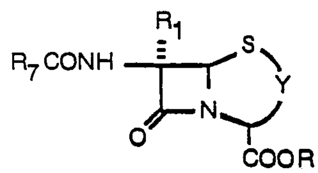

IV

wherein

R is a carboxylic acid protecting group;

$R^1$ is hydrogen or methoxy;

$R_7CO$— is an acyl group derived from a carboxylic acid; and

Y is divalent radical selected from the group consisting of

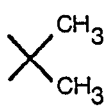 , 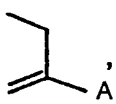 , 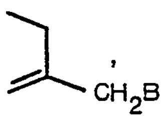 , 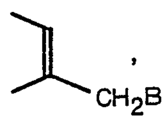 , 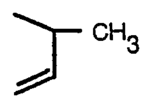 or 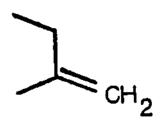

wherein A is hydrogen, chloro, bromo, protected hydroxy, $C_1$—$C_4$ alkoxy, methyl, $C_1$—$C_4$ alkanesulfonyloxy or $C_1$—$C_4$ alkylphenylsulfonyloxy; and

B is

1) $C_2$—$C_4$ alkanoyloxy, carbamoyloxy, or $C_1$—$C_4$ alkylcarbamoyloxy;

2) $C_1$—$C_4$ alkoxy;

3) chloro or bromo;

4) a group of the formula —$SR_9$ wherein $R_9$ is

a) $C_1$—$C_4$ alkanoyl;

b) $C_1$—$C_4$ alkyl, phenyl or phenyl substituted with 1 or 2 substituents selected from the group consisting of $C_1$—$C_4$ alkyl, $C_1$—$C_4$ alkoxy, protected hydroxy, chloro, bromo, fluoro, nitro, cyano, methanesulfonamido and trifluoromethyl; or

c) a 5- or 6-membered heterocyclic ring containing 1 to 4 heteroatoms selected from the group consisting of oxygen, sulfur and nitrogen, said ring being unsubstituted or substituted by $C_1$—$C_4$ alkyl, $C_1$—$C_4$ alkoxy, chloro, bromo, oxo, halo($C_1$—$C_4$ alkyl), protected amino, protected amino($C_1$—$C_4$ alkyl), protected hydroxy, protected hydroxy($C_1$—$C_4$ alkyl), protected carboxy, or protected carboxy($C_1$—$C_4$ alkyl).

4. The process of claim 1(a) or 2 which comprises reacting the triaryl phosphite-halogen complex with a C-7 acylamino cephalosporin is a compound of the formula

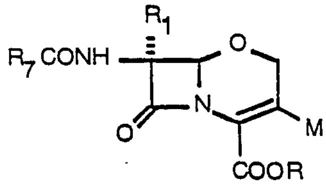

V

wherein

R is a carboxylic acid protecting group;

$R_1$ is hydrogen or methoxy;

$R_7CO$— is an acyl group derived from a carboxylic acid; and

M is hydrogen, chloro, bromo, protected hydroxy, $C_1$—$C_4$ alkoxy, methyl, $C_1$—$C_4$ alkylphenylsulfonyloxy, or a group of the formula —$CH_2B$ wherein B is as defined in claim 3.

5. The process of any one of claims 1(a) to 4 wherein 1.1 to 1.2 equivalents of halogenating compound are employed for each equivalent of C-6 acylamino penicillin or C-7 acylamino cephalosporin starting material.

6. The process of any one of claims 1(a) to 5 wherein one equivalent of tertiary amine base is employed for each equivalent of halogenating compound employed.

7. The process of claim 1(b) for preparing a compound of the formula

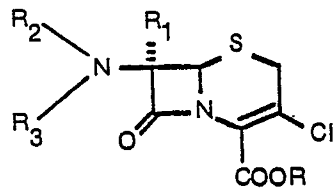

VIII

which comprises reacting a compound of the formula

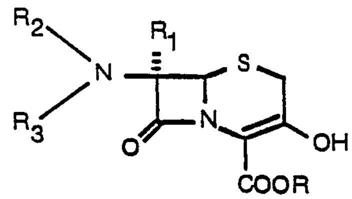

VII

with a triphenyl phosphite-chlorine complex.

8. The process of claim 1(c) for preparing an imino halide compound of the formula

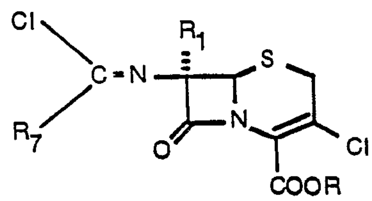

XI

which comprises reacting a compound of the formula

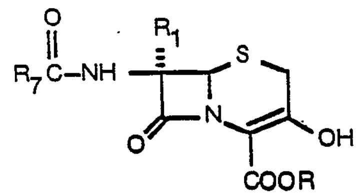

X

with 2.0 to 3.0 equivalents of triphenyl phosphite-chlorine complex.

9. The process of claim 7 or 8 wherein 2.2 to 2.4 equivalents of halogenating compound are employed.

10. The process of any one of claims 7 to 9 wherein the process is carried out in the presence of 1.0 to 1.2 equivalents of a tertiary amine base per equivalent of halogenating compound.

11. The process of claim 10 wherein about 1.0 equivalent of tertiary amine base is employed for each equivalent of halogenating compound.

12. The process of any one of claims 1(a), (b) or (c) to 11 wherein the tertiary amine base has a $pK_b$ value of 6 to 10.

13. The process of claim 1(d) which comprises reacting said cephalosporin sulfoxide with 1.0 to 1.3 equivalents of a triphenyl phosphite-chlorine complex.

14. The process of claim 1(d) or 13 which comprises reacting the triaryl phosphite-halogen complex with a compound of the formula

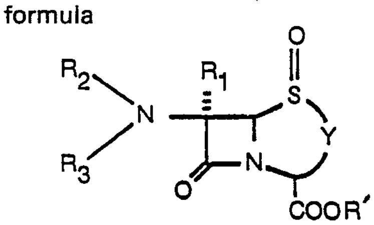

XII

wherein

R' is hydrogen or a carboxylic acid protecting group;
$R_1$ is hydrogen or methoxy

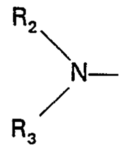

is amino protected by a conventional amino protecting group; or

$R_2$ is hydrogen or an acyl group derived from a carboxylic acid, and

$R_3$ is an acyl group derived from a carboxylic acid; or $R_2$ and $R_3$ taken together with the nitrogen atom to which they are attached form a group of the formula

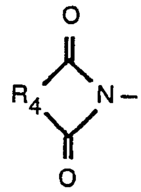

wherein $R_4$ is the residue of an acyl group derived from a dicarboxylic acid; and Y is a divalent radical selected from the group consisting of

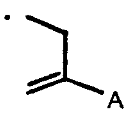 , 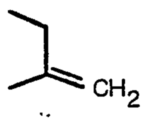 or 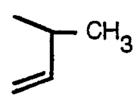

wherein A is hydrogen, chloro, bromo, hydroxy, a protected hydroxy, $C_1$—$C_4$ alkoxy, methyl, $C_1$—$C_4$ alkanesulfonyloxy, $C_1$—$C_4$ alkylphenylsulfonyloxy, or a group of the formula —$CH_2B$ wherein

B is

1) $C_2$—$C_4$ alkanoyloxy, carbamoyloxy, or $C_1$—$C_4$ alkylcarbamoyloxy;

2) $C_1$—$C_4$ alkoxy;

3) chloro or bromo;

4) $C_1$—$C_4$ alkoxycarbonyl or ($C_2$—$C_6$ haloalkoxy)carbonyl; or

5) a group of the formula —$SR_9$ wherein $R_9$ is

a) $C_1$—$C_4$ alkanoyl;

b) $C_1$—$C_4$ alkyl, phenyl or phenyl substituted with 1 or 2 substituents selected from the group consisting of $C_1$—$C_4$ alkyl, $C_1$—$C_4$ alkoxy, protected hydroxy, chloro, bromo, fluoro, nitro, cyano, methanesulfonamido and trifluoromethyl; or

c) a 5- or 6-membered heterocyclic ring containing 1 to 4 heteroatoms selected from the group consisting of oxygen, sulfur and nitrogen, said ring being unsubstituted or substituted by $C_1$—$C_4$ alkyl, $C_1$—$C_4$ alkoxy, chloro, bromo, oxo, halo($C_1$—$C_4$ alkyl), protected amino, protected amino($C_1$—$C_4$ alkyl), protected hydroxy, protected hydroxy($C_1$—$C_4$ alkyl), protected carboxy, or protected carboxy($C_1$—$C_4$) alkyl.

15. The process of claim 1(d), 13 or 14 wherein the triaryl phosphite-halogen complex is reacted with a cephalosporin sulfoxide in which $R_2$ is an acyl group of the formula $R_7CO$— wherein $R_7$ is

1) hydrogen, $C_1$—$C_6$ alkyl, halo($C_1$—$C_4$)alkyl, cyanomethyl, trifluoromethylthiomethyl, or 4-protected amino-4-protected carboxybutyl;

2) the group $R_1$ wherein $R_1$ is phenyl or phenyl substituted with 1 or 2 substituents selected from the group consisting of $C_1$—$C_4$ alkyl, $C_1$—$C_4$ alkoxy, protected hydroxy, chloro, bromo, fluoro, iodo, nitro, cyano, carbamyl, methanesulfonamido and trifluoromethyl;

3) an arylalkyl group of the formula

$$R^o—(Q)_m—CQ_1Q_2—$$

wherein R° is $R_a$ as defined above, 1,4-cyclohexadienyl, or a 5-membered heterocyclic ring containing 1 to 4 heteroatoms selected from the group consisting of oxygen, nitrogen, and sulfur, said ring being unsubstituted or substituted by $C_1$—$C_4$ alkyl, $C_1$—$C_4$ alkoxy, chloro, bromo, oxo, protected amino, protected amino($C_1$—$C_4$ alkyl), protected hydroxy or protected carboxy;

m is 1 or 0;

Q is oxygen or sulfur, and

$Q_1$ and $Q_2$ are independently hydrogen or methyl;

subject to the limitation that in the above formula when m is 1, R° is limited to $R_a$;

4) a substituted arylalkyl group of the formula

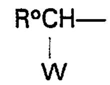

wherein R° is as defined above and W is ureido, protected amino, protected hydroxy or protected carboxy; or

5) a substituted oximino group of the formula

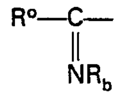

wherein R° is defined as in paragraph (3) immediately hereinabove and $R_b$ is $C_1$—$C_4$ alkoxy.

16. The process of any one of claims 1(d) or 13 to 15 wherein the cephalosporin sulfoxide is a 3-cephem sulfoxide or a 3-exomethylenecepham sulfoxide.

17. The process of claim 1(e) for preparing a compound of the formula

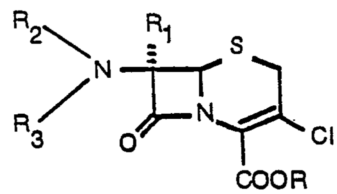

VIII

by reacting a compound of the formula

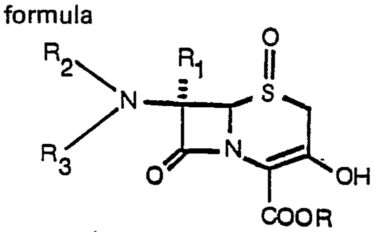

XIII

with triphenyl phosphite-chlorine complex.

18. The process of claim 1(e) or 17 wherein, additionally, the process is conducted in the presence of 1 to 2 equivalents of a tertiary amine base.

19. The process of claim 1(f) for preparing a cephalosporin imino chloride of the formula

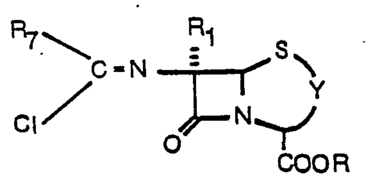

XVI

which comprises reacting a 7-acylamino cephalosporin sulfoxide of the formula

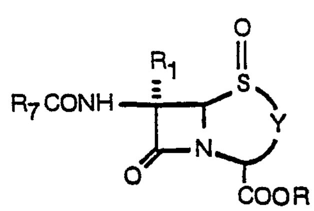

XV

with a triphenyl phosphite-chlorine complex.

20. The process of claim 1(g) for preparing a 3-chlorocephalosporin imino chloride of the formula

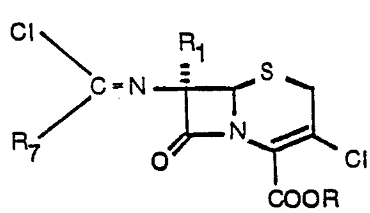

XI

which comprises reacting a 7-acylamino-3-hydroxycephalosporin sulfoxide of the formula

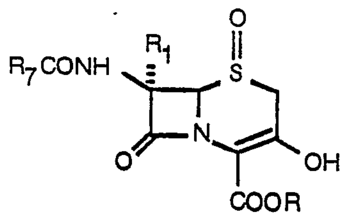

XVII

with 3 to 5 equivalents of a triphenyl phosphite-chlorine complex.

21. The process of claim 20 wherein the triaryl phosphite-halogen complex is reacted with a cephalosporin sulfoxide in which $R_7$ is as defined in claim 15.

22. The process of claim 20 or 21 wherein 4 to 5 equivalents of a triaryl phosphite-halogen complex and 3.5 to 4 equivalents of a tertiary amine base are employed.

23. The process of claim 22 wherein 4.4 equivalents of triphenyl phosphite-chlorine complex and 3.8 equivalents of pyridine are employed.

24. The process of any one of claims 1(d), 1(e), 1(f), 1(g) and 13 to 23 wherein the halogen scavenger is a $C_2$—$C_{10}$ alkene, a cycloalkene having from 5 to 8 ring carbon atoms, a $C_4$—$C_8$ diene or cyclodiene having from 5 to 8 ring carbon atoms, an alkyne having from 2 to 6 carbon atoms or a readily halogenated phenol derivative of the formula

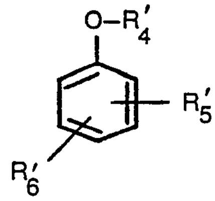

XVIII

wherein $R_4'$ is $C_1$—$C_4$ alkyl, or $C_2$—$C_5$ alkanoyl, and $R_5'$ and $R_6'$ are independently hydrogen, $C_1$—$C_4$ alkoxy, $C_2$—$C_5$ alkanoyl or $C_1$—$C_4$ alkyl.

25. The process of claim 24 wherein the halogen scavenger is a $C_2$—$C_6$ alkene.

26. The process of any one of claims 1(d), 1(e), 1(f), 1(g) and 13 to 25 wherein the temperature is −50° to 30°C.

27. The process of claim 1(g), 20 and 21 wherein the temperature is −30° to 0°C.

28. The process of claim 1 wherein X is Br.

29. The process of claim 28 wherein Z is hydrogen.

30. The process of claim 1 wherein X is Cl.

31. The process of any one of the preceding claims wherein the triaryl phosphite-halogen complex is stabilized with a tertiary amine base.

32. The process of any one of claims 1(a), 1(c), 1(f), 1(g), 2 to 6, 8 to 12, or 18 to 21 wherein the tertiary amine base has a $pK_b$ of 6 to 10.

33. The process of claim 31 or 32 wherein the tertiary amine base is pyridine.

34. The process of any one of the preceding claims wherein the inert organic solvent is an aromatic hydrocarbon or a halogenated hydrogen.

35. The process of claim 34 wherein the inert organic solvent is methylene chlorine.

36. The process of any one of the preceding claims wherein the $C_6$ or $C_7$ acyl group is 2-thienylmethyl, phenoxymethyl or benzyl.

37. The process of claims 1(a), 1(c), 1(f), 1(g), 2 to 6, 8 to 12 or 19 to 21 wherein after formation of the imino chloride product is complete, at least a 3-fold excess of a $C_1$—$C_{15}$ aliphatic alcohol and hydrogen chloride are added to the reaction mixture to provide a nucleus ester hydrochloride of the formula

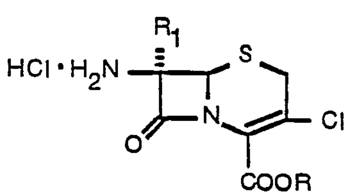

XIX

38. The process of claim 37 wherein after formation of the imino chloride product is complete, at least 3 equivalents of a $C_4$—$C_{12}$ $\beta$-disubstituted primary aliphatic alcohol, a $C_2$—$C_{12}$ 1,2-diol or a $C_3$—$C_{15}$ 1,3-diol and hydrogen chloride are added to the reaction mixture to provide a nucleus ester hydrochloride of the formula

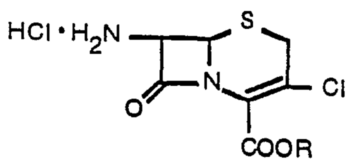

XIX

39. The process of claim 38 wherein the alcohol or diol is isobutanol, 1,2-propanediol, or 1,3-propanediol.

40. The process of any one of the preceding claims wherein the triphenyl phosphite-chlorine complex is of the formula

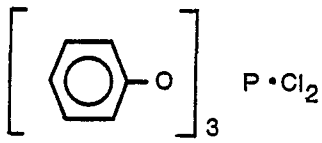

III

which

(a) has a $^{31}P$ nuclear magnetic resonance signal in methylene chloride at —3.7 ppm relative to that of phosphoric acid;

(b) has in methylene chloride an infrared spectrum with has the following characteristic absorptions: 1120—1190 (very strong), 1070 (very strong), 1035 (strong), 1010 (very strong), 990 (very strong), 640 (medium) 625 (medium), 580 (weak), 510 (strong) and 465 (weak);

(c) reacts with water to give HCl and triphenyl phosphate; and

(d) reacts with n-butanol to give HCl, n-butyl chloride, and triphenyl phosphate;

**Patentansprüche**

1. Verfahren zur Herstellung eines Penicillin- oder Cephalosporinderivats, dadurch gekennzeichnet, daß man 1.0 bis 5,0 Äquivalente eines Triarylphosphit-Halogen-Komplexes der Formel

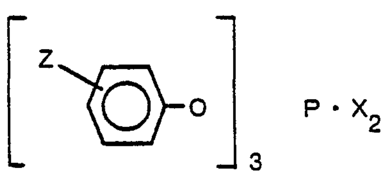

I

worin X für Cl oder Br steht und Z Wasserstoff, Halogen, $C_1$—$C_4$-Alkyl oder $C_1$—$C_4$-Alkoxy ist, welcher das kinetisch gesteuerte Produkt der Reaktion äquivalenter Mengen an Triarylphosphit der Formel

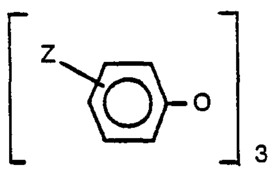

II

und Chlor oder Brom in einem inerten organischen Lösungsmittel ist, umsetzt mit einem Penicillin oder Cephalosporin ausgewählt aus

(a) einem C-6-Acylaminopenicillin oder einem C-7-Acylaminocephalosporin, mit der Maßgabe, daß man im Falle eines durch Hydroxyl-, Amino- oder Carboxylgruppen substituierten C-6-Acylaminopenicillins oder C-7-Acylaminocephalosporins diese Gruppen zuerst durch herkömmliche Hydroxyl-, Amino- oder Carboxylschutzgruppen schützt, und so ein Penicillin- oder Cephalosporiniminohalogenid herstellt, wobei man die Umsetzung in Gegenwart von 1,0 bis 1,2 Äquivalenten einer tertiären Aminbase pro Äquivalent an verwendeter Halogenierungsverbindung durchführt und 1,0 bis 2,0 Äquivalente an Halogenierungsverbindung verwendet,

(b) einer Verbindung der Formel

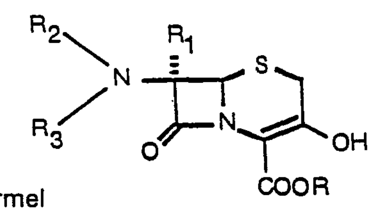

VII

und so eine Verbindung der Formel

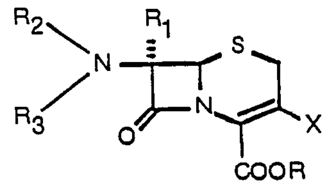

herstellt, worin

X für Cl oder Br steht,

R eine Carbonsäureschutzgruppe ist,

$R_1$ Wasserstoff oder Methoxy bedeutet und

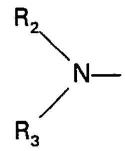

für Amino steht, das durch eine herkömmliche Aminoschutzgruppe geschützt ist oder

$R_2$ Wasserstoff oder eine von einer Carbonsäure abgeleitete Acylgruppe bedeutet und

$R_3$ für eine von einer Carbonsäure abgeleitete Acylgruppe steht oder

$R_2$ und $R_3$ zusammen mit dem Stickstoffatom, an das sie gebunden sind, eine Gruppe der Formel

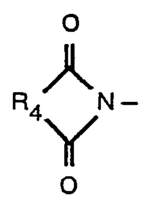

bilden, worin $R_4$ für den Rest einer von einer Dicarbonsäure abgeleiteten Acylgruppe steht, mit der Maßgabe, daß, falls $R_2$ und $R_3$ durch Amino-, Hydroxyl- oder Carboxylgruppen substituiert sind, diese Gruppen zuerst durch eine der herkömmlichen Amino-, Hydroxyl, oder Carboxylschutzgruppen geschützt werden, wobei man diese Umsetzung unter Verwendung von 1,0 bis 1,3 Äquivalenten Halogenierungsverbindung durchführt,

(c) einer Verbindung der Formel

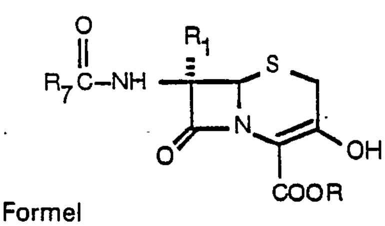

X

und so eine Verbindung der Formel

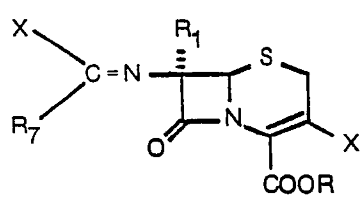

IX

herstellt, worin X, R und $R_1$ die oben angegebenen Bedeutungen haben und $R_7$ für den Rest einer von einer Carbonsäure abgeleiteten Acylgruppe steht, mit der Maßgabe, daß, falls $R_7$ durch Amino-, Hydroxyl- oder Carboxylgruppen substituiert ist, diese Gruppen zuerst durch eine der herkömmlichen Amino-, Hydroxyl- oder Carboxylschutzgruppen geschützt werden, wobei man die Umsetzung in Gegenwart von 1,0 bis 1,2 Äquivalenten einer tertiären Aminbase pro Äquivalent verwendeter Halogenierungsverbindung durchführt und 1,0 bis 1,2 Äquivalente an Halogenierungsverbindung anwendet,

(d) einem Cephalosporinsulfoxid, mit der Maßgabe, daß, falls das Cephalosporinsulfoxid eine freie Amino-, Hydroxyl- oder Carboxylgruppe am C-7-Substituenten aufweist, diese Gruppen zuerst durch herkömmliche Amino-, Hydroxyl- oder Carboxylschutzgruppen geschützt werden, und so das entsprechende Cephalosporin herstellt, wobei man die Umsetzung in Gegenwart von wenigstens einem Äquivalent eines Halogenfängers durchführt und 1,0 bis 1,3 Äquivalente an Halogenierungsverbindung verwendet,

(e) einer Verbindung der Formel

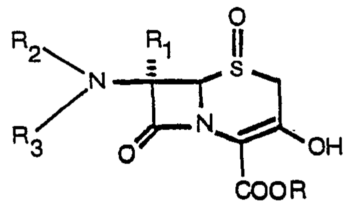

XIII

und so eine Verbindung der Formel

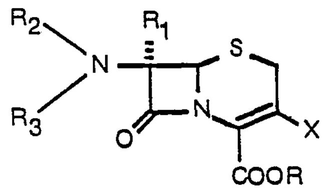

VI

herstellt, worin X, R, $R_1$, $R_2$ und $R_3$ die oben angegebenen Bedeutungen haben, wobei man die Umsetzung in Gegenwart von wenigstens einem Moläquivalent eines Halogenfängers durchführt und 2,0 bis 3,0 Äquivalente an Halogenierungsverbindung verwendet,

(f) einem 7-Acylaminocephalosporinsulfoxid der Formel

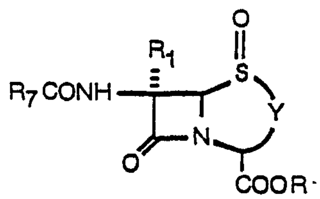

XV

und so ein Cephalosporiniminohalogenid der Formel

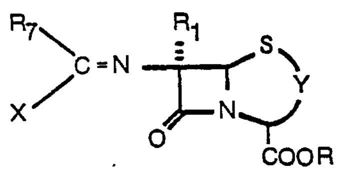

XIV

herstellt, worin X, R und $R_1$ die oben angegebenen Bedeutung haben und

$R_7$ für den Rest einer von einer $C_1$—$C_{20}$-Carbonsäure der Formel $R_7COOH$ abgeleiteten Acylgruppe steht und

Y einen zweiwertigen Rest bedeutet, der ausgewählt ist aus der Gruppe

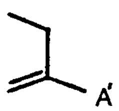 , 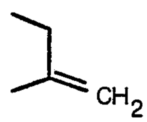 und 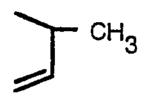

worin A' Wasserstoff, Chlor, Brom, geschütztes Hydroxyl, $C_1$—$C_4$-Alkoxy, Methyl, $C_1$—$C_4$-Alkansulfonyloxy, $C_1$—$C_4$-Alkylphenylsulfonyloxy oder eine Gruppe der Formel —$CH_2B$ ist, worin

B steht für

1) $C_2$—$C_4$-Alkanoyloxy, Carbamoyloxy oder $C_1$—$C_4$-Alkylcarbamoyloxy,

2) $C_1$—$C_4$-Alkoxy,

3) Chlor oder Brom,

4) $C_1$—$C_4$-Alkoxycarbonyl oder ($C_2$—$C_6$-Halogenalkoxy)carbonyl oder

5) eine Gruppe der Formel —$SR_9$, worin $R_9$ folgende Bedeutungen hat

(a) $C_1$—$C_4$-Alkanoyl,

(b) $C_1$—$C_4$-Alkyl, Phenyl oder substituiertes Phenyl, das 1 oder 2 Substituenten aus der Gruppe $C_1$—$C_4$-Alkyl, $C_1$—$C_4$-Alkoxy, geschütztes Hydroxyl, Chlor, Brom, Fluor, Nitro, Cyano, Methansulfonamido oder Trifluormethyl enthält, oder

(c) einen 5- oder 6-gliedrigen heterocyclischen Ring, der 1 bis 4 Heteroatome enthält, die ausgewählt sind aus der Gruppe Sauerstoff, Schwefel und Stickstoff, wobei dieser Ring unsubstituiert oder

substituiert ist durch $C_1$—$C_4$-Alkyl, $C_1$—$C_4$-Alkoxy, Chlor, Brom, Oxo, Halogen($C_1$—$C_4$-alkyl), geschütztes Amino, geschütztes Amino($C_1$—$C_4$-alkyl), geschütztes Hydroxyl, geschütztes Hydroxy($C_1$—$C_4$-alkyl), geschütztes Carboxyl oder geschütztes Carboxy($C_1$—$C_4$-alkyl), mit der Maßgabe, daß, falls $R_7$ durch Hydroxyl-, Amino- oder Carboxylgruppen substituiert ist, diese Gruppen zuerst durch herkömmliche Hydroxyl-, Amino- oder Carboxylschutzgruppen geschützt werden, wobei man die Umsetzung in Gegenwart von wenigstens einem Äquivalent eines Halogenfängers und 1 bis 2 Äquivalenten einer tertiären Aminbase durchführt, und wobei man bei der Umsetzung 2,0 bis 3,0 Äquivalente an Halogenierungsverbindung verwendet,

(g) einem 7-Acylamino-3-hydroxycephalosporinsulfoxid der Formel

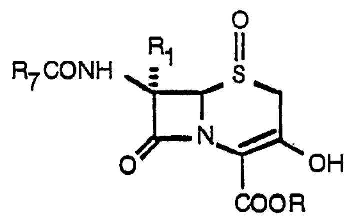

XVII

und so ein 3-Halogencephalosporiniminohalogenid der Formel

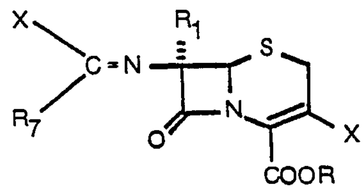

IX

herstellt, worin X, R, $R_1$ und $R_7$ die oben angegebenen Bedeutungen haben, wobei man die Umsetzung in Gegenwart von wenigstens einem Äquivalent eines Halogenfängers und 2,0 bis 5,0 Äquivalenten einer tertiären Aminbase durchführt und 3,0 bis 5,0 Äquivalente an Halogenierungsverbindung verwendet.

2. Verfahren nach Anspruch 1(a), dadurch gekennzeichnet, daß man das C-6-Acylaminopenicillin oder C-7-Acylaminocephalosporin mit einem Triphenylphosphit-Chlor-Komplex umsetzt.

3. Verfahren nach Anspruch 1(a) oder 2, dadurch gekennzeichnet, daß man den Triarylphosphit-Halogen-Komplex mit einer C-6-Acylaminopenicillin- oder C-7-Acylaminocephalosporinverbindung der Formel

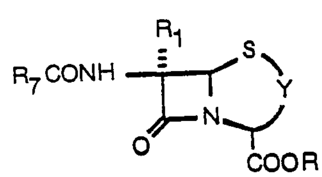

IV

umsetzt, worin

R eine Carbonsäureschutzgruppe ist,

$R_1$ Wasserstoff oder Methoxy bedeutet,

$R_7CO$— für eine von einer Carbonsäure abgeleitete Acylgruppe steht und

Y einen zweiwertigen Rest bedeutet, der ausgewählt ist aus der Gruppe

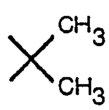 , 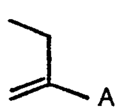 , 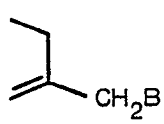 , 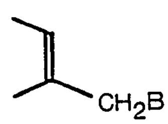 , 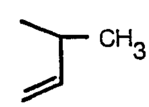 oder 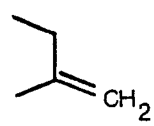

worin A Wasserstoff, Chlor, Brom, geschütztes Hydroxyl, $C_1$—$C_4$-Alkoxy, Methyl, $C_1$—$C_4$-Alkansulfonyloxy oder $C_1$—$C_4$-Alkylphenylsulfonyloxy bedeutet und

B steht für

1) $C_2$—$C_4$-Alkanoyloxy, Carbamoyloxy oder $C_1$—$C_4$-Alkylcarbamoyloxy,

2) $C_1$—$C_4$-Alkoxy,

3) Chlor oder Brom,

4) eine Gruppe der Formel —$SR_9$, worin $R_9$ folgende Bedeutungen hat

(a) $C_1$—$C_4$-Alkanoyl,

(b) $C_1$—$C_4$-Alkyl, Phenyl oder substituiertes Phenyl, das 1 oder 2 Substituenten aus der Gruppe $C_1$—$C_4$-Alkyl, $C_1$—$C_4$-Alkoxy, geschütztes Hydroxyl, Chlor, Brom, Fluor, Nitro, Cyano, Methansulfonamido oder Trifluormethyl enthält, oder

(c) einen 5- oder 6-gliedrigen heterocyclischen Ring, der 1 bis 4 Heteroatome enthält, die ausgewählt sind aus der Gruppe Sauerstoff, Schwefel und Stickstoff, wobei dieser Ring unsubstituiert oder substituiert ist durch $C_1$—$C_4$-Alkyl, $C_1$—$C_4$-Alkoxy, Chlor, Brom, Oxo, Halogen($C_1$—$C_4$-alkyl), geschütztes Amino, geschütztes Amino($C_1$—$C_4$-alkyl), geschütztes Hydroxyl, geschütztes Hydroxy($C_1$—$C_4$-alkyl), geschütztes Carboxyl oder geschütztes Carboxy($C_1$—$C_4$-alkyl).

4. Verfahren nach Anspruch 1(a) oder 2, dadurch gekennzeichnet, daß man den Triarylphosphit-Halogen-Komplex mit einer C-7-Acylaminocephalosporinverbindung der Formel

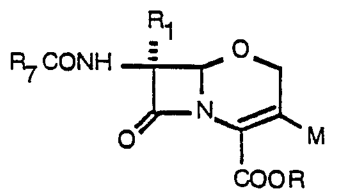

V

umsetzt, worin

R eine Carbonsäureschutzgruppe ist,

$R_1$ Wasserstoff oder Methoxy bedeutet,

$R_7CO$— für eine von einer Carbonsäure abgeleitete Acylgruppe steht und

M Wasserstoff, Chlor, Brom, geschütztes Hydroxyl, $C_1$—$C_4$-Alkoxy, Methyl, $C_1$—$C_4$-Alkylphenylsulfonyloxy oder eine Gruppe der Formel —$CH_2B$ bedeutet, worin B die in Anspruch 3 angegebene Bedeutung hat.

5. Verfahren nach einem der Ansprüche 1(a) bis 4, dadurch gekennzeichnet, daß man 1,1 bis 1,2 Äquivalente an Halogenierungsverbindung je Äquivalent an C-6-Acylaminopenicillin- oder C-7-Acylaminocephalosporinausgangsmaterial verwendet.

6. Verfahren nach einem der Ansprüche 1(a) bis 5, dadurch gekennzeichnet, daß man ein Äquivalent an tertiärer Aminbase je Äquivalent an Halogenierungsverbindung verwendet.

7. Verfahren nach Anspruch 1(b) zur Herstellung einer Verbindung der Formel

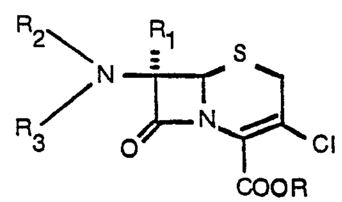

VIII

dadurch gekennzeichnet, daß man eine Verbindung der Formel

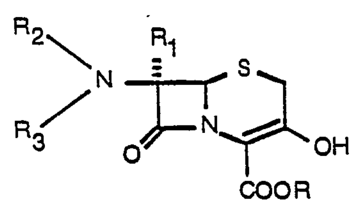

VII

mit einem Triphenylphosphit-Chlor-Komplex umsetzt.

8. Verfahren nach Anspruch 1(c) zur Herstellung einer Iminohalogenidverbindung der Formel

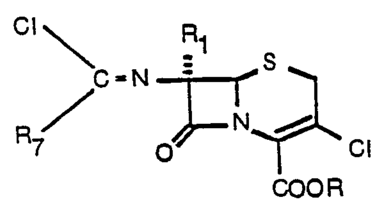

XI

dadurch gekennzeichnet, daß man eine Verbindung der Formel

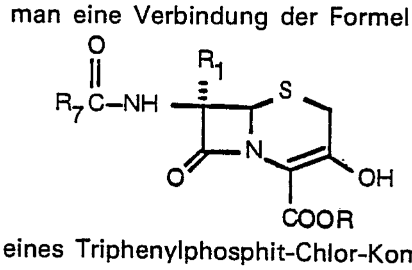

X

mit 2,0 bis 3,0 Äquivalenten eines Triphenylphosphit-Chlor-Komplexes umsetzt.

9. Verfahren nach Anspruch 7 oder 8, dadurch gekennzeichnet, daß man 2,2 bis 2,4 Äquivalente an Halogenierungsverbindung verwendet.

10. Verfahren nach einem der Ansprüche 7 bis 9, dadurch gekennzeichnet, daß man die Umsetzung in Gegenwart von 1,0 bis 1,2 Äquivalenten einer tertiären Aminbase pro Äquivalent an Halogenierungsverbindung durchführt.

11. Verfahren nach Anspruch 10, dadurch gekennzeichnet, daß man etwa 1,0 Äquivalent an tertiärer Aminbase pro Äquivalent an Halogenierungsverbindung verwendet.

12. Verfahren nach einem der Ansprüche 1(a), (b) oder (c) bis 11, dadurch gekennzeichnet, daß man eine tertiäre Aminbase mit einem $pK_b$-Wert von 6 bis 10 verwendet.

13. Verfahren nach Anspruch 1(d), dadurch gekennzeichnet, daß man das Cephalosporinsulfoxid mit 1,0 bis 1,3 Äquivalenten an Triphenylphosphit-Chlor-Komplex umsetzt.

14. Verfahren nach Anspruch 1(d) oder 13, dadurch gekennzeichnet, daß man den Triarylphosphit-Halogen-Komplex mit einer Verbindung der Formel

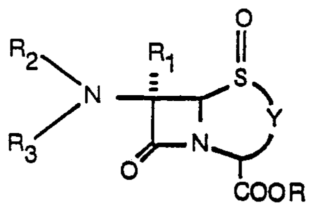

XII

umsetzt, worin

R' Wasserstoff oder eine Carbonsäureschutzgruppe ist,

$R_1$ Wasserstoff oder Methoxy beduetet,

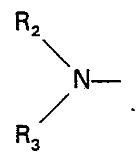

für Amino steht, das durch eine herkömmliche Aminoschutzgruppe geschützt ist, oder

$R_2$ Wasserstoff oder eine von einer Carbonsäure abgeleitete Acylgruppe bedeutet und

$R_3$ für eine von einer Carbonsäure abgeleitete Acylgruppe steht oder

$R_2$ und $R_3$ zusammen mit dem Stickstoffatom, an das sie gebunden sind, eine Gruppe der Formel

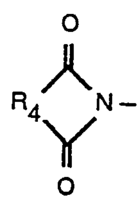

bilden, worin $R_4$ für den Rest einer von einer Dicarbonsäure abgeleiteten Acylgruppe steht, und

Y einen zweiwertigen Rest bedeutet, der ausgewählt ist aus der Gruppe

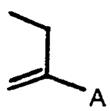 , 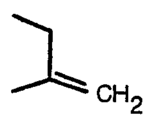 oder 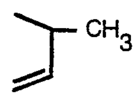

worin A Wasserstoff, Chlor, Brom, Hydroxyl, geschütztes Hydroxyl, $C_1$—$C_4$-Alkoxy, Methyl, $C_1$—$C_4$-Alkansulfonyloxy, $C_1$—$C_4$-Alkylphenylsulfonyloxy oder eine Gruppe der Formel —$CH_2B$ ist, worin

B steht für

1) $C_2$—$C_4$-Alkanoyloxy, Carbamoyloxy oder $C_1$—$C_4$-Alkylcarbamoyloxy,

2) $C_1$—$C_4$-Alkoxy,

3) Chlor oder Brom,

4) $C_1$—$C_4$-Alkoxycarbonyl oder ($C_2$—$C_6$-Halogenalkoxy)carbonyl oder

5) eine Gruppe der Formel —$SR_9$, worin $R_9$ folgende Bedeutungen hat

(a) $C_1$—$C_4$-Alkanoyl,

(b) $C_1$—$C_4$-Alkyl, Phenyl oder substituiertes Phenyl, das 1 oder 2 Substituenten aus der Gruppe $C_1$—$C_4$-Alkyl, $C_1$—$C_4$-Alkoxy, geschütztes Hydroxyl, Chlor, Brom, Fluor, Nitro, Cyano, Methansulfonamido oder Trifluormethyl enthält, oder

(c) einen 5- oder 6-gliedrigen heterocyclischen Ring, der 1 bis 4 Heteroatome enthält, die ausgewählt sind aus der Gruppe Sauerstoff, Schwefel und Stickstoff, wobei dieser Ring unsubstituiert oder substituiert ist durch $C_1$—$C_4$-Alkyl, $C_1$—$C_4$-Alkoxy, Chlor, Brom, Oxo, Halogen($C_1$—$C_4$-alkyl), geschütztes Amino, geschütztes Amino($C_1$—$C_4$-alkyl), geschütztes Hydroxyl, geschütztes Hydroxy($C_1$—$C_4$-alkyl), geschütztes Carboxyl oder geschütztes Carboxy($C_1$—$C_4$-alkyl).

15. Verfahren nach Anspruch 1(d), 13 oder 14, dadurch gekennzeichnet, daß man den Triarylphosphit-Halogen-Komplex mit einem Cephalosporinsulfoxid umsetzt, worin $R_2$ eine Acylgruppe der Formel $R_7CO$— ist, in welcher $R_7$ folgende Bedeutung hat:

1) Wasserstoff, $C_1$—$C_6$-Alkyl, Halogen($C_1$—$C_4$)alkyl, Cyanomethyl, Trifluormethylthiomethyl oder 4-Geschütztes-amino-4-geschütztes-carboxybutyl,

2) die Gruppe $R_a$, worin $R_a$ Phenyl oder substituiertes Phenyl ist, das mit 1 oder 2 Substituenten aus der Gruppe $C_1$—$C_4$-Alkyl, $C_1$—$C_4$-Alkoxy, geschütztes Hydroxyl, Chlor, Brom, Fluor, Jod, Nitro, Cyano, Carbamyl, Methansulfonamido und Trifluormethyl substituiert ist,

3) eine Arylalkylgruppe der Formel

$$R^\circ—(Q)_m—CQ_1Q_2$$

worin R° für den oben angegebenen Substituenten $R_a$ oder für 1,4-Cyclohexadienyl oder einen 5-gliedrigen heterocyclischen Ring steht, der 1 bis 4 Heteroatome enthält, die ausgewählt sind aus der Gruppe Sauerstoff, Stickstoff und Schwefel, wobei dieser Ring unsubstituiert oder substituiert ist durch $C_1$—$C_4$-Alkyl, $C_1$—$C_4$-Alkoxy, Chlor, Brom, Oxo, geschütztes Amino, geschütztes Amino($C_1$—$C_4$-alkyl), geschütztes Hydroxyl oder geschütztes Carboxyl,

m für 1 oder 0 steht,

Q Sauerstoff oder Schwefel ist und

$Q_1$ und $Q_2$ unabhängig voneinander Wasserstoff oder Methyl sind,

mit der Maßgabe, daß in obiger Formel der Substituent R° auf $R_a$ beschränkt ist, falls m für 1 steht,

4) eine substituierte Arylalkylgruppe der Formel

$$\begin{array}{l} R^\circ CH— \\ \quad\;| \\ \quad\;W \end{array}$$

worin R° die oben angegebene Bedeutung hat und W für Ureido, geschütztes Amino, geschütztes Hydroxyl oder geschütztes Carboxyl steht, oder

5) eine substituierte Oximinogruppe der Formel

$$\begin{array}{l} R^\circ—C— \\ \qquad\;\| \\ \qquad NR_b \end{array}$$

worin R° die unmittelbar oben im Abschnitt 3) angegebene Bedeutung besitzt und $R_b$ für $C_1$—$C_4$-Alkoxy steht.

16. Verfahren nach einem der Ansprüche 1(d) oder 13 bis 15, dadurch gekennzeichnet, daß das Cephalosporinsulfoxid ein 3-Cephemsulfoxid oder ein 3-Exomethylencephamsulfoxid ist.

17. Verfahren nach Anspruch 1(e) zur Herstellung einer Verbindung der Formel

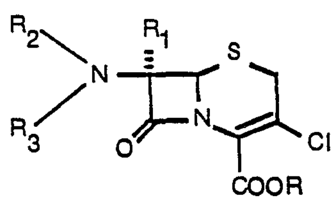

VIII

dadurch gekennzeichnet, daß man eine Verbindung der Formel

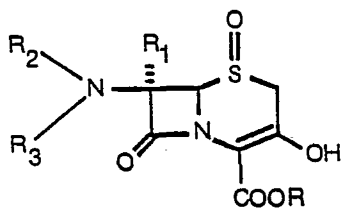

XIII

mit einem Triphenylphosphit-Chlor-Komplex umsetzt.

18. Verfahren nach Anspruch 1(e) oder 17, dadurch gekennzeichnet, daß man die Umsetzung zusätzlich in Gegenwart von 1 bis 2 Äquivalenten einer tertiären Aminbase durchführt.

19. Verfahren nach Anspruch 1(f) zur Herstellung eines Cephalosporiniminochlorids der Formel

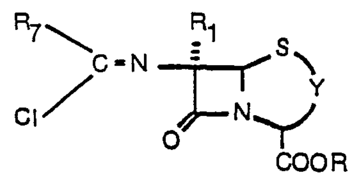

XVI

dadurch gekennzeichnet, daß man ein 7-Acylaminocephalosporinsulfoxid der Formel

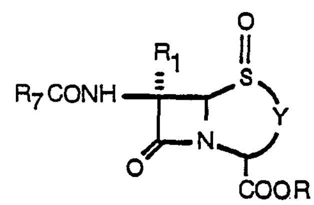

XV

mit einem Triphenylphosphit-Chlor-Komplex umsetzt.

20. Verfahren nach Anspruch 1(g) zur Herstellung eines 3-Chlorcephalosporiniminochlorids der Formel

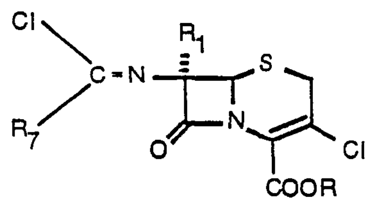

XI

dadurch gekennzeichnet, daß man ein 7-Acylamino-3-hydroxycephalosporinsulfoxid der Formel

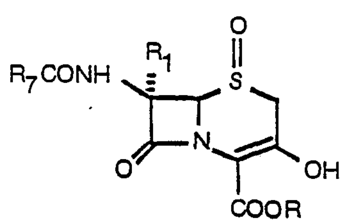

XVII

mit 3 bis 5 Äquivalenten eines Triphenylphosphit-Chlor-Komplexes umsetzt.

21. Verfahren nach Anspruch 20, dadurch gekennzeichnet, daß man den Triarylphosphit-Halogen-Komplex mit einem Cephalosporinsulfoxid umsetzt, worin $R_7$ die in Anspruch 15 angegebene Bedeutung hat.

22. Verfahren nach Anspruch 20 oder 21, dadurch gekennzeichnet, daß man 4 bis 5 Äquivalente eines Triarylphosphit-Halogen-Komplexes und 3,5 bis 4 Äquivalente einer tertiären Aminbase verwendet.

23. Verfahren nach Anspruch 22, dadurch gekennzeichnet, daß man 4,4 Äquivalente eines Triphenylphosphit-Chlor-Komplexes und 3,8 Äquivalente Pyridin verwendet.

24. Verfahren nach einem der Ansprüche 1(d), 1(e), 1(f), 1(g) und 13 bis 23, dadurch gekennzeichnet, daß der Halogenfänger ein $C_2$—$C_{10}$-Alken, ein Cycloalken mit 5 bis 8 Ringkohlenstoffatomen, ein $C_4$—$C_8$-Dien oder ein Cyclodien mit 5 bis 8 Ringkohlenstoffatomen, ein Alkin mit 2 bis 6 Kohlenstoffatomen oder ein leicht halogenierbares Phenolderivat der Formel

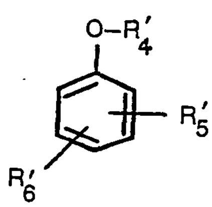

XVIII

ist, worin $R_4'$, für $C_1$—$C_4$-Alkyl oder $C_2$—$C_5$-Alkanoyl stehen und $R_5'$ sowie $R_6'$ unabhängig voneinander Wasserstoff, $C_1$—$C_4$-Alkoxy, $C_2$—$C_5$-Alkanoyl oder $C_1$—$C_4$-Alkyl bedeuten.

25. Verfahren nach Anspruch 24, dadurch gekennzeichnet, daß der Halogenfänger ein $C_2$—$C_6$-Alken ist.

26. Verfahren nach einem der Ansprüche 1(d), 1(e), 1(f), 1(g) und 13 bis 25, dadurch gekennzeichnet, daß die Temperatur —50°C bis 30°C beträgt.

27. Verfahren nach Anspruch 1(g), 20 und 21, dadurch gekennzeichnet, daß die Temperatur —30°C bis 0°C beträgt.

28. Verfahren nach Anspruch 1, dadurch gekennzeichnet, daß X für Br steht.

29. Verfahren nach Anspruch 28, dadurch gekennzeichnet, daß Z Wasserstoff ist.

30. Verfahren nach Anspruch 1, dadurch gekennzeichnet, daß X für Cl steht.

31. Verfahren nach einem der vorhergehenden Ansprüche, dadurch gekennzeichnet, daß der Triarylphosphit-Halogen-Komplex mit einer tertiären Aminbase stabilisiert ist.

32. Verfahren nach einem der Ansprüche 1(a), 1(c), 1(f), 1(g), 2 bis 6, 8 bis 12 oder 18 bis 21, dadurch gekennzeichnet, daß die tertiäre Aminbase einen $pK_b$-Wert von 6 bis 10 hat.

33. Verfahren nach Anspruch 31 oder 32, dadurch gekennzeichnet, daß die tertiäre Aminbase Pyridin ist.

34. Verfahren nach einem der vorhergehenden Ansprüche, dadurch gekennzeichnet, daß das inerte organische Lösungsmittel ein aromatischer Kohlenwasserstoff oder ein halogenierter Kohlenwasserstoff ist.

35. Verfahren nach Anspruch 34, dadurch gekennzeichnet, daß das inerte organische Lösungsmittel Methylenchlorid ist.

36. Verfahren nach einem der vorhergehenden Ansprüche, dadurch gekennzeichnet, daß die $C_6$- oder $C_7$-Acylgruppe 2-Thienylmethyl, Phenoxymethyl oder Benzyl ist.

37. Verfahren nach Anspruch 1(a), 1(c), 1(f), 1(g), 2 bis 6, 8 bis 12 oder 19 bis 21, dadurch gekennzeichnet, daß man nach beendeter Bildung des Iminochloridprodukts das Reaktionsgemisch mit einem wenigstens dreifachen Überschuß an einem $C_1$—$C_{15}$-aliphatischen Alkohol und an Chlorwasserstoff versetzt und so ein Kernesterhydrochlorid der Formel

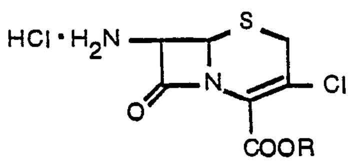

XIX

herstellt.

38. Verfahren nach Anspruch 37, dadurch gekennzeichnet, daß man nach beendeter Bildung des Iminochloridprodukts das Reaktionsgemisch mit wenigstens drei Äquivalenten eines $C_4$—$C_{12}$-$\beta$-disubstituierten primären aliphatischen Alkohols, eines $C_2$—$C_{12}$-1,2-Diols oder eines $C_3$—$C_{15}$-1,3-Diols und Chlorwasserstoff versetzt und so ein Kernesterhydrochlorid der Formel

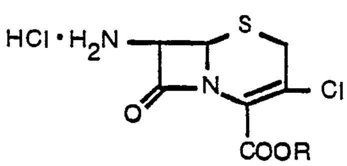

XIX

herstellt.

39. Verfahren nach Anspruch 38, dadurch gekennzeichnet, daß man als Alkohol oder Diol Isobutanol, 1,2-Propandiol oder 1,3-Propandiol verwendet.

40. Verfahren nach einem der vorhergehenden Ansprüche, dadurch gekennzeichnet, daß der Triphenylphosphit-Chlor-Komplex die Formel

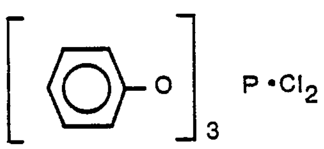

III

hat, welcher

(a) ein $^{31}$p-NMR-Resonanzsignal in Methylenchlorid von —3,7 ppm in Verhältnis zu dem von Phosphorsäure hat,

(b) in Methylenchlorid ein Infrarotspektrum mit folgenden charakteristischen Absorptionen aufweist: 1120 bis 1190 (sehr stark), 1070 (sehr stark), 1035 (stark), 1010 (sehr stark), 990 (sehr stark), 640 (mittel), 625 (mittel), 580 (schwach), 510 (stark) und 465 (schwach),

(c) mit Wasser unter Bildung von HCl und Triphenylphosphat reagiert und
(d) mit n-Butanol unter Bildung von HCl, n-Butylchlorid und Triphenylphosphat reagiert.

## Revendications

1. Procédé de préparation d'un dérivé de pénicilline ou de céphalosporine, caractérisé en ce qu'il consiste à faire réagir 1 à 5 équivalents d'un complexe de phosphite de triarylehalogène de formule:

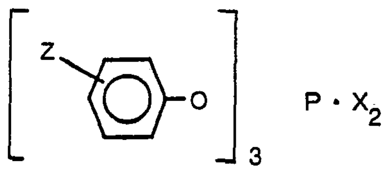

I

dans laquelle X représente Cl ou Br et Z représente un atome d'hydrogène, un atome d'halogène, un groupe alkyle en $C_1$—$C_4$ ou un groupe alcoxy en $C_1$—$C_4$, ce complexe étant le produit cinétiquement contrôlé de la réaction de quantités équivalentes d'un phosphite de triaryle de formule:

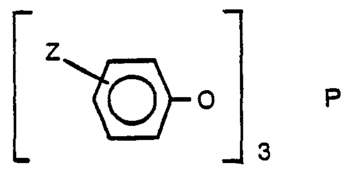

II

et de chlore ou de brome dans un solvant organique inerte, dans un solvant organique inerte pratiquement anhydre à une température de 30°C ou moins, avec une pénicilline ou une céphalosporine choisie parmi:

a) une C-6-acylamino-pénicilline ou une C-7-acylamino-céphalosporine à condition que, lorsque la C-6-acylamino-pénicilline ou la C-7-acylamino-céphalosporine est substituée par des groupes hydroxy, amino ou carboxy, ces groupes soient tout d'abord protégés par des groupes classiques protégeant les groupes hydroxy-amino ou carboxy, pour obtenir un imino-halogénure de pénicilline ou de céphalosporine; la réaction étant effectuée en présence de 1 à 1,2 équivalent d'une base d'amine tertiaire par équivalent de composé d'halogénation employé, ainsi qu'en utilisant 1 à 2 équivalents d'un composé d'halogénation;

b) un composé de formule:

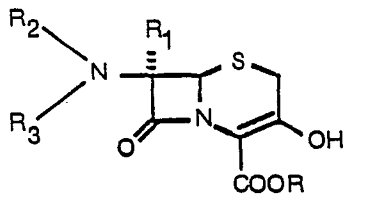

VII

pour obtenir un composé de formule:

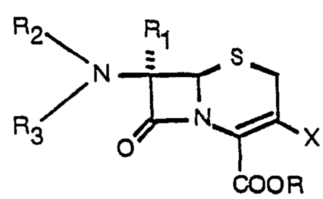

dans laquelle X représente Cl ou Br;

R représente un groupe protecteur d'acide carboxylique;

$R_1$ représente un atome d'hydrogène ou un groupe méthoxy; et

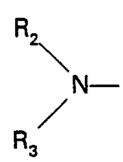

représente un groupe amino protégé par un groupe classique protégeant les groupes amino; ou

$R_2$ représente un atome d'hydrogène ou un groupe acyle dérivant d'un acide carboxylique; et

$R_3$ représente un groupe acyle dérivant d'un acide carboxylique; ou encore

$R_2$ et $R_3$ pris ensemble avec l'atome d'azote auquel ils sont reliés, forment un gorupe de formule:

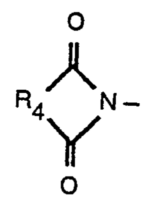

dans laquelle $R_4$ représente le radical d'un groupe acyle dérivant d'un acide dicarboxylique à condition que, lorsque $R_2$ et $R_3$ sont substitués par des groupes amino, hydroxy ou carboxy, ces groupes soient tout d'abord protégés par un des groupes classiques protégeant les groupes amino, hydroxy ou carboxy; en utilisant, au cours de la réaction, 1 à 1,3 équivalent d'un composé d'halogénation;

c) un composé de formule:

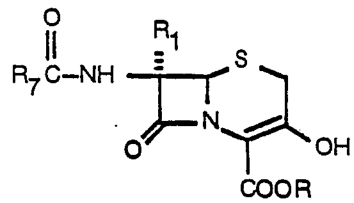

X

pour obtenir un composé de formule:

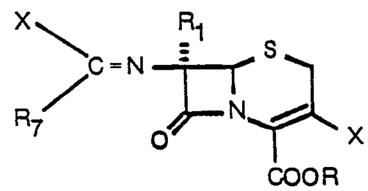

IX

où X, R et $R_1$ ont les significations définies ci-dessus et $R_7$ représente le radical d'un groupe acyle dérivant d'un acide carboxylique à condition que, lorsque $R_7$ est substitué par des groupes amino, hydroxy ou carboxy, ces groupes soient tout d'abord protégés par un des groupes classiques protégeant les groupes amino, hydroxy ou carboxy; la réaction étant effectuée en présence de 1 à 1,2 équivalent d'une base d'amine tertiaire par équivalent de composé d'halogénation employé et en utilisant 1 à 1,2 équivalent d'un composé d'halogénation;

d) un solfoxyde de céphalosporine à condition que, lorsque ce sulfoxyde de céphalosporine comporte un groupe amino, hydroxy ou carboxy libre sur le substituant C-7, ce groupe soit tout d'abord protégé par des groupes classiques protégeant les groupes amino, hydroxy ou carboxy, afin d'obtenir la céphalosporine correspondante; la réaction étant effectuée en présence d'au moins un équivalent d'un fixateur d'halogène et en utilisant 1 à 1,3 équivalent d'un composé d'halogénation;

e) un composé de formule:

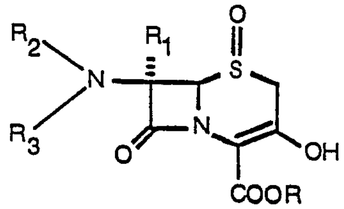

XIII

pour obtenir un composé de formule:

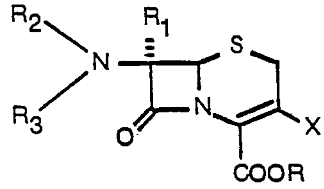

VI

où X, R, $R_1$, $R_2$ et $R_3$ ont les significations défines ci-dessus; la réaction étant effectuée en présence d'au

moins 1 équivalent molaire d'un fixateur d'halogène et en utilisant 2 à 3 équivalents d'un composé d'halogénation;

f) un sulfoxyde de 7-acylamino-céphalosporine de formule:

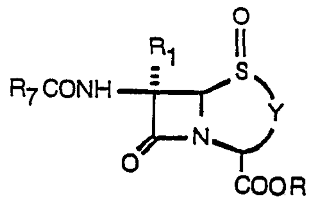

XV

pour obtenir un imino-halogénure de céphalosporine de formule:

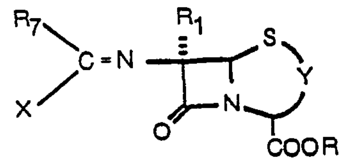

XIV

où X, R et $R_1$ ont les significations définies ci-dessus, et

$R_7$ représente le radical d'un groupe acyle dérivant d'un acide carboxylique en $C_1$—$C_{20}$ de formule $R_7COOH$; tandis que

Y représente un radical bivalent choisi parmi le groupe comprenant

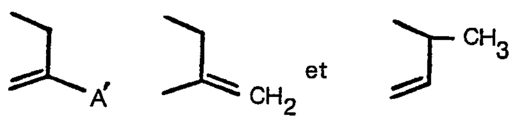

où A' représente un atome d'hydrogène, un atom de chlore, un atome de brome, un groupe hydroxy protégé, un groupe alcoxy en $C_1$—$C_4$, un groupe méthyle, un groupe alcane(en $C_1$—$C_4$)sulfonyloxy, un groupe alkyl(en $C_1$—$C_4$)phényl-sulfonyloxy ou un groupe de formule —$CH_2B$ où

B représente:

1) un groupe alcanoyloxy en $C_2$—$C_4$, un groupe carbamoyloxy ou un groupe alkyl(en $C_1$—$C_4$)-carbamoyloxy;

2) un groupe alcoxy en $C_1$—$C_4$;

3) un atome de chlore ou un atome de brome;

4) un groupe alcoxy(en $C_1$—$C_4$)carbonyle ou un groupe (halo-alcoxy(en $C_2$—$C_6$))carbonyle; ou

5) un groupe de formule —$SR_9$ dans laquelle $R_9$ représente:

(a) un groupe alcanoyle en $C_1$—$C_4$;

(b) un groupe alkyle en $C_1$—$C_4$, un groupe phényle, ou un groupe phényle substitué par un ou deux substituants choisis parmi le groupe comprenant un groupe alkyle en $C_1$—$C_4$, un groupe alcoxy en $C_1$—$C_4$, un groupe hydroxy protégé, un atome de chlore, un atome de brome, un atome de fluor, un groupe nitro, un groupe cyano, un groupe méthane-sulfonamido et un groupe trifluorométhyle; ou

(c) un noyau hétérocyclique pentagonal ou hexagonal contenant 1 à 4 hétéro-atomes choisis parmi le groupe comprenant l'oxygène, le soufre et l'azote, ce noyau étant non substitué ou substitué par un groupe alkyle en $C_1$—$C_4$, par un groupe alcoxy en $C_1$—$C_4$, par un atome de chlore, par un atome de brome, par un groupe oxo, par un groupe halo(alkyle(en $C_1$—$C_4$)), par un groupe amino protégé, par un groupe amino(alkyle(en $C_1$—$C_4$)) protégé, par un groupe hydroxy protégé, par un groupe hydroxy(alkyle(en $C_1$—$C_4$)) protégé, par un groupe carboxy protégé ou par un groupe carboxy(alkyle(en $C_1$—$C_4$)) protégé; à condition que, lorsque $R_7$ est substitué par des groupes hydroxy, amino ou carboxy, ces groupes soient tout d'abord protégés par des groupes classiques protégeant les groupes hydroxy, amino ou carboxy; la réaction étant effectuée en présence d'au moins un équivalent d'un fixateur d'halogène et de 1 à 2 équivalents d'une base d'amine tertiaire en employant, lors de la réaction, 2 à 3 équivalents d'un composé d'halogénation;

(d) un sulfoxyde de 7-acylamino-3-hydroxy-céphalosporine de formule:

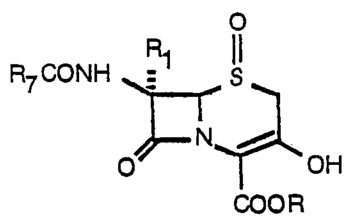

XVII

pour obtenir un imino-halogénure de 3-halocéphalosporine de formule:

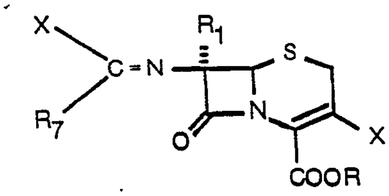

IX

dans laquelle X, R, $R_1$ et $R_7$ ont les significations définies ci-dessus; la réaction étant effectuée en présence d'au moins un équivalent d'un fixateur d'halogène et de 2 à 5 équivalents d'une base d'amine tertiaire, en utilisant 3 à 5 équivalents d'un composé d'halogénation.

2. Procédé suivant la revendication 1(a), caractérisé en ce qu'il consiste à faire réagir la C-6-acylamino-pénicilline ou la C-7-acylamino-céphalosporine avec un complexe de phosphite de triphényle/chlore.

3. Procédé suivant la revendication 1(a) ou 2, caractérisé en ce qu'il consiste à faire réagir le complexe de phosphite de triaryle/halogène avec un composé de C-6-acylamino-pénicilline ou de C-7-acylamino-céphalosporine de formule:

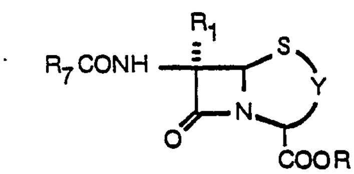

IV

dans laquelle

R représente un groupe protecteur d'acide carboxylique;

$R_1$ représente un atome d'hydrogène ou un groupe méthoxy;

$R_7CO$— représente un groupe acyle dérivant d'un acide carboxylique; et

Y représente un radical bivalent choisi parmi le groupe comprenant

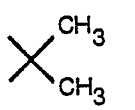 , 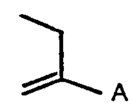 , 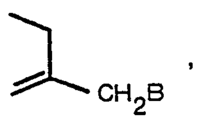 , 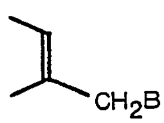 , 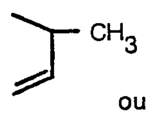 ou 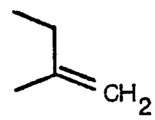

où A représente un atome d'hydrogène, un atome de chlore, un atome de brome, un groupe hydroxy protégé, un groupe alcoxy en $C_1$—$C_4$, un groupe méthyle, un groupe alcane(en $C_1$—$C_4$)sulfonyloxy ou un groupe alkyl(en $C_1$—$C_4$)phényl-sulfonyloxy; et

B représente

1) un groupe alcanoyloxy en $C_2$—$C_4$, un groupe carbamoyloxy ou un groupe alkyl(en $C_1$—$C_4$)carbamoyloxy;

2) un groupe alcoxy en $C_1$—$C_4$;

3) un atome de chlore ou un atome de brome;

4) un groupe de formule —$SR_9$ dans laquelle $R_9$ représente:

(a) un groupe alcanoyle en $C_1$—$C_4$;

(b) un groupe alkyle en $C_1$—$C_4$, un groupe phényle ou un groupe phényle substitué par un ou deux substituants choisis parmi le groupe comprenant un groupe alkyle en $C_1$—$C_4$, un groupe alcoxy en $C_1$—$C_4$, un groupe hydroxy protégé, un atome de chlore, un atome de brome, un atome de fluor, un groupe nitro, un groupe cyano, un groupe méthane-sulfonamido et un groupe trifluorométhyle; ou

(c) un noyau hétérocylique pentagonal ou hexagonal contenant 1 à 4 hétéro-atomes choisis parmi le groupe comprenant l'oxygène, le soufre et l'azote, ce noyau étant non substitué ou substitué par un groupe alkyle en $C_1$—$C_4$, par un groupe alcoxy en $C_1$—$C_4$, par un atome de chlore, par un atome de brome, par une groupe oxo, par un groupe halo(alkyle(en $C_1$—$C_4$)), par un groupe amino protégé, par un groupe amino(alkyle(en $C_1$—$C_4$)) protégé, par un groupe hydroxy protégé, par un groupe hydroxy(alkyle(en $C_1$—$C_4$)) protégé, par un groupe carboxy protégé ou par un groupe carboxy(alkyle(en $C_1$—$C_4$)) protégé.

4. Procédé suivant la revendication 1(a) ou 2, caractérisé en ce qu'il consiste à faire réagir le complexe de phosphite de triaryle/halogène avec une C-7-acylamino-céphalosporine qui est un composé de formule:

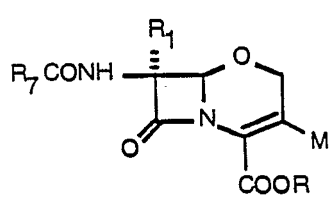

V

dans laquelle

R représente un groupe protecteur d'acide carboxylique;
$R_1$ représente un atome d'hydrogène ou un groupe méthoxy;
$R_7CO$— représente un groupe acyle dérivant d'un acide carboxylique, et
M représente un atome d'hydrogène, un atome de chlore, un atome de brome, un groupe hydroxy protégé, un groupe alcoxy en $C_1$—$C_4$, un groupe méthyle, un groupe alkyl(en $C_1$—$C_4$)phényl-sulfonyloxy ou un groupe de formule —$CH_2B$ dans laquelle B a la signification définie dans la revendication 3.

5. Procédé suivant l'une quelconque des revendications 1(a) à 4, caractérisé en ce qu'on emploie 1,1 à 1,2 équivalent d'un composé d'halogénation pour chaque équivalent de la matière de départ de C-6-acylamino-pénicilline ou de C-7-acylamino-céphalosporine.

6. Procédé suivant l'une quelconque des revendications 1(a) à 5, caractérisé en ce qu'on emploie un équivalent d'une base d'amine tertiaire pour chaque équivalent du composé d'halogénation utilisé.

7. Procédé suivant la revendication 1(b) en vue de préparer un composé de formule:

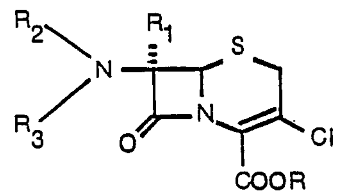

VIII

caractérisé en ce qu'il consiste à faire réagir un composé de formule:

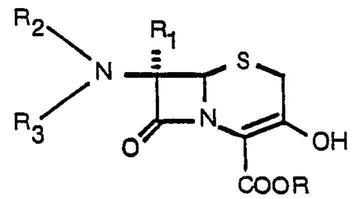

VII

avec un complexe de phosphite de triphényle/chlore.

8. Procédé suivant la revendication 1(c) pour la préparation d'un composé d'imino-halogénure de formule:

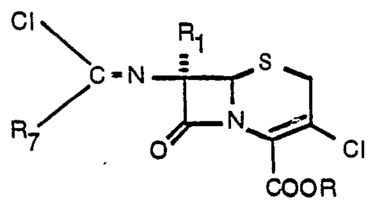

XI

caractérisé en ce qu'il consiste à faire réagir un composé de formule:

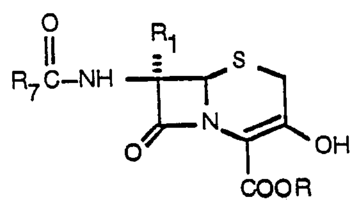

X

avec 2 à 3 équivalents d'un complexe de phosphite de triphényle/chlore.

9. Procédé suivant la revendication 7 ou 8, caractérisé en ce qu'on utilise 2,2 à 2,4 équivalents d'un composé d'halogénation.

10. Procédé suivant l'une quelconque des revendications 7 à 9, caractérisé en ce qu'on l'effectue en présence de 1 à 1,2 équivalent d'une base d'amine tertiaire par équivalent de composé d'halogénation.

11. Procédé suivant la revendication 10, caractérisé en ce qu'on utilise environ 1 équivalent d'une base d'amine tertiaire pour chaque équivalent du composé d'halogénation.

12. Procédé suivant l'une quelconque des revendications 1(a), (b) ou (c) à 11, caractérisé en ce que la base d'amine tertiaire a une valeur $pK_b$ de 6 à 10.

13. Procédé suivant la revendication 1(d), caractérisé en ce qu'il consiste à faire réagir le sulfoxyde de céphalosporine avec 1 à 1,3 équivalent d'un complexe de phosphite de triphényle/chlore.

14. Procédé suivant la revendication 1(d) ou 13, caractérisé en ce qu'il consiste à faire réagir le complexe de phosphite de triaryle/halogène avec un composé de formule:

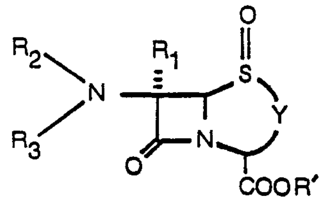

XII

dans laquelle

R' représente un atome d'hydrogène ou un groupe protecteur d'acide carboxylique;

$R_1$ représente un atome d'hydrogène ou un groupe méthoxy;

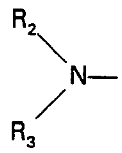

représente un groupe amino protégé par un groupe classique protégeant les groupes amino; ou

$R_2$ représente un atome d'hydrogène ou un groupe acyle dérivant d'un acide carboxylique, et $R_3$ représente un groupe acyle dérivant d'un acide carboxylique; ou encore $R_2$ et $R_3$ pris ensemble avec l'atome d'azote auquel ils sont reliés, forment un groupe de formule:

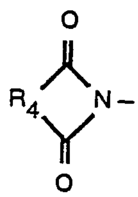

dans laquelle $R_4$ représente le radical d'un groupe acyle dérivant d'un acide dicarboxylique et Y représente un radical bivalent choisi parmi le groupe comprenant:

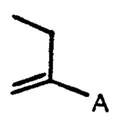 , 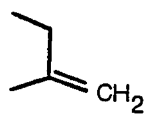 ou 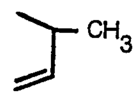

où A représente un atome d'hydrogène, un atome de chlore, un atome de brome, un groupe hydroxy, un groupe hydroxy protégé, un groupe alcoxy en $C_1$—$C_4$, un groupe méthyle, un groupe alcane(en $C_1$—$C_4$)sulfonyloxy, un groupe alkyl(en $C_1$—$C_4$)phényl-sulfonyloxy ou un groupe de formule —$CH_2B$ où B représente:

(1) un groupe alcanoyloxy en $C_2$—$C_4$, un groupe carbamoyloxy ou un groupe alkyl(en $C_1$—$C_4$)-carbamoyloxy;

(2) un groupe alcoxy en $C_1$—$C_4$;

(3) un atome de chlore ou un atome de brome;

(4) un groupe alcoxy(en $C_1$—$C_4$)carbonyle ou un groupe (halo-alcoxy(en $C_2$—$C_6$))carbonyle; ou

(5) un groupe de formule —$SR_9$ dans laquelle $R_9$ représente:

(a) un groupe alcanoyle en $C_1$—$C_4$;

(b) un groupe alkyle en $C_1$—$C_4$, un groupe phényle, ou un groupe phényle substitué par un ou deux substituants choisis parmi le groupe comprenant un groupe alkyle en $C_1$—$C_4$, un groupe alcoxy en $C_1$—$C_4$, un groupe hydroxy protégé, un atome de chlore, un atome de brome, un atome de fluor, un groupe nitro, un groupe cyano, un groupe méthane-sulfonamido et un groupe trifluorométhyle; ou

(c) un noyau hétérocyclique pentagonal ou hexagonal contenant 1 à 4 hétéro-atomes choisis parmi le groupe comprenant l'oxygène, le soufre et l'azote, ce noyau étant non substitué ou substitué par un groupe alkyle en $C_1$—$C_4$, par un groupe alcoxy en $C_1$—$C_4$, par un atome de chlore, par un atome de brome, par un groupe oxo, par un groupe halo(alkyle(en $C_1$—$C_4$)), par un groupe amino protégé, par un groupe amino(alkyle(en $C_1$—$C_4$)) protégé, par un groupe hydroxy protégé, par un groupe hydroxy(alkyle(en $C_1$—$C_4$)) protégé, par un groupe carboxy protégé ou par un groupe carboxy(alkyle(en $C_1$—$C_4$)) protégé.

15. Procédé suivant la revendication 1(d), 13 ou 14, caractérisé en ce qu'on fait réagir le complexe de phosphite de triaryle/halogène avec un sulfoxyde de céphalosporine dans lequel $R_5$ est un groupe acyle de formule $R_7CO$— où $R_7$ représente:

1) un atome d'hydrogène, un groupe alkyle en $C_1$—$C_6$, un groupe halo(alkyle(en $C_1$—$C_4$)), un groupe cyanométhyle, un groupe trifluorométhylthiométhyle, ou un groupe amino-4-protégé-carboxybutyle-4-protégé;

2) le groupe $R_a$ où $R_a$ représente un groupe phényle ou un groupe phényle substitué par un ou deux substituantes choisis parmi le groupe comprenant un groupe alkyle en $C_1$—$C_4$, un groupe alcoxy en $C_1$—$C_4$, un groupe hydroxy protégé, un atome de chlore, un atome de brome, un atome de fluor, un atome d'iode, un groupe nitro, un groupe cyano, un groupe carbamyle, un groupe méthane-sulfonamido et un groupe trifluorométhyle;

3) un groupe arylalkyle de formule:

$$R^o—(Q)_m—CQ_1Q_2—$$

où $R^o$ représente $R_a$ comme défini ci-dessus, un groupe 1,4-cyclohexadiényle ou un noyau hétérocyclique pentagonal contenant 1 à 4 hétéro-atomes choisis parmi le groupe comprenant l'oxygène, l'azote et le soufre, ce noyau étant non substitué par un groupe alkyle en $C_1$—$C_4$, par un groupe alcoxy en $C_1$—$C_4$, par un atome de chlore, par un atome de brome, par un groupe oxo, par un groupe amino protégé, par un groupe amino(alkyle(en $C_1$—$C_4$)) protégé, par un groupe hydroxy protégé ou par un groupe carboxy protégé;

m représente 1 ou 0;

Q représente un atome d'oxygène ou de soufre, et

$Q_1$ et $Q_2$ représentent chacun indépendamment l'un de l'autre un atome d'hydrogène ou un groupe méthyle, avec cette restriction que, si, dans la formule ci-dessus, m représente 1, $R^o$ est limité à $R_a$;

4) un groupe arylalkyle substitué de formule:

$$\begin{array}{l}R^oCH— \\ \ \ \ \ | \\ \ \ \ \ W\end{array}$$

dans laquelle $R^o$ a la signification définie ci-dessus et W représente un groupe uréido, un groupe amino protégé, un groupe hydroxy protégé ou un groupe carboxy protégé; ou

5) un groupe oximino substitué de formule:

$$\begin{array}{l}R^o—C— \\ \ \ \ \ \ \ \ \| \\ \ \ \ \ \ \ NR_b\end{array}$$

dans laquelle $R^o$ a la signification définie dans le paragraphe (3) qui précède immédiatement ci-dessus et $R_b$ représente un groupe alcoxy en $C_1$—$C_4$.

16. Procédé suivant l'une quelconque des revendications 1(d) ou 13 à 15, caractérisé en ce que le sulfoxyde de céphalosporine est un 3-céphem-sulfoxyde ou un 3-exométhylène-cépham-sulfoxyde.

17. Procédé suivant la revendication 1(e) en vue de préparer un composé de formule:

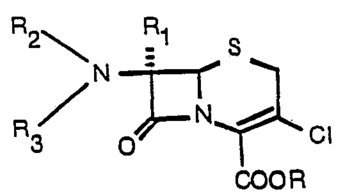

VIII

en faisant réagir un composé de formule:

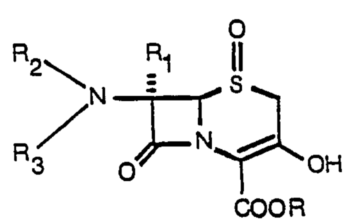

XIII

avec un complexe de phosphite de triphényle/chlore.

18. Procédé suivant la revendication 1(e) ou 17, caractérisé en ce que, en outre, on l'effectue en présence de 1 à 2 équivalents d'une base d'amine tertiaire.

19. Procédé suivant la revendication 1(f) en vue de préparer un imino-chlorure de céphalosporine de formule:

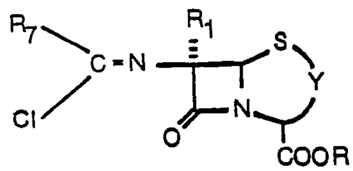

XVI

caractérisé en ce qu'il consiste à faire réagir un sulfoxyde de 7-acylamino-céphalosporine de formule:

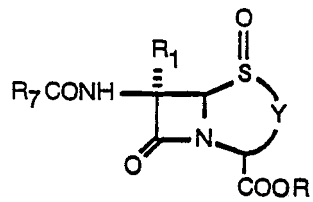

XV

avec un complexe de phosphite de triphényle/chlore.

20. Procédé suivant la revendication 1(g) en vue de préparer un imino-chlorure de 3-chloro-céphalosporine de formule:

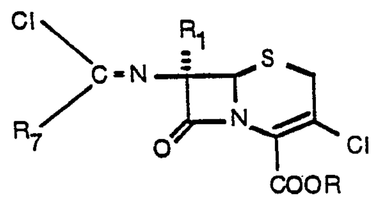

XI

caractérisé en ce qu'il consiste à faire réagir un sulfoxyde de 7-acylamino-3-hydroxy-céphalosporine de formule:

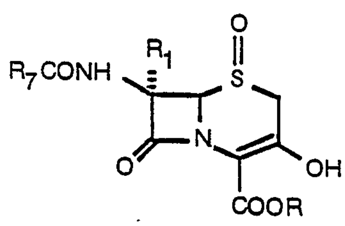

XVII

avec 3 à 5 équivalents d'un complexe de phosphite de triphényle/chlore.

21. Procédé suivant la revendication 20, caractérisé en ce qu'on fait réagir le complexe de phosphite de triaryle/halogène avec un sulfoxyde de céphalosporine dans lequel $R_7$ a la signification définie dans la revendication 15.

22. Procédé suivant la revendication 20 ou 21, caractérisé en ce qu'on utilise 4 à 5 équivalents d'un complexe de phosphite de triaryle/halogène et 3,5 à 4 équivalents d'une base d'amine tertiaire.

23. Procédé suivant la revendication 22, caractérisé en ce qu'on utilise 4,4 équivalents du complexe de phosphite de triphényle/chlore et 3,8 équivalents de pyridine.

24. Procédé suivant l'une quelconque des revendications 1(d), 1(e), 1(f), 1(g) et 13 à 23, caractérisé en ce que le fixateur d'halogène est un alcène en $C_2$—$C_{10}$, un cyclo-alcène contenant 5 à 8 atomes de carbone cycliques, un diène en $C_4$—$C_8$ ou un cyclodiène contenant 5 à 8 atomes de carbone cycliques, un alcyne contenant 2 à 6 atomes de carbone ou un dérivé du phénol aisément halogéné répondant à la formule:

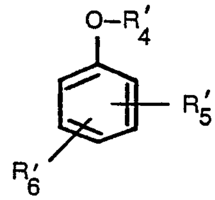

XVIII

dans laquelle $R'_4$ représente un groupe alkyle en $C_1$—$C_4$ ou un groupe alcanoyle en $C_2$—$C_5$, tandis que $R'_5$ et $R'_6$ représentent chacun indépendamment l'un de l'autre un atome d'hydrogène, un groupe alcoxy en $C_1$—$C_4$, un groupe alcanoyle en $C_2$—$C_5$ ou un groupe alkyle en $C_1$—$C_4$.

25. Procédé suivant la revendication 24, caractérisé en ce que le fixateur d'halogène est un alcène en $C_2$—$C_6$.

26. Procédé suivant l'une quelconque des revendications 1(d), 1(e), 1(f), 1(g) et 13 à 25, caractérisé en ce que la température se situe entre −50 et 30°C.

27. Procédé suivant les revendications 1(g), 20 et 21, caractérisé en ce que la température se situe entre −30 et 0°C.

28. Procédé suivant la revendication 1, caractérisé en ce que X représente Br.

29. Procédé suivant la revendication 28, caractérisé en ce que Z est un atome d'hydrogène.

30. Procédé suivant la revendication 1, caractérisé en ce que X représente Cl.

31. Procédé suivant l'une quelconque des revendications précédentes, caractérisé en ce que le complexe de phosphite de triaryle/halogène est stabilisé avec une base d'amine tertiaire.

32. Procédé suivant l'une quelconque des revendications 1(a), 1(c), 1(f), 1(g), 2 à 6, 8 à 12 ou 18 à 21, caractérisé en ce que la base d'amine tertiaire a une valeur $pK_b$ de 6 à 10.

33. Procédé suivant la revendication 31 ou 32, caractérisé en ce que la base d'amine tertiaire est la pyridine.

34. Procédé suivant l'une quelconque des revendications précédentes, caractérisé en ce que le solvant organique inerte est un hydrocarbure aromatique ou un hydrocarbure halogéné.

35. Procédé suivant la revendication 34, caractérisé en ce que le solvant organique inerte est le chlorure de méthylène.

36. Procédé suivant l'une quelconque des revendications précédentes, caractérisé en ce que le groupe acyle en $C_6$ ou $C_7$ est un groupe 2-thiényl-méthyle, un groupe phénoxy-méthyle ou un groupe benzyle.

37. Procédé suivant les revendications 1(a), 1(c), 1(f), 1(g), 2 à 6, 8 à 12 ou 19 à 21, caractérisé en ce que, lorsque la formation du produit d'imino-chlorure est achevée, on ajoute au moins un excès triple d'un alcool aliphatique en $C_1$—$C_{15}$ et de chlorure d'hydrogène au mélange réactionnel pour obtenir un chlorhydrate d'ester nucléaire de formule:

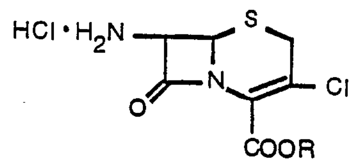

XIX

38. Procédé suivant la revendication 37, caractérisé en ce que, lorsque la formation du produit d'imino-chlorure est achevée, on ajoute au moins 3 équivalents d'un alcool aliphatique primaire $\beta$-disubstitué en $C_4$—$C_{12}$, un 1,2-diol en $C_2$—$C_{12}$ ou un 1,3-diol en $C_3$—$C_{15}$ et du chlorure d'hydrogène au mélange réactionnel pour obtenir un chlorhydrate d'ester nucléaire de formule:

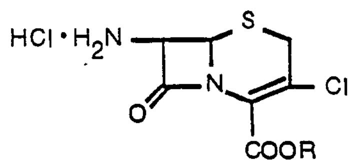

XIX.

39. Procédé suivant la revendication 38, caractérisé en ce que l'alcool ou le diol est l'isobutanol, le 1,2-propane-diol ou le 1,3-propane-diol.

40. Procédé suivant l'une quelconque des revendications précédentes, caractérisé en ce que le complexe de phosphite de triphényle/chlore répond à la formule:

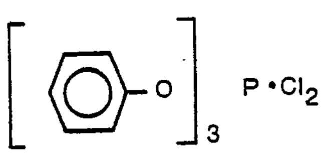

III

ce complexe:

(a) a un signal de résonance magnétique nucléaire $^{31}P$ dans le chlorure de méthylène à −3,7 parties par million par rapport à celui de l'acide phosphorique;

(b) a, dans le chlorure de méthylène, un spectre infrarouge présentant les absorptions caractéristiques suivantes: 1120—1190 (très forte), 1070 (très forte), 1035 (forte), 1010 (très forte), 990 (très forte), 640 (moyenne), 625 (moyenne), 580 (faible), 510 (forte) et 465 (faible);

(c) réagit avec l'eau pour former HCl et le phosphate de triphényle; et

(d) réagit avec le n-butanol pour former HCl, le chlorure de n-butyle et le phosphate de triphényle.